# EUROPEAN PATENT SPECIFICATION

(11) **EP 1 002 034 B1**
(45) Date of publication and mention of the grant of the patent: **31.01.2007**
(21) Application number: 97934949.5
(22) Date of filing: 09.07.1997
(51) Int. Cl.: C11D 3/386

(54) **CLEANING COMPOSITIONS COMPRISING A SPECIFIC OXYGENASE**
REINIGUNGSMITTEL ENTHALTEND EINE SPEZIELLE OXYGENASE
COMPOSITIONS DE LAVAGE/NETTOYAGE CONTENANT UNE OXYGENASE SPECIFIQUE

(43) Date of publication of application: 24.05.2000
(73) Proprietor: THE PROCTER & GAMBLE COMPANY, Cincinnati, Ohio 45202 (US)
(72) Inventor: HERBOTS, Ivan, Maurice, Alfons, Jan, B-9230 Wetteren (BE); BUSCH, Alfred, B-1840 Londerzeel (BE)
(74) Representative: Morelle, Evelyne Charlotte Isabelle
(86) International application number: PCT/US1997/012280
(87) International publication number: WO 1999/002632

(56) References cited:
- EP-A- 0 086 139
- WO-A-96/06532
- US-A- 3 933 593
- US-A- 4 645 760

## Description

### Field of the Invention

The present invention relates to cleaning compositions, including laundry, dishwashing, hard surface cleaner, oral/dental cleaning compositions, comprising a proteinic substrate based oxygenase.

### Background of the invention

Performance of a cleaning product, for use in washing or cleaning method, is judged by a number of factors, including the ability to remove soils, and the ability to prevent the redeposition of the soils, or the breakdown products of the soils on the articles in the wash.

Many consumer relevant stains contain proteinic components and are difficult to remove from the washed surfaces. Food stains originating from dairy products as such, like milk or egg, or processed within food or drink products, are particularly challenging to remove.

In addition, the complex nature of everyday "body" soils typically found on pillow cases, T-shirts, collars and socks, provides a continuous thorough cleaning challenge for detergents. These soils are difficult to remove completely and often residues build up on fabric leading to dinginess and yellowing. Moreover, body fluids stains, such as blood and menstrual fluids, are often difficult to remove effectively from a soiled item, especially when the stains have been ageing. Everyday body soils are also found on sanitary and kitchen surfaces such as bathtubs, toilet bowl and dishware.

The items can be fabrics, hard surfaces, dishware such as plasticware, glassware or chinaware, teeth and mouth.

Traditionally, protease enzymes and/or high levels of bleaching compounds, optionally with bleach precursors and/or bleach enhancers, are incorporated in cleaning compositions. Bleaching agents are compounds which are precursors of hydrogen peroxide which is formed in the course of the washing procedure. Perborates and percarbonates are the most important examples of such hydrogen peroxide precursors.

In view of the above, there exits clearly a continuous need to provide cleaning compositions which have an excellent detergency performance. Accordingly it is an object of the present invention to provide a cleaning composition which provides effective and efficient cleaning of proteic based stains and/or soils such as protein containing food stains/soils and everyday body soils. It is a further object to provide a cleaning composition which provides fabric realistic items cleaning and whitening.

The above objective has been met by formulating cleaning compositions comprising a proteinic substrate based oxygenase.

It has been surprisingly found that an enzymatic bleach system based on a proteinic substrate based oxygenase delivers in a cleaning composition, bleach-like benefits in an unexpected broad range of performance areas such as dingy cleaning, whiteness maintenance and stain removal. It has also been found that the cleaning compositions of the present invention provide sanitisation of the treated surfaces.

It has been further found that the performance of the cleaning compositions of the present invention is improved by the addition of another enzymatic bleach system, a conventional activated bleach system, a metallo catalyst based bleach system and/or another detergent enzyme, especially a protease.

In a preferred embodiment, the present invention relates to a laundry composition comprising a proteinic substrate based oxygenase, further providing fabric realistic items cleaning and whitening. In a second embodiment, the present invention relates to dishwashing or household cleaning compositions comprising a proteinic substrate based oxygenase and in a third embodiment, the present invention relates to oral/dental care compositions comprising a proteinic substrate based oxygenase.

The use of a proteinic substrate based oxygenase in a cleaning composition for substantive removal of proteic based stains/soils such as protein containing food stains/soils and everyday body soils and for sanitisation, has never been previously recognised. Nor have been recognised the substantive fabric realistic items cleaning and whitening performance when the proteinic substrate based oxygenase is used within a laundry detergent composition.

WO96/06532 discloses compositions capable of killing microbial cells or inhibiting growing microbial cells, comprising a basic protein or peptide of biological origin, optionally with a cell wall degrading enzyme or an oxidoreductase. EP 086 139 is directed to novel expression vectors of the catechol 2, 3 oxygenase and the application of this enzyme in the food and pharmaceutical industries. US 3.933,593 relates to a portable automated chemical analyser having a polarographic oxygen electrode submerged in a batch of stirred solution and to methods for analysing glucose-glucose oxidase, catalase-H2O2 and various enzyme systems. US 4,645,760 relates to aminoglycosides and aminoglycoside-arninocytols which have been oxygen-radical activated for broad spectrum of antibacterial activity. WO98/28400 discloses enzymatic bleach compositions comprising a surfactant and an extra-cellular enzyme capable of oxidising substrates by the built-in of one or more oxygen atoms in the substrate using molecular oxygen, in particular a dioxygenase.

### Summary of the invention

The present invention relates to cleaning compositions, including laundry, dishwashing, hard surface cleaner, oral/dental cleaning compositions, comprising a proteinic substrate based oxygenase, which provide effective and efficient cleaning of proteinic based stains and/or soils such as protein containing food stains/soils and/or everyday body soils and provide sanitisation of the treated surfaces.

Furthermore, the cleaning compositions of the present invention provide fabric realistic items cleaning and whitening performance when formulated as a laundry detergent composition.

### Detailed description of the invention

The essential component of the cleaning compositions of the present invention is a proteinic substrate based oxygenase. Preferably said enzyme is further characterised by being an iron sulphur or iron heme oxygenase and/or a heavy metal dependant oxygenase.

All oxygenases of the present invention will pass the following stain removal performance test achieved on standard test fabrics and being compared to a nil-oxygenase reference in same conditions.
The small scale test is done in a launderometer Washtec^{™} ROACHES^{™} equipped with stainless steel jars of 500 ml. The small scale test is done at 30-40 °C with total wash time of 30 minutes. Tests is done in a 1% detergent solution using the detergent as described in example 10, composition 5. The detergent is dissolved in 400 ml of water with a total hardness between 2.0 - 3.0 mmol Ca2+/L. The pH of the 1% detergent solution is adjusted with an acid (citric acid) or an alkali (NaOH) to pH 8-9. Each jar also contains 15 steel ball for better agitation. Standard stain test fabrics are supplied by wfk-Testgewebe Gmbh (Christenfeld,10-Brueggen, Germany). At least two replicates are run per test. The size of the stains is 3 cmx4 cm. The standard test fabric having the protein substrate are the wfk F10EM (eggyolk + milk stained cotton). The enzyme level in this small scale test is 1mg enzyme protein/L. Levels of cofactor(s) when required are calculated according to the enzyme to cofactor(s) ratio known from literature.
For the reference treatment no enzyme or co-factor is added to the solution. In the test treatment the pre-weighted enzyme and cofactor is added to the solution and in a second test treatment only the co-factor is added to the solution. After that the test tracers are put in the jars. The launderometer is set on the required temperature. The launderometer is preheated to the right temperature before starting the test so the test is run at a constant temperature.
The jars are closed and put in the preheated launderometer. The cycle time is set and the test is started. After 30 minutes the test is stopped, the jars are taken out of the equipment and are opened. The standard test fabrics are taken out and rinsed 3 times in cold city water (hardness 2-3 mmole Ca2+/L). After rinsing the tracers are tumble dried in a normal household drier to completely dry.

The stain removal performance of the enzyme is analyzed by visual grading by an expert panel or preferentially by an instrumental stain removal measurement e.g. with the Spectraflash^{™} 500 apparatus from Datacolor. The Spectraflash^{™} 500 settings used for this test are : specular excluded, aperture small and U.V. filter FL40 (= UV cut off filter at 400nm) and calibration is done versus a standard white and black.
The result expressed in color difference (Cielab) dE, is calculated between the test and reference treatment. Test 1 comprises the proteinic substrate based oxygenase of the present invention, test 2 comprises a monophenol monooxygenase of EC 1.14.18.1. The enzymes of the invention (Test 1) have a dE of 1 or more versus the monophenol monooxygenase (Test 2) tested in same conditions.

It has been found that the cleaning compositions of the present invention provide effective and efficient cleaning of proteinic based stains and/or soils such as protein containing food stains/soils and everyday body soils, and in particular, achieve fabric realistic items cleaning and whitening when formulated as laundry detergent composition. Indeed it has been surprisingly found that the proteinic substrate based oxygenase can be efficient on a broader range of proteic materials than the current detergent proteases and on protein compounds not degraded by current detergent proteases.

Without wishing to be bound by theory, It is believed that the performance of this enzyme is achieved through decyclising, decarboxylation and side chain cleavage reactions of the amino acids, peptides and proteins orignating from the protein fraction of the glycoproteins and lipoproteins found in the stains / soils. These reactions are achieved by catalytical direct insertion of molecular oxygen into the amino acids structure.

In addition, the cleaning compositions of the present invention provide sanitisation of the treated surfaces.
Sanitisation includes all positive effects obtained by the inhibition or reduction of microbial activity on fabrics and other surfaces, such as the prevention of malodour development and bacterial/fungal growth. For example, it provides prevention of malodour development on stored and weared fabrics, on stored dishware, especially plastic kitchen gear and in toilets. In particular, the composition of the invention will inhibit or at least reduce the bacterial and/or fungal development on moist fabric waiting for further laundry processing and thereby preventing the formation of malodour. In addition, bacterial and/or fungal growth on hard surfaces such as tiles and their silicone joints, sanitary installations, will be prevented.
The sanitisation potential of the cleaning compositions of the present invention can be enhanced by the addition of chemical sanitisers such as Triclosan and/or hexemidine. Parfums Cosmétiques Actualités No 125, Nov, 1995, 51-4 describes suitable chemical sanitisers.
The sanitisation benefits of the cleaning compositions of the present invention can be evaluated by the Minimum Inhibitory Concentration (MIC) as described in Tuber. Lung. Dis. 1994 Aug; 75(4):286-90; J. Clin. Microbiol. 1994 May; 32(5):1261-7 and J. Clin. Microbiol. 1992 Oct; 30(10):2692-7.

Without wishing to be bound by theory, it is believed that "everyday body soils" contain sebum excreted by the human body. Sebum is believed to contain large quantities of saturated and unsaturated fatty acids, sterols and sterols esters (The physiology and Pathology of the skin, Vol. 9, A. Jarret, (1986)). The fragmentation of the substrate and the formation of ionizable groups or hydrophilic substituents by a proteinic substrate based oxygenase enzyme renders the enzymatic reaction products more soluble and hence easier to be removed from the soiled items.

The proteinic substrate based oxygenases of the present invention are listed below:

| EC NUMBER | RECOMMENDED NAME |
|---|---|
| 1.13.11.11 | TRYPTOPHAN 2,3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11.30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12. 9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROLIN,2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN,2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYLGLYCINE MONOOXYGENASE |

Some of the proteinic substrate based oxygenases of the present invention require the presence of a cofactor. In this instance, the cleaning compositions of the present invention will further comprise a cofactor such as Ascorbate, oxoglutarate, Flavine Mononucleotide (FMN), Flavine Adenine Dinucleotide (FAD), Nicotine amide Adenine Dinucleotide (Phosphate) (NAD(P)H). When therein include, the cofactor will be comprised at a weight ratio of pure oxygenase to cofactor comprised generally between 10:1 to 1:10, preferably between 5:1 to 1:8, more preferably between 1:2 to 1:5.

The proteinic substrate based oxygenase enzyme is incorporated into the cleaning compositions in accordance with the invention preferably at a level of from 0.0001 % to 2%, more preferably from 0.001% to 0.5%, most preferably from 0.002% to 0.1% pure enzyme by weight of the composition.

Preferred proteinic substrate based oxygenases for specific applications are alkaline proteinic substrate based oxygenases, i.e. enzymes having an enzymatic activity of at least 10%, preferably at least 25%, more preferably at least 40% of its maximum activity at a pH ranging from 7 to 12. More preferred proteinic substrate based oxygenases are enzymes having their maximum activity at a pH ranging from 7 to 12.

Enzymes homologue to the proteinic substrate based oxygenase of the present invention are also contemplated. The term "homologue" is intended to indicate a polypeptide encode by DNA which hybridises to the same probe as the DNA coding for the proteinic substrate based oxygenase enzyme with this amino acid sequence under certain specific conditions (such as presoaking in 5xSSC and prehybridising for 1 h at ~40°C in a solution of 20% formamide, 5xDenhardt's solution, 50 mM sodium phosphate, pH 6.8, and 50 µg of denaturated sonicated calf thymus DNA, followed by hybridisation in the same solution supplemented with 100 µM ATP for 18 h at ~40°C). The term is intended to include derivatives of the proteinic substrate based oxygenase enzyme sequence obtained by addition of one or more amino acid residues to either or both the C- and N-terminal of the native sequence, substitution of one or more amino acid residues at one or more sites in the native sequence, deletion of one or more amino acid residues at either or both ends of the native amino acid sequence or at one or more sites within the native sequence, or insertion of one or more amino acid residues at one or more sites of the native sequence.

The above-mentioned enzymes may be of any suitable origin, such as vegetable, animal, bacterial, fungal and yeast origin. Origin can further be mesophilic or extremophilic (psychrophilic, psychrotrophic, thermophilic, barophilic, alkalophilic, acidophilic, halophilic, etc.). Purified or non-purified forms of these enzymes may be used. Nowadays, it is common practice to modify wild-type enzymes via protein / genetic engineering techniques in order to optimise their performance efficiency in the cleaning compositions of the invention. For example, the variants may be designed such that the compatibility of the enzyme to commonly encountered ingredients of such compositions is increased. Alternatively, the variant may be designed such that the optimal pH, bleach or chelant stability, catalytic activity and the like, of the enzyme variant is tailored to suit the particular cleaning application.

In particular, attention should be focused on amino acids sensitive to oxidation in the case of bleach stability and on surface charges for the surfactant compatibility. The isoelectric point of such enzymes may be modified by the substitution of some charged amino acids, e.g. an increase in isoelectric point may help to improve compatibility with anionic surfactants. The stability of the enzymes may be further enhanced by the creation of e.g. additional salt bridges and enforcing calcium binding sites to increase chelant stability.

### Detergent components

The cleaning compositions of the present invention may also contain additional detergent components. The precise nature of these additional components, and levels of incorporation thereof will depend on the physical form of the composition, and the nature of the cleaning operation for which it is to be used.

The cleaning compositions preferably further comprise another enzymatic bleach system, a conventional activated bleach system, a metallo catalyst based bleach system and/or another detergent enzyme.

In a preferred embodiment, the present invention relates to a laundry and/or fabric care composition comprising a proteinic substrate based oxygenase (Examples 1-18). In a second embodiment, the present invention relates to dishwashing or household cleaning compositions including sanitisation compositions (Examples 19-28), and in a third embodiment, the present invention relates to oral/dental care compositions (Examples 29-31).

The cleaning compositions according to the invention can be liquid, paste, gels, bars, tablets, spray, foam, powder or granular forms. Granular compositions can also be in "compact" form, the liquid compositions can also be in a "concentrated" form.

The compositions of the invention may for example, be formulated as hand and machine dishwashing compositions, hand and machine laundry detergent compositions including laundry additive compositions and compositions suitable for use in the soaking and/or pretreatment of stained fabrics, rinse added fabric softener compositions, and compositions for use in general household hard surface cleaning operations. Compositions containing such proteinic substrate based oxygenase can also be formulated as oral /dental care compositions.

When formulated as compositions for use in manual dishwashing methods the compositions of the invention preferably contain a surfactant and preferably other detergent compounds selected from organic polymeric compounds, suds enhancing agents, group II metal ions, solvents, hydrotropes and additional enzymes.

When formulated as compositions suitable for use in a laundry machine washing method, the compositions of the invention preferably contain both a surfactant and a builder compound and additionally one or more detergent components preferably selected from organic polymeric compounds, bleaching agents, additional enzymes, suds suppressors, dispersants, lime-soap dispersants, soil suspension and anti-redeposition agents and corrosion inhibitors. Laundry compositions can also contain softening agents, as additional detergent components.
Such compositions containing proteinic subtrate based oxygenase can provide fabric cleaning, stain removal, whiteness maintenance, softening, colour appearance, dye transfer inhibition and sanitisation when formulated as laundry detergent compositions.

The compositions of the invention can also be used as detergent additive products. Such additive products are intended to supplement or boost the performance of conventional detergent compositions.

If needed the density of the laundry detergent compositions herein ranges from 400 to 1200 g/litre, preferably 600 to 950 g/litre of composition measured at 20°C.

The "compact" form of the compositions herein is best reflected by density and, in terms of composition, by the amount of inorganic filler salt; inorganic filler salts are conventional ingredients of detergent compositions in powder form; in conventional detergent compositions, the filler salts are present in substantial amounts, typically 17-35% by weight of the total composition.
In the compact compositions, the filler salt is present in amounts not exceeding 15% of the total composition, preferably not exceeding 10%, most preferably not exceeding 5% by weight of the composition.

The inorganic filler salts, such as meant in the present compositions are selected from the alkali and alkaline-earth-metal salts of sulphates and chlorides.
A preferred filler salt is sodium sulphate.

Liquid detergent compositions according to the present invention can also be in a "concentrated form", in such case, the liquid detergent compositions according the present invention will contain a lower amount of water, compared to conventional liquid detergents.
Typically the water content of the concentrated liquid detergent is preferably less than 40%, more preferably less than 30%, most preferably less than 20% by weight of the detergent composition.

### Conventional detergent enzymes

The cleaning compositions of the present invention can in addition to the proteinic substrate based oxygenase enzyme further comprise one or more enzymes which provide cleaning performance, fabric care and/or sanitisation benefits. It has been found that the combination of said specific oxygenase with a detergent enzyme provides improved cleaning of proteic based stains and/or soils such as protein containing food stains/soils and everyday body soils and when formulated as laundry composition, improved fabric realistic items cleaning and whitening.

Preferably, the cleaning compositions of the present invention will comprise a protease. Indeed it has been surprisingly found that the proteinic substrate based oxygenase can be efficient on a broader range of proteic materials than current detergent proteases and on protein compounds not degraded by current detergent proteases. The combination of the proteinic substrate based oxygenase and a protease gives synergistic performance on removal of proteic based stains/soils and everyday body soils.

Said enzymes include enzymes selected from cellulases, hemicellulases, peroxidases, proteases, gluco-amylases, amylases, xylanases, lipases, phospholipases, esterases, cutinases, pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase or mixtures thereof.

A preferred combination is a cleaning composition having cocktail of conventional applicable enzymes like protease, amylase, lipase, cutinase and/or cellulase in conjunction with one or more plant cell wall degrading enzymes.
Suitable proteases are the subtilisins which are obtained from particular strains of *B. subtilis* and *B. licheniformis* (subtilisin BPN and BPN'). One suitable protease is obtained from a strain of *Bacillus,* having maximum activity throughout the pH range of 8-12, developed and sold as ESPERASE^{®} by Novo Industries A/S of Denmark, hereinafter "Novo". The preparation of this enzyme and analogous enzymes is described in GB 1,243,784 to Novo. Other suitable proteases include ALCALASE^{®}, DURAZYM^{®} and SAVINASE^{®} from Novo and MAXATASE^{®}, MAXACAL^{®}, PROPERASE^{®} and MAXAPEM^{®} (protein engineered Maxacal) from Gist-Brocades. Proteolytic enzymes also encompass modified bacterial serine proteases, such as those described in European Patent Application Serial Number 87 303761.8, filed April 28, 1987 (particularly pages 17, 24 and 98), and which is called herein "Protease B", and in European Patent Application 199,404, Venegas, published October 29, 1986, which refers to a modified bacterial serine protealytic enzyme which is called "Protease A" herein. Suitable is what is called herein "Protease C", which is a variant of an alkaline serine protease from *Bacillus* in which lysine replaced arginine at position 27, tyrosine replaced valine at position 104, serine replaced asparagine at position 123, and alanine replaced threonine at position 274. Protease C is described in EP 90915958:4, corresponding to WO 91/06637, Published May 16, 1991. Genetically modified variants, particularly of Protease C, are also included herein.
A preferred protease referred to as "Protease D" is a carbonyl hydrolase variant having an amino acid sequence not found in nature, which is derived from a precursor carbonyl hydrolase by substituting a different amino acid for a plurality of amino acid residues at a position in said carbonyl. hydrolase equivalent to position +76, preferably also in combination with one or more amino acid residue positions equivalent to those selected from the group consisting of +99, +101, +103, +104, +107, +123, +27, +105, +109, +126, +128, +135, +156, +166, +195, +197, +204, +206, +210, +216, +217, +218, +222, +260, +265, and/or +274 according to the numbering of *Bacillus amyloliquefaciens* subtilisin, as described in WO95/10591 and in the patent application of C. Ghosh, et al, "Bleaching Compositions Comprising Protease Enzymes" having US Serial No. 08/322,677, filed October 13, 1994. Also suitable are variants having a different proteolytic activity, stability, substrate specificity, pH profile and/or performance characteristic as compared to the precursor carbonyl hydrolase from which the amino acid sequence of the variant is derived. As stated earlier, the protease enzymes are designed to have trypsin-like specificity and preferably also be bleach stable. The precursor carbonyl hydrolase may be a naturally-occurring carbonyl hydrolase or recombinant hydrolase. Specifically, such carbonyl hydrolase variants have an amino acid sequence not found in nature, which is derived by replacement of a plurality of amino acid residues of a precursor carbonyl hydrolase with different amino acids. The plurality of amino acid residues of the precursor enzyme correspond to position +210 in combination with one or more of the following residues: +33, +62, +67, +76, +100, +101, +103, +104, +107, +128, +129, +130, +132, +135, +156, +158, +164, +166, +167, +170, +209, +215, +217, +218, and +222, where the numbered position corresponds to naturally-occurring subtilisin from *Bacillus amyloliquefaciens* or to equivalent amino acid residues in other carbonyl hydrolases or subtilisins, such as *Bacillus lentus* subtilisin.
The carbonyl hydrolase variants which are protease enzymes useful in the present invention compositions comprise replacement of amino acid residue +210 in combination with one or more additional modifications. While any combination of the above listed amino acid substitutions may be employed, the preferred variant protease enzymes useful for the present invention comprise the substitution, deletion or insertion of amino acid residues in the following combinations: 210/156; 210/166; 210/76; 210/103; 210/104; 210/217; 210/156/166; 210/156/217; 210/166/217; 210/76/156; 210/76/166; 210/76/217; 210/76/156/166; 210/76/156/217; 210/76/166/217; 210/76/103/156; 210/76/103/166; 210/76/103/217; 210176/104/156; 210/76/104/166: 210/76/104/217; 210/76/103/104/156; 210/76/103/104/166; 210/76/103/104/217; 210/76/103/104/156/166; 210/76/103/104/156/217; 210/76/1031104/166/217 and/or 210/76/103/104/156/166/217; 210/76/103/104/166/222; 210/67/76/103/104/166/222; 210/67176/103/104/166/218/222. Most preferably the variant enzymes useful for the present invention comprise the substitution, deletion or insertion of an amino acid residue in the following combination of residues: 210/156; 210/166; 210/217; 210/156/166; 210/156/217; 210/166/217; 210/76/156/166; 210/76/103/156/166 and 210/76/103/104/156/166 of *B. lentus* subtilisin with 210/76/103/104/156/166 being the most preferred.
Variant DNA sequences encoding such carbonyl hydrolase or subtilisin variants are derived from a precursor DNA sequence which encodes a naturally-occurring or recombinant precursor enzyme: The variant DNA sequences are derived by modifying the precursor DNA sequence to encode the substitution of one or more specific amino acid residues encoded by the precursor DNA sequence corresponding to positions +210, +33, +62, +67, +76, +100, +101, +103, +104, +107, +128, +129, +130, +132, +135, +156, +158, +164, +166, +167, +170, +209, +215, +217, +218, and +222 in *Bacillus lentus* or any combination thereof. Although the amino acid residues identified for modification herein are identified according to the numbering applicable to B. *amyloliquefaciens* (which has become the conventional method for identifying residue positions in all subtilisins), the preferred precursor DNA sequence useful for the present invention is the DNA sequence of *Bacillus lentus.* These recombinant DNA sequences encode carbonyl hydrolase variants having a novel amino acid sequence and, in general, at least one property which is substantially different from the same property of the enzyme encoded by the precursor carbonyl hydrolase DNA sequence. Such properties include proteolytic activity, substrate specificity, stability, altered pH profile and/or enhanced performance characteristics.
The protease enzymes useful herein encompass the substitution of any of the nineteen naturally occurring L-amino acids at the designated amino acid residue positions. Such substitutions can be made in any precursor subtilisin (procaryotic, eucaryotic, mammalian, etc.). Throughout this application reference is made to various amino acids by way of common one- and three-letter codes. Such codes are identified in Dale, M.W. (1989), Molecular Genetics of Bacteria, John Wiley & Sons, Ltd., Appendix B.
Preferably, the substitution to be made at each of the identified amino acid residue positions include but are not limited to substitutions at position +210 including I, V, L, and A, substitutions at positions +33, +62, +76, +100, +101, +103, +104, +107, +128, +129, +130, +132, +135, +156, +158, +164, +166, +167, +170, +209, +215, +217, and +218 of D or E, substitutions at position 76 including D, H, E, G, F, K, P and N; substitutions at position 103 including Q, T, D, E, Y, K, G, R and S; and substitutions at position 104 including S, Y, I, L, M, A, W, D, T, G and V; and substitutions at position 222 including S, C, A. The specifically preferred amino acid(s) to be substituted at each such position are designated below in Table I. Although specific amino acids are shown in Table I, it should be understood that any amino acid may be substituted at the identified residues.

**Table I**

| Amino Acid | Preferred Amino Acid to |
|---|---|
| Residue | be Substituted/Inserted |
| +210 | I, V, L, A |
| +33, +62, +100, +101, +107 | D,E |
| +128, +129, +130, +135 | |
| +156,+158,+164,+166 | |
| +167, +170, +209, +215 +217 and +218 | |
| +76 | D,H |
| +103 | A,Q,T,D,E,Y,K,G,R |
| +104 | I,Y,S,L,A,T,G |
| +222 | S, C, A |

A comparison of the preferred amino acid residues identified herein for substitution versus the preferred substitution for each such position is provided in Table II.

**Table II**

| | +210 | +156 | +166 | +217 | +76 | +103 | +104 |
|---|---|---|---|---|---|---|---|
| *B. amyloliquefaciens* (wild-type) | P | E | G | Y | N | Q | Y |
| *B. lentus* (wild-type) | P | S | S | L | N | S | V |
| Most Preferred Substitution | I | E/D | E/D | E/D | D | A | I/Y |

Also suitable for the present invention are proteases described in patent applications EP 251 446 and WO 91/06637, protease SLAP^{®} described in WO91/02792 and their variants described in WO 95/23221.
See also a high pH protease from Bacillus sp. NCIMB 40338 described in WO 93/18140 A to Novo. Enzymatic detergents comprising protease, one or more other enzymes, and a reversible protease inhibitor are described in WO 92/03529 A to Novo. When desired, a protease having decreased adsorption and increased hydrolysis is available as described in WO 95/07791 to Procter & Gamble. A recombinant trypsin-like protease for detergents suitable herein is described in WO 94/25583 to Novo. Other suitable proteases are described in EP 516 200 by Unilever.

Preferred proteases for the purpose of the present invention is a serine protease, more preferably a a bacterial serine protease obtained from *Bacillus,* preferably *Bacillus subtilis* and/or *Bacillus licheniformis.*

The proteolytic enzymes are incorporated in the detergent compositions of the present invention a level of from 0.0001% to 2%, preferably from 0.001% to 0.2%, more preferably from 0.005% to 0.1% pure enzyme by weight of the composition.

The cellulases usable in the present invention include both bacterial or fungal cellulases. Preferably, they will have a pH optimum of between 5 and 12 and an activity above 50 CEVU (Cellulose Viscosity Unit). Suitable cellulases are disclosed in U.S. Patent 4,435,307, Barbesgoard et al, J61078384 and WO96/02653 which discloses fungal cellulase produced respectively from Humicola insolens, Trichoderma, Thielavia and Sporotrichum. EP 739 982 describes cellulases isolated from novel Bacillus species. Suitable cellulases are also disclosed in GB-A-2.075.028; GB-A-2.095.275; DE-OS-2.247.832 and WO95/26398.
Examples of such cellulases are cellulases produced by a strain of Humicola insolens (Humicola grisea var. thermoidea), particularly the Humicola strain DSM 1800.
Other suitable cellulases are cellulases originated from Humicola insolens having a molecular weight of about 50KDa, an isoelectric point of 5.5 and containing 415 amino acids; and a ⁻43kD endoglucanase derived from Humicola insolens, DSM 1800, exhibiting cellulase activity; a preferred endoglucanase component has the amino acid sequence disclosed in PCT Patent Application No. WO 91/17243. Also suitable cellulases are the EGIII cellulases from Trichoderma longibrachiatum described in WO94/21801, Genencor, published September 29, 1994. Especially suitable cellulases are the cellulases having color care benefits. Examples of such cellulases are cellulases described in European patent application No. 91202879.2, filed November 6, 1991 (Novo). Carezyme and Celluzyme (Novo Nordisk A/S) are especially useful. See also WO91/17244 and WO91/21801. Other suitable cellulases for fabric care and/or cleaning properties are described in WO96/34092, WO96/17994 and WO95/24471.

Said cellulases are normally incorporated in the detergent composition at levels from 0.0001 % to 2% of active enzyme by weight of the detergent composition.

Other preferred enzymes that can be included in the detergent compositions of the present invention include lipases. Suitable lipase enzymes for detergent usage include those produced by microorganisms of the Pseudomonas group, such as Pseudomonas stutzeri ATCC 19.154, as disclosed in British Patent 1,372,034. Suitable lipases include those which show a positive immunological cross-reaction with the antibody of the lipase, produced by the microorganism *Pseudomonas fluorescent* IAM 1057. This lipase is available from Amano Pharmaceutical Co. Ltd., Nagoya, Japan, under the trade name Lipase P "Amano," hereinafter referred to as "Amano-P". Other suitable commercial lipases include Amano-CES, lipases ex *Chromobacter viscosum,* e.g. *Chromobacter viscosum var. lipolyticum* NRRLB 3673 from Toyo Jozo Co., Tagata, Japan; *Chromobacter viscosum* lipases from U.S. Biochemical Corp., U.S.A. and Disoynth Co., The Netherlands, and lipases ex *Pseudomonas* gladioli. Especially suitable lipases are lipases such as M1 Lipase^{R} and Lipomax^{R} (Gist-Brocades) and Lipolase^{R} and Lipolase Ultra^{R}(Novo) which have found to be very effective when used in combination with the compositions of the present invention. Also suitables are the lipolytic enzymes described in EP 258 068, WO 92/05249 and WO 95/22615 by Novo Nordisk and in WO 94/03578, WO 95/35381 and WO 96/00292 by Unilever.
Also suitable are cutinases [EC 3.1.1.50] which can be considered as a special kind of lipase, namely lipases which do not require interfacial activation. Addition of cutinases to detergent compositions have been described in e.g. WO-A-88/09367 (Genencor); WO 90/09446 (Plant Genetic System) and WO 94/14963 and WO 94/14964 (Unilever).
The lipases and/or cutinases are normally incorporated in the detergent composition at levels from 0.0001% to 2% of active enzyme by weight of the detergent composition.

Amylases (α and/or β) can be included for removal of carbohydrate-based stains. WO94/02597, Novo Nordisk A/S published February 03, 1994, describes cleaning compositions which incorporate mutant amylases. See also WO95/10603, Novo Nordisk A/S, published April 20, 1995. Other amylases known for use in cleaning compositions include both α- and β-amylases. α-Amylases are known in the art and include those disclosed in US Pat. no. 5,003,257; EP 252,666; WO/91/00353; FR 2,676,456; EP 285,123; EP 525,610; EP 368,341; and British Patent specification no. 1,296,839 (Novo). Other suitable amylases are stability-enhanced amylases described in WO94/18314, published August 18, 1994 and WO96/05295, Genencor, published February 22, 1996 and amylase variants having additional modification in the immediate parent available from Novo Nordisk A/S, disclosed in WO 95/10603, published April 95. Also suitable are amylases described in EP 277 216, WO95/26397 and WO96/23873 (all by Novo Nordisk).
Examples of commercial α-amylases products are Purafect Ox Am^{®} from Genencor and Termamyl^{®}, Ban^{®} Fungamyl^{®} and Duramyl^{®}, all available from Novo Nordisk A/S Denmark. WO95/26397 describes other suitable amylases : α-amylases characterised by having a specific activity at least 25% higher than the specific activity of Termamyl^{®} at a temperature range of 25°C to 55°C and at a pH value in the range of 8 to 10, measured by the Phadebas^{®} α-amylase activity assay. Suitable are variants of the above enzymes, described in WO96/23873 (Novo Nordisk). Other amylolytic enzymes with improved properties with respect to the activity level and the combination of thermostability and a higher activity level are described in WO95/35382.
The amylolytic enzymes are incorporated in the detergent compositions of the present invention a level of from 0.0001 % to 2%, preferably from 0.00018% to 0.06%, more preferably from 0.00024% to 0.048% pure enzyme by weight of the composition.

The above-mentioned enzymes may be of any suitable origin, such as vegetable, animal, bacterial, fungal and yeast origin. Origin can further be mesophilic or extremophilic (psychrophilic, psychrotrophic, thermophilic, barophilic, alkalophilic, acidophilic, halophilic, etc.). Purified or non-purified forms of these enzymes may be used. Also included by definition, are mutants of native enzymes. Mutants can be obtained e.g. by protein and/or genetic engineering, chemical and/or physical modifications of native enzymes. Common practice as well is the expression of the enzyme via host organisms in which the genetic material responsible for the production of the enzyme has been cloned.

Said enzymes are normally incorporated in the detergent composition at levels from 0.0001% to 2% of active enzyme by weight of the detergent composition. The enzymes can be added as separate single ingredients (prills, granulates, stabilized liquids, etc... containing one enzyme) or as mixtures of two or more enzymes ( e.g. cogranulates ).

Other suitable detergent ingredients that can be added are enzyme oxidation scavengers which are described in EP 533 607 filed on January 31, 1992. Examples of such enzyme oxidation scavengers are ethoxylated tetraethylene polyamines.

A range of enzyme materials and means for their incorporation into synthetic detergent compositions is also disclosed in WO 9307263 A and WO 9307260 A to Genencor International, WO 8908694 A to Novo, and U.S. 3,553,139, January 5, 1971 to McCarty et al. Enzymes are further disclosed in U.S. 4,101,457, Place et al, July 18, 1978, and in U.S. 4,507,219, Hughes, March 26, 1985. Enzyme materials useful for liquid detergent formulations, and their incorporation into such formulations, are disclosed in U.S. 4,261,868, Hora et al, April 14, 1981. Enzymes for use in detergents can be stabilised by various techniques. Enzyme stabilisation techniques are disclosed and exemplified in U.S. 3,600,319, August 17, 1971, Gedge et al, EP 199,405 and EP 200,586, October 29, 1986, Venegas. Enzyme stabilisation systems are also described, for example, in U.S. 3,519,570. A useful Bacillus, sp. AC13 giving proteases, xylanases and cellulases, is described in WO 9401532 A to Novo.

### Bleaching agent

Preferred additional optional detergent ingredients that can be included in the cleaning compositions of the present invention include conventional activated-, other enzymatic- and/or metallo catalyst- based bleach systems. It has been found that the combination of said specific oxygenase with another bleach system provides improved cleaning of proteic based stains and/or soils such as protein containing food stains/soils and everyday body soils and when formulated as laundry composition, improved fabric realistic items cleaning and whitening while providing colour safety.

The bleaching agent component for use herein can be any of the bleaching agents useful for cleaning compositions including oxygen bleaches as well as others known in the art. The bleaching agent suitable for the present invention can be an activated or non-activated bleaching agent.

Bleaching agents are such as hydrogen peroxide, PB1, PB4 and percarbonate with a particle size of 400-800 microns. These bleaching agent components can include one or more oxygen bleaching agents and, depending upon the bleaching agent chosen, one or more bleach activators. When present oxygen bleaching compounds will typically be present at levels of from about 1% to about 25%.

The hydrogen peroxide releasing agents can be used in combination with bleach activators such as tetraacetylethylenediamine (TAED), nonanoyloxybenzene-sulfonate (NOBS, described in US 4,412,934), 3,5,-trimethylhexanoloxybenzenesulfonate (ISONOBS, described in EP 120,591) or pentaacetylglucose (PAG)or Phenolsulfonate ester of N-nonanoyl-6-aminocaproic acid (NACA-OBS, described in WO94/28106), which are perhydrolyzed to form a peracid as the active bleaching species, leading to improved bleaching effect. Also suitable activators are acylated citrate esters and unsymetrical acyclic imide bleach activator of the following formula as disclosed in the Procter & Gamble WO 98/04664 : 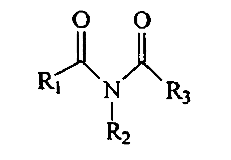 wherein R₁ is a C₇-C₁₃ linear or branched chain saturated or unsaturated alkyl group, R₂ is a C₁-C₈, linear or branched chain saturated or unsaturated alkyl group and R₃ is a C₁-C₄ linear or branched chain saturated or unsaturated alkyl group.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid. Such bleaching agents are disclosed in U.S. Patent 4,483,781, U.S. Patent Application 740,446, European Patent Application 0,133,354 and U.S. Patent 4,412,934. Highly preferred bleaching agents also include 6-nonylamino-6-oxoperoxycaproic acid as described in U.S. Patent 4,634,551.
Another category of bleaching agents that can be used encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight.

Useful bleaching agents, including peroxyacids and bleaching systems comprising bleach activators and peroxygen bleaching compounds for use in detergent compositions according to the invention are described in our co-pending applications WO 85/10592, WO 97/00937, WO95/27772. WO95/27773, WO95/27774 and WO95/27775.

The hydrogen peroxide may also be present by adding an enzymatic system (i.e. an enzyme and a substrate therefore) which is capable of generating hydrogen peroxide at the beginning or during the washing and/or rinsing process. Such enzymatic systems are disclosed in EP 537 381 filed October 9, 1991.
Peroxidase enzymes are used in combination with oxygen, hydrogen peroxide sources, e.g. percarbonate, perborate, persulfate, hydrogen peroxide, etc and a bleach enhancer. They are used for "solution bleaching", i.e. to prevent transfer of dyes or pigments removed from substrates during wash operations to other substrates in the wash solution. Peroxidase enzymes are known in the art, and include, for example, horseradish peroxidase, ligninase and haloperoxidase such as chloro- and bromo-peroxidase. Peroxidase-containing detergent compositions are disclosed, for example, in PCT International Application WO 89/099813. WO89/09813 and in European Patent application EP 540 784 filed on November 6, 1991. Also suitable is the laccase enzyme.
Enhancers are generally comprised at a level of from 0.1% to 5% by weight of total composition. Preferred enhancers are substitued phenthiazine and phenoxazine 10-Phenothiazinepropionicacid (PPT), 10-ethylphenothiazine-4-carboxylic acid (EPC), 10-phenoxazinepropionic acid (POP) and 10-methylphenoxazine (described in WO 94/12621) and substitued syringates (C3-C5 substitued alkyl syringates) and phenols. Sodium percarbonate or perborate are preferred sources of hydrogen peroxide.
Said peroxidases are normally incorporated in the detergent composition at levels from 0.0001% to 2% of active enzyme by weight of the detergent composition.

Metal-containing catalysts for use in bleach compositions, include cobalt-containing catalysts such as Pentaamine acetate cobalt(III) salts and manganese-containing catalysts such as those described in EPA 549 271; EPA 549 272; EPA 458 397; US 5,246,621; EPA 458 398; EPA 458 397; US 5,194,416 and US 5,114,611. Bleaching composition comprising a peroxy compound, a manganese-containing bleach catalyst and a chelating agent is described in the patent application No 94870206.3 (EP-A-0718398).
Preferred metal-contaning catalyst for the purpose of the present invention is a transition metal complex of a macropolycyclic rigid ligand. The phrase "macropolycyclic rigid ligand" is sometimes abbreviated as "MRL" in discussion below. The amount used is a catalytically effective amount, suitably about 1 ppb or more, for example up to about 99.9%, more typically about 0.001 ppm or more, preferably from about 0.05 ppm to about 500 ppm (wherein "ppb" denotes parts per billion by weight and "ppm" denotes parts per million by weight).
Suitable transition metals e.g., Mn are illustrated hereinafter. "Macropolycyclic" means a MRL is both a macrocycle and is polycyclic. "Polycyclic" means at least bicyclic. The term "rigid" as used herein herein includes "having a superstructure" and "cross-bridged". "Rigid" has been defined as the constrained converse of flexibility: see D.H. Busch., Chemical Reviews., (1993), 93, 847-860, incorporated by reference. More particularly, "rigid" as used herein means that the MRL must be determinably more rigid than a macrocycle ("parent macrocycle") which is otherwise identical (having the same ring size and type and number of atoms in the main ring) but lacking a superstructure (especially linking moieties or, preferably cross-bridging moieties) found in the MRL's. In determining the comparative rigidity of macrocycles with and without superstructures, the practitioner will use the free form (not the metal-bound form) of the macrocycles. Rigidity is well-known to be useful in comparing macrocycles; suitable tools for determining, measuring or comparing rigidity include computational methods (see, for example, Zimmer, Chemical Reviews, (1995), 95(38), 2629-2648 or Hancock et al., Inorganica Chimica Acta, (1989), 164, 73-84. A determination of whether one macrocycle is more rigid than another can be often made by simply making a molecular model, thus it is not in general essential to know configurational energies in absolute terms or to precisely compute them. Excellent comparative determinations of rigidity of one macrocycle vs. another can be made using inexpensive personal computer-based computational tools, such as ALCHEMY III, commercially available from Tripos Associates. Tripos also has available more expensive software permitting not only comparative, but absolute determinations; alternately, SHAPES can be used (see Zimmer cited supra). One observation which is significant in the context of the present invention is that there is an optimum for the present purposes when the parent macrocycle is distinctly flexible as compared to the cross-bridged form. Thus, unexpectedly, it is preferred to use parent macrocycles containing at least four donor atoms, such as cyclam derivatives, and to cross-bridge them, rather than to start with a more rigid parent macrocycle. Another observation is that cross-bridged macrocycles are significantly preferred over macrocycles which are bridged in other manners.
Preferred MRL's herein are a special type of ultra-rigid ligand which is cross-bridged. A "cross-bridge" is nonlimitingly illustrated in 1.11 hereinbelow. In 1.11, the cross-bridge is a -CH₂CH₂- moiety. It bridges N¹ and N⁸ in the illustrative structure. By comparison, a "same-side" bridge, for example if one were to be introduced across N¹ and N¹² in 1.11, would not be sufficient to constitute a "cross-bridge" and accordingly would not be preferred.
Suitable metals in the rigid ligand complexes include Mn(II), Mn(III), Mn(IV), Mn(V), Fe(II), Fe(III), Fe(IV), Co(I), Co(II), Co(III), Ni(I), Ni(II), Ni(III), Cu(I), Cu(II), Cu(III), Cr(II), Cr(III), Cr(IV), Cr(V), Cr(VI), V(III), V(IV), V(V), Mo(IV), Mo(V), Mo(VI), W(IV), W(V), W(VI), Pd(II), Ru(II), Ru(III), and Ru(IV). Preferred transition-metals in the instant transition-metal bleach catalyst include manganese, iron and chromium. Preferred oxidation states include the (II) and (III) oxidation states. Manganese(II) in both the low-spin configuration and high spin complexes are included. It is to be noted that complexes such as low-spin Mn(II) complexes are rather rare in all of coordination chemistry. The designation (II) or (III) denotes a coordinated transition metal having the requisite oxidation state; the coordinated metal atom is not a free ion or one having only water as a ligand.
In general, as used herein, a "ligand" is any moiety capable of direct covalent bonding to a metal ion. Ligands can be charged or neutral and may range widely, including simple monovalent donors, such as chloride, or simple amines which form a single coordinate bond and a single point of attachment to a metal; to oxygen or ethylene, which can form a three-membered ring with a metal and thus can be said to have two potential points of attachment, to larger moieties such as ethylenediamine or aza macrocycles, which form up to the maximum number of single bonds to one or more metals that are allowed by the available sites on the metal and the number of lone pairs or alternate bonding sites of the free ligand. Numerous ligands. can form bonds other than simple donor bonds, and can have multiple points of attachment.
Ligands useful herein can fall into several groups: the MRL, preferably a cross-bridged macropolycycle (preferably there will be one MRL in a useful transition-metal complex, but more, for example two, can be present, but not in preferred mononuclear transition-metal complexes); other, optional ligands, which in general are different from the MRL (generally there will be from 0 to 4, preferably from 1 to 3 such ligands); and ligands associated transiently with the metal as part of the catalytic cycle, these latter typically being related to water, hydroxide, oxygen or peroxides. Ligands of the third group are not essential for defining the metal bleach catalyst, which is a stable, isolable chemical compound that can be fully characterized. Ligands which bind to metals through donor atoms each having at least a single lone pair of electrons available for donation to a metal have a donor capability, or potential denticity, at least equal to the number of donor atoms. In general, that donor capability may be fully or only partially exercised.
Generally, the MRL's herein can be viewed as the result of imposing additional structural rigidity on specifically selected "parent macrocycles".
More generally, the MRL's (and the corresponding transition-metal catalysts) herein suitably comprise:
(a) at least one macrocycle main ring comprising four or more heteroatoms; and
(b) a covalently connected non-metal superstructure capable of increasing the rigidity of the macrocycle, preferably selected from
   (i) a bridging superstructure, such as a linking moiety;
   (ii) a cross-bridging superstructure, such as a cross-bridging linking moiety; and
   (iii) combinations thereof.
The term "superstructure" is used herein as defined in the literature by Busch et al., see, for example, articles by Busch in "Chemical Reviews".
Preferred superstructures herein not only enhance the rigidity of the parent macrocycle, but also favor folding of the macrocycle so that it co-ordinates to a metal in a cleft. Suitable superstructures can be remarkably simple, for example a linking moiety such as any of those illustrated in 1.9 and 1.10 below, can be used. 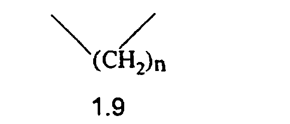 wherein n is an integer, for example from 2 to 8, preferably less than 6, typically 2 to 4, or 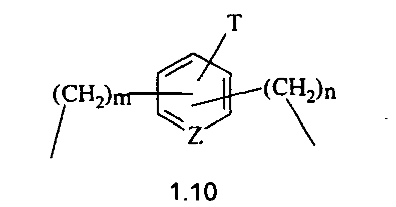 wherein m and n are integers from about 1 to 8, more preferably from 1 to 3; Z is N or CH; and T is a compatible substituent, for example H, alkyl, trialkylammonium, halogen, nitro, sulfonate, or the like. The aromatic ring in 1.10 can be replaced by a saturated ring, in which the atom in Z connecting into the ring can contain N, O, S or C.
Without intending to be limited by theory, it is believed that the preorganization built into the MRL's herein that leads to extra kinetic and/or thermodynamic stability of their metal complexes arises from either or both of topological constraints and enhanced rigidity (loss of flexibility) compared to the free parent macrocycle which has no superstructure. The MRL's as defined herein and their preferred cross-bridged sub-family, which can be said to be "ultra-rigid", combine two sources of fixed preorganization. In preferred MRL's herein, the linking moieties and parent macrocycle rings are combined to form ligands which have a significant extent of "fold", typically greater than in many known superstructured ligands in which a superstructure is attached to a largely planar, often unsaturated macrocycle. See, for example: D.H. Busch, Chemical Reviews. (1993), 93, 847 - 880. Further, the preferred MRL's herein have a number of particular properties, including (1) they are characterized by very high proton affinities, as in so-called "proton sponges"; (2) they tend to react slowly with multivalent transition metals, which when combined with (1) above, renders synthesis of their complexes with certain hydrolyzable metal ions difficult in hydroxylic solvents; (3) when they are coordinated to transition metal atoms as identified herein, the MRL's result in complexes that have exceptional kinetic stability such that the metal ions only dissociate extremely slowly under conditions that would destroy complexes with ordinary ligands; and (4) these complexes have exceptional thermodynamic stability; however, the unusual kinetics of MRL dissociation from the transition metal may defeat conventional equilibrium measurements that might quantitate this property.
In one aspect of the present invention, the MRL's include those comprising:
(i) an organic macrocycle ring containing four or more donor atoms (preferably at least 3, more preferably at least 4, of these donor atoms are N) separated from each other by covalent linkages of at least one, preferably 2 or 3, non-donor atoms, two to five (preferably three to four, more preferably four) of these donor atoms being coordinated to the same transition metal in the complex; and
(ii) a linking moiety, preferably a cross-bridging chain, which covalently connects at least 2 (preferably non-adjacent) donor atoms of the organic macrocycle ring, said covalently connected (preferably non-adjacent) donor atoms being bridgehead donor atoms which are coordinated to the same transition metal in the complex, and wherein said linking moiety (preferably a cross-bridged chain) comprises from 2 to about 10 atoms (preferably the cross-bridged chain is selected from 2, 3 or 4 non-donor atoms, and 4-6 non-donor atoms with a further donor atom).
   Suitable MRL's are further nonlimitingly illustrated by the following compound:

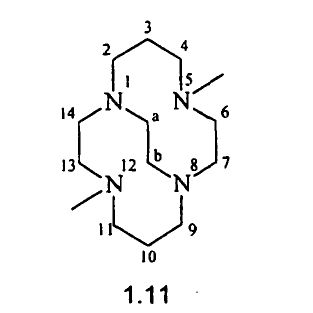

This is a MRL in accordance with the invention which is a highly preferred, cross-bridged, methyl-substituted (all nitrogen atoms tertiary) derivative of cyclam. Formally, this ligand is named 5,12-dimethyt-1.5,8,12-tetraazabicyclo[6.6.2]hexadecane using the extended von Baeyer system. See "A Guide to IUPAC Nomenclature of Organic Compounds: Recommendations 1993", R. Panico, W.H. Powell and J-C Richer (Eds.), Blackwell Scientific Publications, Boston, 1993; see especially section R-2.4.2.1. According to conventional terminology, N1 and N8 are "bridgehead atoms"; as defined herein, more particularly "bridgehead donor atoms" since they have lone pairs capable of donation to a metal. N1 is connected to two non-bridgehead donor atoms, N5 and N12, by distinct saturated carbon chains 2,3,4 and 14,13 and to bridgehead donor atom N8 by a "linking moiety" a,b which here is a saturated carbon chain of two carbon atoms. N8 is connected to two non-bridgehead donor atoms, N5 and N12, by distinct chains 6,7 and 9,10,11. Chain a,b is a "linking moiety" as defined herein, and is of the special, preferred type referred to as a "cross-bridging" moiety. The "macrocyclic ring" of the ligand supra, or "main ring" (IUPAC), includes all four donor atoms and chains 2,3,4; 6,7; 9,10,11 and 13,14 but not a,b. This ligand is conventionally bicyclic. The short bridge or "linking moiety" a,b is a "cross-bridge" as defined herein, with a,b bisecting the macrocyclic ring.
The MRL's herein are of course not limited to being synthesized from any preformed macrocycle plus preformed "rigidizing" or "conformation-modifying" element: rather, a wide variety of synthetic means, such as template syntheses, are useful. See for example Busch et al., reviewed in "Heterocyclic compounds: Aza-crown macrocycles", J.S. Bradshaw et. al.
Transition-metal bleach catalysts useful in the invention compositions can in general include known compounds where they conform with the definition herein, as well as, more preferably, any of a large number of novel compounds expressly designed for the present laundry or cleaning uses, and non-limitingly illustrated by any of the following:
Dichloro-5,12-dimethyl-1,5,8,12-tetraazabicyclo[6.6.2]hexadecane Manganese(II)
Dichloro-4,10-dimethyl-1,4,7,10-tetraazabicyclo[5.5.2]tetradecane Manganese(II)
Diaquo-5,12-dimethyl-1,5,8,12-tetraazabicyclo[6.6.2]hexadecane Manganese(II) Hexafluorophosphate
Aquo-hydroxy-5,12-dimethyl-1,5, 8,12-tetraazabicyclo[6.6.2]hexadecane Manganese(III) Hexafluorophosphate
Diaquo-4,10-dimethyl-1,4,7,10-tetraazabicyclo[5.5.2]tetradecane Manganese(II) Hexafluorophosphate
Diaquo-5,12-dimethyl-1,5,8,12-tetraazabicyclo[6.6.2]hexadecane Manganese(II) Tetrafluoroborate
Diaquo-4,10-dimethyl-1,4,7,10-tetraazabicyclo[5.5.2]tetradecane Manganese(II) Tetrafluoroborate
Dichloro-5,12-dimethyl-1,5,8,12-tetraazabicyclo[6.6.2]hexadecane Manganese(III) Hexafluorophosphate
Dichloro-5,12-di-n-butyl-1,5,8,12-tetraaza- bicyclo[6.6.2]hexadecane Manganese(II)
Dichloro-5,12-dibenzyl-1,5,8,12-tetraazabicyclo[6.6.2]hexadecane Manganese(II)
Dichloro-5-n-butyl-12-methyl-1,5,8,12-tetraaza- bicyclo[6.6.2]hexadecane Manganese(II)
Dichloro-5-n-octyl-12-methyl-1,5,8,12-tetraaza- bicyclo[6.6.2]hexadecane Manganese(II)
Dichloro-5-n-butyl-12-methyl-1,5,8,12-tetraaza- bicyclo[6.6.2]hexadecane Manganese(II)
Dichloro-5,12-dimethyl-1,5,8,12-tetraazabicyclo[6.6.2]hexadecane Iron(II)
Dichloro-4,10-dimethyl-1,4,7,10-tetraazabicyclo[5.5.2]tetradecane Iron(II)
Dichloro-5,12-dimethyl-1,5,8,12-tetraazabicyclo[6.6.2]hexadecane Copper(II)
Dichloro-4,10-dimethyl-1,4,7,10-tetraazabicyclo[5.5.2]tetradecane Copper(II)
Dichloro-5,12-dimethyl-1,5,8,12-tetraazabicyclo[6.6.2]hexadecane Cobalt(II)
Dichloro-4,10-dimethyl-1,4,7,10-tetraazabicyclo[5.5.2]tetradecane Cobalt(II)
Dichloro 5,12-dimethyl-4-phenyl-1,5,8,12-tetraazabicyclo[6.6.2]hexadecane Manganese(II)
Dichloro-4,10-dimethyl-3-phenyl-1,4,7,10-tetraazabicycio[5.5.2]tetradecane Manganese(II)
Dichloro-5,12-dimethyl-4,9-diphenyl-1,5,8,12-tetraazabicyclo[6.6.2]hexadecane Manganese(II)
Dichloro-4,10-dimethyl-3,8-diphenyl-1,4,7,10-tetraazabicyclo[5.5.2]tetradecane Manganese(II)
Dichloro-5,12-dimethyl-2,11-diphenyl-1, 5,8,12-tetraazabicyclo[6.6.2]hexadecane Manganese(II)
Dichloro-4,10-dimethyl-4,9-diphenyl-1,4,7,10-tetraazabicyclo[5.5.2]tetradecane Manganese(II)
Dichloro-2,4,5,9,11,12-hexamethyl-1,5,8,12-tetraazabicyclo[6.6.2]hexadecane Manganese(II)
Dichloro-2,3,5,9,10,12-hexamethyl-1,5,8,12-tetraazabicyclo[6.6.2]hexadecane Manganese(II)
Dichloro-2,2,4,5,9,9,11,12-octamethyl-1,5,8,12-tetraazabicyclo[6.6.2]hexadecane Manganese(II)
Dichloro-2,2,4,5,9,11,11,12-octamethyl-1,5,8,12-tetraazabicyclo[6.6.2]hexadecane Manganese(II)
Dichloro-3,3,5,10,10,12-hexamethyl-1,5,8,12-tetraazabicyclo[6.6.2]hexadecane Manganese(II)
Dichloro-3,5,10,12-tetramethyl-1,5,8,12-tetraazabicyclo[6.6.2]hexadecane Manganese(II)
Dichloro-3-butyl-5,10,12-trimethyl-1,5,8,12-tetraazabicyclo[6.6.2]hexadecane Manganese(II)
Dichloro-1,5,8,12-tetraazabicyclo[6.6.2]hexadecane Manganese(II)
Dichloro-1,4,7,10-tetraazabicyclo[5.5.2]tetradecane Manganese(II)
Dichloro-1,5,8,12-tetraazabicyclo[6.6.2]hexadecane Iron(II)
Dichloro-1,4,7,10-tetraazabicyclo[5.5.2]tetradecane Iron(II)
Aquo-chloro-2-(2-hydroxyphenyl)-5,12-dimethy1,5,8,12-tetraazabicyclo[6.6.2]hexadecane Manganese(II)
Aquo-chloro-10-(2-hydroxybenzyl)-4,10-dimethyl-1,4,7,10-tetraazabicyclo[5.5.2]tetradecane Manganese(II)
Chloro-2-(2-hydroxybenzyl)-5-methy1,5,8,12-tetraazabicyclo[6.6.2]hexadecane Manganese(II)
Chloro-10-(2-hydroxybenzyl)-4-methyl-1,4,7, 10-tetraazabicyclo[5.5.2]tetradecane Manganese(II)
Chloro-5-methyl-12-(2-picolyl)-1,5,8,12-tetraazabicyclo[6.6.2]hexadecane Manganese(II) Chloride
Chloro-4-methyl-10-(2-picolyl)-1,4,7,10-tetraazabicyclo[5.5.2]tetradecane Manganese(II) Chloride
Dichloro-5-(2-sulfato)dodecyl-12-methyl-1,5,8,12-tetraazabicyclo[6.6.2]hexadecane Manganese(III)
Aquo-Chloro-5-(2-sulfato)dodecyl-12-methyl-1,5,8,12-tetraazabicyclo[6.6.2]hexadecane Manganese(II)
Aquo-Chloro-5-(3-sulfonopropyl)-12-methyl-1,5,8,12-tetraazabicyclo[6.6.2]hexadecane Manganese(II)
Dichloro-5-(Trimethylammoniopropyl)dodecyl-12-methyl-1,5,8,12-tetraazabicyclo[6.6.2]hexadecane Manganese(III) Chloride
Dichloro-5,12-dimethyl-1,4,7,10,13-pentaazabicyclo[8.5.2]heptadecane Manganese(II)
Dichloro-14,20-dimethyl-1,10,14,20-tetraazatriyclo[8.6.6]docosa-3(8),4,6-triene Manganese(II)
Dichloro-4,11-dimethyl-1,4,7,11-tetraazabicyclo[6.5.2]pentadecane Manganese(II)
Dichloro-5,12-dimethyl-1,5,8,12-tetraazabicyclo[7.6.2]heptadecane Manganese(II)
Dichloro-5,13-dimethyl-1,5,9,13-tetraazabicyclo[7.7.2]heptadecane Manganese(II)
Dichloro-3,10-bis(butylcarboxy)-5,12-dimethyl-1,5,8,12-tetraazabicyclo[6.6.2]hexadecane Manganese(II)
Diaquo-3,10-dicarboxy-5,12-dimethyl-1,5,8,12-tetraazabicycto[6.6.2]hexadecane Manganese(II)
Chloro-20-methyl-1,9,20,24,25-pentaaza-tetracyclo[7.7.7.1^{3,7}.1^{11,15}.]pentacosa-3,5,7(24),11,13,15(25)-hexaene Manganese(II) Hexafluorophosphate
Trifluoromethanesulfono-20-methyl-1,9,20,24,25-pentaaza-tetracyclo[7.7.7.1^{3,7}.1^{11,15}.]pentacosa-3,5,7(24),11,13,15(25)-hexaene Manganese(II) Trifluoromethanesulfonate
Trifluoromethanesulfono-20-methyl-1,9,20,24,25-pentaaza-tetracyclo[7.7.7.1^{3,7}.1^{11,15}.]pentacosa-3,5,7(24),11,13,15(25)-hexaene Iron(II) Trifluoromethanesulfonate
Chloro-5,12,17-trimethyl-1,5,8,12,17-pentaazabicyclo[6.6.5]nonadecane Manganese(II) Hexafluorophosphate
Chloro-4,10,15-trimethyl-1,4,7,10,15-pentaazabicyclo[5.5.5]heptadecane Manganese(II) Hexafluorophosphate
Chloro-5,12,17-trimethyl-1,5,8,12,17-pentaazabicyclo[6.6.5]nonadecane Manganese(II) Chloride
Chloro-4,10,15-trimethyl-1,4,7,10,15-pentaazabicyclo[5.5.5]heptadecane Manganese(II) Chloride

The practitioner may further benefit if certain terms receive additional definition and illustration. As used herein, "macrocyclic rings" are covalently connected rings formed from four or more donor atoms (i.e., heteroatoms such as nitrogen or oxygen) with carbon chains connecting them, and any macrocycle ring as defined herein must contain a total of at least ten, preferably at least twelve, atoms in the macrocycle ring. A MRL herein may contain more than one ring of any sort per ligand, but at least one macrocycle ring must be identifiable. Moreover, in the preferred embodiments, no two hetero-atoms are directly connected. Preferred transition-metal bleach catalysts are those wherein the MRL comprises an organic macrocycle ring (main ring) containing at least 10-20 atoms, preferably 12-18 atoms, more preferably from about 12 to about 20 atoms, most preferably 12 to 16 atoms.
"Donor atoms" herein are heteroatoms such as nitrogen, oxygen, phosphorus or sulfur, which when incorporated into a ligand still have at least one lone pair of electrons available for forming a donor-acceptor bond with a metal. Preferred transition-metal bleach catalysts are those wherein the donor atoms in the organic macrocycle ring of the cross-bridged MRL are selected from the group consisting of N, O, S, and P, preferably N and O, and most preferably all N. Also preferred are cross-bridged MRL's comprising 4 or 5 donor atoms; all of which are coordinated to the same transition metal. Most preferred transition-metal bleach catalysts are those wherein the cross-bridged MRL comprises 4 nitrogen donor atoms all coordinated to the same transition metal, and those wherein the cross-bridged MRL comprises 5 nitrogen atoms all coordinated to the same transition metal.
"Non-donor atoms" of the MRL herein are most commonly carbon, though a number of atom types can be included, especially in optional exocyclic substituents (such as "pendant" moieties, illustrated hereinafter) of the macrocycles, which are neither donor atoms for purposes essential to form the metal catalysts, nor are they carbon. Thus, in the broadest sense, the term "non-donor atoms" can refer to any atom not essential to forming donor bonds with the metal of the catalyst. Examples of such atoms could include heteroatoms such as sulfur as incorporated in a non-coordinatable sulfonate group, phosphorus as incorporated into a phosphonium salt moiety, phosphorus as incorporated into a P(V) oxide, a non-transition metal, or the like. In certain preferred embodiments, all non-donor atoms are carbon.

Transition metal complexes of MRL's can be prepared in any convenient manner. Two such preparations are illustrated as follows:

### Synthesis of [Mn(Bcyclam)Cl₂]

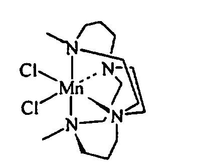

### (a) Method I.

"Bcyclam" (5,12-dimethyl-1,5,8,12-tetraaza-bicyclo[6.6.2]hexadecane) is prepared by a synthesis method described by G.R. Weisman, et al., J.Amer.Chem.Soc., (1990), 112, 8604. Bcyclam (1.00 g., 3.93 mmol) is dissolved in dry CH₃CN (35 mL, distilled from CaH₂). The solution is then evacuated at 15 mm until the CH₃CN begins to boil. The flask is then brought to atmospheric pressure with Ar. This degassing procedure is repeated 4 times. Mn(pyridine)₂Cl₂ (1.12 g., 3.93 mmol), synthesized according to the literature procedure of H. T. Witteveen et al., J. Inorg. Nucl. Chem., (1974), 36, 1535, is added under Ar. The cloudy reaction solution slowly begins to darken. After stirring overnight at room temperature, the reaction solution becomes dark brown with suspended fine particulates. The reaction solution is filtered with a 0.2µ filter. The filtrate is a light tan color. This filtrate is evaporated to dryness using a rotoevaporator. After drying overnight at 0.05 mm at room temperature, 1.35 g. off-white solid product is collected, 90% yield. Elemental Analysis: %Mn, 14.45; %C, 44.22; %H, 7.95; theoretical for [Mn(Bcyclam)Cl₂], MnC₁₄H₃₀N₄Cl₂, MW = 380.26. Found: %Mn, 14.98; %C, 44.48; %H, 7.86; Ion Spray Mass Spectroscopy shows one major peak at 354 mu corresponding to [Mn(Bcyclam)(formate)]⁺.

### (b) Method II.

Freshly distilled Bcyclam (25.00 g., 0.0984 mol), which is prepared by the same method as above, is dissolved in dry CH₃CN (900 mL, distilled from CaH₂). The solution is then evacuated at 15 mm until the CH₃CN begins to boil. The flask is then brought to atmospheric pressure with Ar. This degassing procedure is repeated 4 times. MnCl₂ (11.25 g., 0.0894 mol) is added under Ar. The cloudy reaction solution immediately darkens. After stirring 4 hrs. under reflux, the reaction solution becomes dark brown with suspended fine particulates. The reaction solution is filtered through a 0.2µ filter under dry conditions. The filtrate is a light tan color. This filtrate is evaporated to dryness using a rotoevaporator. The resulting tan solid is dried overnight at 0.05 mm at room temperature. The solid is suspended in toluene (100 mL) and heated to reflux. The toluene is decanted off and the procedure is repeated with another 100 mL of toluene. The balance of the toluene is removed using a rotoevaporator. After drying overnight at.05 mm at room temperature, 31.75 g. of a light blue solid product is collected, 93.5% yield. Elemental Analysis: %Mn, 14.45; %C, 44.22; %H, 7.95; %N, 14.73; %Cl, 18.65; theoretical for [Mn(Bcyclam)Cl₂], MnC₁₄H₃ON₄Cl₂, MW = 380.26. Found: %Mn, 14.69; %C, 44.69; %H, 7.99; %N, 14.78; %Cl, 18.90 (Karl Fischer Water, 0.68%). lon Spray Mass Spectroscopy shows one major peak at 354 mu corresponding to [Mn(Bcyclam)(formate)]⁺.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in U.S. Patent 4,033,718. Typically, detergent compositions will contain about 0.025% to about 1.25%, by weight, of sulfonated zinc phthalocyanine.

### Surfactant system

The cleaning compositions according to the present invention comprise a surfactant system wherein the surfactant can be selected from nonionic and/or anionic and/or cationic and/or ampholytic and/or zwitterionic and/or semi-polar surfactants.

The surfactant is typically present at a level of from 0.1 % to 60% by weight. More preferred levels of incorporation are 1% to 35% by weight, most preferably from 1% to 30% by weight of cleaning compositions in accord with the invention.

The surfactant is preferably formulated to be compatible with enzyme components present in the composition. In liquid or gel compositions the surfactant is most preferably formulated such that it promotes, or at least does not degrade, the stability of any enzyme in these compositions.

Preferred surfactant systems to be used according to the present invention comprise as a surfactant one or more of the nonionic and/or anionic surfactants described herein.

Polyethylene, polypropylene, and polybutylene oxide condensates of alkyl phenols are suitable for use as the nonionic surfactant of the surfactant systems of the present invention, with the polyethylene oxide condensates being preferred. These compounds include the condensation products of alkyl phenols having an alkyl group containing from about 6 to about 14 carbon atoms, preferably from about 8 to about 14 carbon atoms, in either a straight-chain or branched-chain configuration with the alkylene oxide. In a preferred embodiment, the ethylene oxide is present in an amount equal to from about 2 to about 25 moles, more preferably from about 3 to about 15 moles, of ethylene oxide per mole of alkyl phenol. Commercially available nonionic surfactants of this type include Igepal^{™} CO-630, marketed by the GAF Corporation; and Triton^{™} X-45, X-114, X-100 and X-102, all marketed by the Rohm & Haas Company. These surfactants are commonly referred to as alkylphenol alkoxylates (e.g., alkyl phenol ethoxylates).

The condensation products of primary and secondary aliphatic alcohols with from about 1 to about 25 moles of ethylene oxide are suitable for use as the nonionic surfactant of the nonionic surfactant systems of the present invention. The alkyl chain of the aliphatic alcohol can either be straight or branched, primary or secondary, and generally contains from about 8 to about 22 carbon atoms. Preferred are the condensation products of alcohols having an alkyl group containing from about 8 to about 20 carbon atoms, more preferably from about 10 to about 18 carbon atoms, with from about 2 to about 10 moles of ethylene oxide per mole of alcohol. About 2 to about 7 moles of ethylene oxide and most preferably from 2 to 5 moles of ethylene oxide per mole of alcohol are present in said condensation products. Examples of commercially available nonionic surfactants of this type include Tergitol^{™} 15-S-9 (the condensation product of C₁₁-C₁₅ linear alcohol with 9 moles ethylene oxide), Tergitol^{™} 24-L-6 NMW (the condensation product of C₁₂-C₁₄ primary alcohol with 6 moles ethylene oxide with a narrow molecular weight distribution), both marketed by Union Carbide Corporation; Neodol^{™} 45-9 (the condensation product of C₁₄-C₁₅ linear alcohol with 9 moles of ethylene oxide), Neodol^{™} 23-3 (the condensation product of C₁₂-C₁₃ linear alcohol with 3.0 moles of ethylene oxide), Neodol^{™} 45-7 (the condensation product of C₁₄-C₁₅ linear alcohol with 7 moles of ethylene oxide), Neodol^{™} 45-5 (the condensation product of C₁₄-C₁₅ linear alcohol with 5 moles of ethylene oxide) marketed by Shell Chemical Company, Kyro^{™} EOB (the condensation product of C₁₃-C₁₅ alcohol with 9 moles ethylene oxide), marketed by The Procter & Gamble Company, and Genapol LA 030 or O5O (the condensation product of C₁₂-C₁₄ alcohol with 3 or 5 moles of ethylene oxide) marketed by Hoechst. Preferred range of HLB in these products is from 8-11 and most preferred from 8-10.

Also useful as the nonionic surfactant of the surfactant systems of the present invention are the alkylpolysaccharides disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e.g. a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6- positions on the preceding saccharide units. The preferred alkylpolyglycosides have the formula

R²O(CₙH₂ₙO)ₜ(glycosyl)ₓ

wherein R² is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms: n is 2 or 3, preferably 2; t is from 0 to about 10, preferably 0; and x is from about 1.3 to about 10, preferably from about 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominately the 2-position.

The condensation products of ethylene oxide with a hydrophobic base formed by the condensation of propylene oxide with propylene glycol are also suitable for use as the additional nonionic surfactant systems of the present invention. The hydrophobic portion of these compounds will preferably have a molecular weight of from about 1500 to about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially-available Plurafac^{™} LF404 and Pluronic^{™} surfactants, marketed by BASF.

Also suitable for use as the nonionic surfactant of the nonionic surfactant system of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2500 to about 3000. This hydrophobic moiety is condensed with ethylene oxide to the extent that the condensation product contains from about 40% to about 80% by weight of polyoxyethylene and has a molecular weight of from about 5,000 to about 11,000. Examples of this type of nonionic surfactant include certain of the commercially available Tetronic^{™} compounds, marketed by BASF.

Preferred for use as the nonionic surfactant of the surfactant systems of the present invention are polyethylene oxide condensates of alkyl phenols, condensation products of primary and secondary aliphatic alcohols with from about 1 to about 25 moles of ethylene oxide, alkylpolysaccharides, and mixtures thereof. Most preferred are C₈-C₁₄ alkyl phenol ethoxylates having from 3 to 15 ethoxy groups and C₈-C₁₈ alcohol ethoxylates (preferably C₁₀ avg.) having from 2 to 10 ethoxy groups, and mixtures thereof.

Highly preferred nonionic surfactants are polyhydroxy fatty acid amide surfactants of the formula. 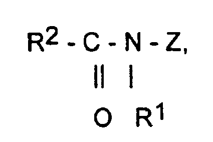 wherein R¹ is H, or R¹ is C₁₋₄ hydrocarbyl, 2-hydroxy ethyl, 2-hydroxy propyl or a mixture thereof, R² is C₅₋₃₁ hydrocarbyl, and Z is a polyhydroxyhydrocarbyl having a linear hydrocarbyl chain with at least 3 hydroxyls directly connected to the chain, or an alkoxylated derivative thereof. Preferably, R¹ is methyl, R² is a straight C₁₁₋₁₅ alkyl or C₁₆₋₁₈ alkyl or alkenyl chain such as coconut alkyl or mixtures thereof, and Z is derived from a reducing sugar such as glucose, fructose, maltose, lactose, in a reductive amination reaction.

Suitable anionic surfactants to be used are linear alkyl benzene sulfonate, alkyl ester sulfonate surfactants including linear esters of C₈-C₂₀ carboxylic acids (i.e., fatty acids) which are sulfonated with gaseous SO₃ according to "The Journal of the American Oil Chemists Society", 52 (1975), pp. 323-329. Suitable starting materials would include natural fatty substances as derived from tallow, palm oil, etc.
The preferred alkyl ester sulfonate surfactant, especially for laundry applications, comprise alkyl ester sulfonate surfactants of the structural formula : 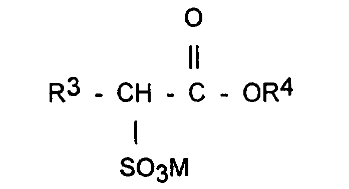 wherein R³ is a C₈-C₂₀ hydrocarbyl, preferably an alkyl, or combination thereof, R⁴ is a C₁-C₆ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as monoethanolamine, diethanolamine, and triethanolamine. Preferably, R³ is C₁₀-C₁₆ alkyl, and R⁴ is methyl, ethyl or isopropyl. Especially preferred are the methyl ester sulfonates wherein R³ is C₁₀-C₁₆ alkyl.

Other suitable anionic surfactants include the alkyl sulfate surfactants which are water soluble salts or acids of the formula ROSO₃M wherein R preferably is a C₁₀-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of C₁₂-C₁₆ are preferred for lower wash temperatures (e.g. below about 50°C) and C₁₆₋₁₈ alkyl chains are preferred for higher wash temperatures (e.g. above about 50°C).

Other anionic surfactants useful for detersive purposes can also be included in the cleaning compositions of the present invention. These can include salts (including, for example, sodium, potassium, ammonium, and substituted ammonium salts such as mono-, di- and triethanolamine salts) of soap, C₈-C₂₂ primary of secondary alkanesulfonates, C₈-C₂₄ olefinsulfonates, sulfonated polycarboxylic acids prepared by sulfonation of the pyrolyzed product of alkaline earth metal citrates, e.g., as described in British patent specification No. 1,082,179, C₈-C₂₄ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinates (especially saturated and unsaturated C₁₂-C₁₈ monoesters) and diesters of sulfosuccinates (especially saturated and unsaturated C₆-C₁₂ diesters), acyl sarcosinates, sulfates of alkylpolysaccharides such as the sulfates of alkylpolyglucoside (the nonionic nonsulfated compounds being described below), branched primary alkyl sulfates, and alkyl polyethoxy carboxylates such as those of the formula RO(CH₂CH₂O)ₖ-CH₂COO-M+ wherein R is a C₈-C₂₂ alkyl, k is an integer from 1 to 10, and M is a soluble salt-forming cation. Resin acids and hydrogenated resin acids are also suitable, such as rosin, hydrogenated rosin, and resin acids and hydrogenated resin acids present in or derived from tall oil.

Further examples are described in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23 (herein incorporated by reference).
When included therein, the laundry detergent compositions of the present invention typically comprise from about 1% to about 40%, preferably from about 3% to about 20% by weight of such anionic surfactants.

Highly preferred anionic surfactants include alkyl alkoxylated sulfate surfactants hereof are water soluble salts or acids of the formula RO(A)ₘSO3M wherein R is an unsubstituted C₁₀-C₂₄ alkyl or hydroxyalkyl group having a C₁₀-C₂₄ alkyl component, preferably a C₁₂-C₂₀ alkyl or hydroxyalkyl, more preferably C₁₂-C₁₈ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are C₁₂-C₁₈ alkyl polyethoxylate (1.0) sulfate (C₁₂-C₁₈E(1.0)M), C₁₂-C₁₈ alkyl polyethoxylate (2.25) sulfate (C₁₂-C₁₈E(2.25)M), C₁₂-C₁₈ alkyl polyethoxylate (3.0) sulfate (C₁₂-C₁₈E(3.0)M), and C₁₂-C₁₈ alkyl polyethoxylate (4.0) sulfate (C₁₂-C₁₈E(4.0)M), wherein M is conveniently selected from sodium and potassium.

The cleaning compositions of the present invention may also contain cationic, ampholytic, zwitterionic, and semi-polar surfactants, as well as the nonionic and/or anionic surfactants other than those already described herein.

Cationic detersive surfactants suitable for use in the cleaning compositions of the present invention are those having one long-chain hydrocarbyl group. Examples of such cationic surfactants include the ammonium surfactants such as alkyltrimethylammonium halogenides, and those surfactants having the formula:

[R²(OR³)_{y}][R⁴(OR³)_{y}]₂R⁵N+X-

wherein R² is an alkyl or alkyl benzyl group having from about 8 to about 18 carbon atoms in the alkyl chain, each R³ is selected from the group consisting of -CH₂CH₂-, -CH₂CH(CH₃)-, -CH₂CH(CH₂OH)-, -CH₂CH₂CH₂-, and mixtures thereof; each R⁴ is selected from the group consisting of C₁-C₄ alkyl, C₁-C₄ hydroxyalkyl, benzyl ring structures formed by joining the two R⁴ groups, - CH₂CHOH-CHOHCOR⁶CHOHCH₂OH wherein R⁶ is any hexose or hexose polymer having a molecular weight less than about 1000, and hydrogen when y is not 0; R⁵ is the same as R⁴ or is an alkyl chain wherein the total number of carbon atoms of R² plus R⁵ is not more than about 18; each y is from 0 to about 10 and the sum of the y values is from 0 to about 15; and X is any compatible anion.

Quaternary ammonium surfactant suitable for the present invention has the formula (I): 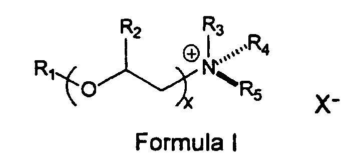 whereby R1 is a short chainlength alkyl (C6-C10) or alkylamidoalkyl of the formula (II) : 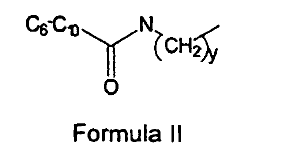 y is 2-4, preferably 3.
whereby R2 is H or a C1-C3 alkyl,
whereby x is 0-4, preferably 0-2, most preferably 0,
whereby R3, R4 and R5 are either the same or different and can be either a short chain alkyl (C1-C3) or alkoxylated alkyl of the formula III,
whereby X- is a counterion, preferably a halide, e.g. chloride or methylsulfate. 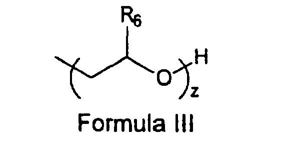 R6 is C₁-C₄ and z is 1 or 2.

Preferred quat ammonium surfactants are those as defined in formula I whereby
R₁ is C₈, C₁₀ or mixtures thereof, x=o,
R₃, R₄ = CH₃ and R₅ = CH₂CH₂OH.

Highly preferred cationic surfactants are the water-soluble quaternary ammonium compounds useful in the present composition having the formula:

R₁R₂R₃R₄N⁺X⁻ (i)

wherein R₁ is C₈-C₁₆ alkyl, each of R₂, R₃ and R₄ is independently C₁-C₄ alkyl, C₁-C₄ hydroxy alkyl, benzyl, and -(C₂H₄₀)ₓH where x has a value from 2 to 5, and X is an anion. Not more than one of R₂, R₃ or R₄ should be benzyl.
The preferred alkyl chain length for R₁ is C₁₂-C₁₅ particularly where the alkyl group is a mixture of chain lengths derived from coconut or palm kernel fat or is derived synthetically by olefin build up or OXO alcohols synthesis. Preferred groups for R₂R₃ and R₄ are methyl and hydroxyethyl groups and the anion X may be selected from halide, methosulphate, acetate and phosphate ions.
Examples of suitable quaternary ammonium compounds of formulae (i) for use herein are :
coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
C₁₂₋₁₅ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl (ethenoxy)₄ ammonium chloride or bromide;
choline esters (compounds of formula (i) wherein R₁ is 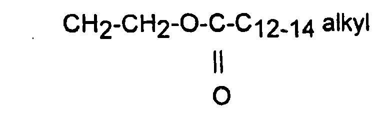 and R₂R₃R₄ are methyl).
di-alkyl imidazolines [compounds of formula (i)].

Other cationic surfactants useful herein are also described in U.S. Patent 4,228,044, Cambre, issued October 14, 1980 and in European Patent Application EP 000,224.

Typical cationic fabric softening components include the water-insoluble quaternary-ammonium fabric softening actives or thei corresponding amine precursor, the most commonly used having been di-long alkyl chain ammonium chloride or methyl sulfate.
Preferred cationic softeners among these include the following:
1) ditallow dimethylammonium chloride (DTDMAC);
2) dihydrogenated tallow dimethylammonium chloride;
3) dihydrogenated tallow dimethylammonium methylsulfate;
4) distearyl dimethylammonium chloride;
5) dioleyl dimethylammonium chloride;
6) dipalmityl hydroxyethyl methylammonium chloride;
7) stearyl benzyl dimethylammonium chloride;
8) tallow trimethylammonium chloride;
9) hydrogenated tallow trimethylammonium chloride;
10) C₁₂₋₁₄ alkyl hydroxyethyl dimethylammonium chloride;
11) C₁₂₋₁₈ alkyl dihydroxyethyl methylammonium chloride;
12) di(stearoyloxyethyl) dimethylammonium chloride (DSOEDMAC);
13) di(tallow-oxy-ethyl)dimethylammonium chloride;
14) ditallow imidazolinium methylsulfate;
15) 1-(2-tallowylamidoethyl)-2-tallowyl imidazolinium methylsulfate.

Biodegradable quaternary ammonium compounds have been presented as alternatives to the traditionally used di-long alkyl chain ammonium chlorides and methyl sulfates. Such quaternary ammonium compounds contain long chain alk(en)yl groups interrupted by functional groups such as carboxy groups. Said materials and fabric softening compositions containing them are disclosed in numerous publications such as EP-A-0,040,562, and EP-A-0,239,910.
The quaternary ammonium compounds and amine precursors herein have the formula (I) or (II), below : 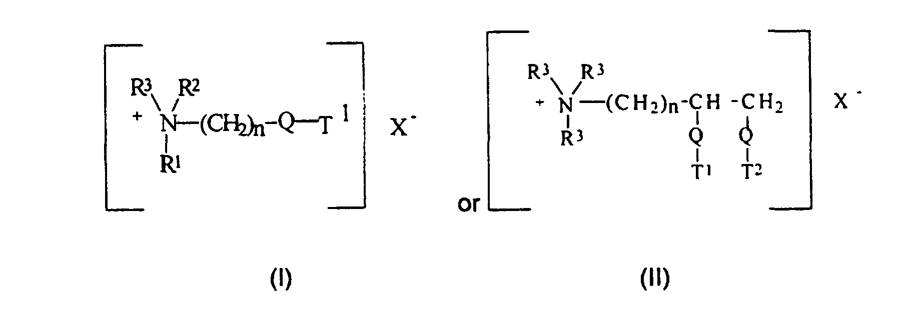 wherein Q is selected from -O-C(O)-, -C(O)-O-, -O-C(O)-O-, -NR⁴-C(O)-, -C(O)-NR⁴-;
R¹ is (CH₂)ₙ-Q-T² or T³;
R² is (CH₂)ₘ-Q-T⁴ or T⁵ or R³;
R³ is C₁-C₄ alkyl or C₁-C₄ hydroxyalkyl or H;
R⁴ is H or C₁-C₄ alkyl or C₁-C₄ hydroxyalkyl;
T¹, T², T³, T⁴, T⁵ are independently C₁₁-C₂₂ alkyl or alkenyl;
n and m are integers from 1 to 4; and
X⁻ is a softener-compatible anion.

Non-limiting examples of softener-compatible anions include chloride or methyl sulfate.

The alkyl, or alkenyl, chain T¹, T², T³, T⁴, T⁵ must contain at least 11 carbon atoms, preferably at least 16 carbon atoms. The chain may be straight or branched.

Tallow is a convenient and inexpensive source of long chain alkyl and alkenyl material. The compounds wherein T¹, T² T³, T⁴, T⁵ represents the mixture of long chain materials typical for tallow are particularly preferred.
Specific examples of quaternary ammonium compounds suitable for use in the aqueous fabric softening compositions herein include:
1) N,N-di(tallowyl-oxy-ethyl)-N,N-dimethyl ammonium chloride;
2) N,N-di(tallowyl-oxy-ethyl)-N-methyl, N-(2-hydroxyethyl) ammonium methyl sulfate;
3) N,N-di(2-tallowyl-oxy-2-oxo-ethyl)-N,N-dimethyl ammonium chloride;
4) N,N-di(2-tallowyl-oxy-ethylcarbonyl-oxy-ethyl)-N,N-dimethyl ammonium chloride;
5) N-(2-tallowyl-oxy-2-ethyl)-N-(2-tallowyl-oxy-2-oxo-ethyl)-N,N-dimethyl ammonium chloride;
6) N,N,N-tri(tallowyl-oxy-ethyl)-N-methyl ammonium chloride;
7) N-(2-tallowyl-oxy-2-oxo-ethyl)-N-(tallowyl-N,N-dimethyl-ammonium chloride; and
8) 1,2-ditallowyl-oxy-3-trimethylammoniopropane chloride;
and mixtures of any of the above materials.

When included therein, the cleaning compositions of the present invention typically comprise from 0.2% to about 25%, preferably from about 1% to about 8% by weight of such cationic surfactants.

Ampholytic surfactants are also suitable for use in the cleaning compositions of the present invention. These surfactants can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least about 8 carbon atoms, typically from about 8 to about 18 carbon atoms, and at least one contains an anionic water-solubilizing group, e.g. carboxy, sulfonate, sulfate. See U.S. Patent No. 3,929,678 to Laughlin et al., issued December 30, 1975⁻ at column 19, lines 18-35, for examples of ampholytic surfactants.

When included therein, the cleaning compositions of the present invention typically comprise from 0.2% to about 15%, preferably from about 1% to about 10% by weight of such ampholytic surfactants.

Zwitterionic surfactants are also suitable for use in cleaning compositions. These surfactants can be broadly described as derivatives of secondary and tertiary amines, derivatives of heterocyclic secondary and tertiary amines, or derivatives of quaternary ammonium, quaternary phosphonium or tertiary sulfonium compounds. See U.S. Patent No. 3,929,678 to Laughlin et al., issued December 30, 1975 at column 19, line 38 through column 22, line 48, for examples of zwitterionic surfactants.

When included therein, the cleaning compositions of the present invention typically comprise from 0.2% to about 15%, preferably from about 1% to about 10% by weight of such zwitterionic surfactants.

Semi-polar nonionic surfactants are a special category of nonionic surfactants which include water-soluble amine oxides containing one alkyl moiety of from about 10 to about 18 carbon atoms and 2 moieties selected from the group consisting of alkyl groups and hydroxyalkyl groups containing from about 1 to about 3 carbon atoms; water-soluble phosphine oxides containing one alkyl moiety of from about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety of from about 10 to about 18 carbon atoms and a moiety selected from the group consisting of alkyl and hydroxyalkyl moieties of from about 1 to about 3 carbon atoms.
Semi-polar nonionic detergent surfactants include the amine oxide surfactants having the formula 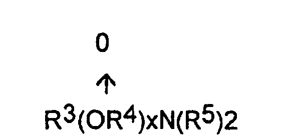 wherein R³ is an alkyl, hydroxyalkyl, or alkyl phenyl group or mixtures therof containing from about 8 to about 22 carbon atoms; R⁴ is an alkylene or hydroxyalkylene group containing from about 2 to about 3 carbon atoms or mixtures thereof; x is from 0 to about 3; and each R⁵ is an alkyl or hydroxyalkyl group containing from about 1 to about 3 carbon atoms or a polyethylene oxide group containing from about 1 to about 3 ethylene oxide groups. The R⁵ groups can be attached to each other, e.g., through an oxygen or nitrogen atom, to form a ring structure.

These amine oxide surfactants in particular include C₁₀-C₁₈ alkyl dimethyl amine oxides and C₈-C₁₂ alkoxy ethyl dihydroxy ethyl amine oxides.

When included therein, the cleaning compositions of the present invention typically comprise from 0.2% to about 15%, preferably from about 1% to about 10% by weight of such semi-polar nonionic surfactants.

The cleaning composition of the present invention may further comprise a cosurfactant selected from the group of primary or tertiary amines. Suitable primary amines for use herein include amines according to the formula R₁NH₂ wherein R₁ is a C₆-C₁₂, preferably C₆-C₁₀ alkyl chain or R₄X(CH₂)ₙ, X is -O-,-C(O)NH- or -NH-, R₄ is a C₆-C₁₂ alkyl chain n is between 1 to 5, preferably 3. R₁ alkyl chains may be straight or branched and may be interrupted with up to 12, preferably less than 5 ethylene oxide moieties. Preferred amines according to the formula herein above are n-alkyl amines. Suitable amines for use herein may be selected from 1-hexylamine, 1-octylamine, 1-decylamine and laurylamine. Other preferred primary amines include C8-C10 oxypropylamine, octyloxypropylamine, 2-ethylhexyloxypropylamine, lauryl amido propylamine and amido propylamine.

Suitable tertiary amines for use herein include tertiary amines having the formula R₁R₂R₃N wherein R1 and R2 are C₁-C₈ alkylchains or 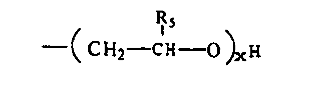 R₃ is either a C₆-C₁₂, preferably C₆-C₁₀ alkyl chain, or R₃ is R₄X(CH₂)ₙ, whereby X is -O-, -C(O)NH- or -NH-,R₄ is a C₄-C₁₂, n is between 1 to 5, preferably 2-3. R₅ is H or C₁-C₂ alkyl and x is between 1 to 6 .
R₃ and R₄ may be linear or branched ; R₃ alkyl chains may be interrupted with up to 12, preferably less than 5, ethylene oxide moieties.

Preferred tertiary amines are R₁R₂R₃N where R1 is a C6-C12 alkyl chain, R2 and R3 are C1-C3 alkyl or 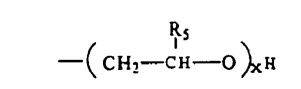 where R5 is H or CH3 and x = 1-2.

Also preferred are the amidoamines of the formula: 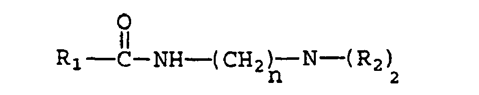 wherein R₁ is C₆-C₁₂ alkyl; n is 2-4,
preferably n is 3; R₂ and R₃ is C₁-C₄

Most preferred amines of the present invention include 1-octylamine, 1-hexylamine, 1-decylamine, 1-dodecylamine,C8-10oxypropylamine, N coco 1-3diaminopropane, coconutalkyldimethylamine, lauryldimethylamine, lauryl bis(hydroxyethyl)amine, coco bis(hydroxyehtyl)amine, lauryl amine 2 moles propoxylated, octyl amine 2 moles propoxylated, lauryl amidopropyldimethylamine, C8-10 amidopropyldimethylamine and C10 amidopropyldimethylamine.
The most preferred amines for use in the compositions herein are 1-hexylamine, 1-octylamine, 1-decylamine, 1-dodecylamine. Especially desirable are n-dodecyldimethylamine and bishydroxyethylcoconutalkylamine and oleylamine 7 times ethoxylated, lauryl amido propylamine and cocoamido propylamine.

### Color care and fabric care benefits

Technologies which provide a type of color care benefit can also be included. Examples of these technologies are metallo catalysts for color maintenance. Such metallo catalysts are described in copending European Patent Application No. 92870181.2. Dye fixing agents, polyolefin dispersion for anti-wrinkles and improved water absorbancy, perfume and amino-functional polymer for color care treatment and perfume substantivity are further examples of color care / fabric care technologies and are described in the co-pending Patent Application No. 96870140.9, filed November 07, 1996.

Fabric softening agents can also be incorporated into cleaning compositions in accordance with the present invention. These agents may be inorganic or organic in type. Inorganic softening agents are exemplified by the smectite clays disclosed in GB-A-1 400 898 and in USP 5,019,292. Organic fabric softening agents include the water insoluble tertiary amines as disclosed in GB-A1 514 276 and EP-BO 011 340 and their combination with mono C12-C14 quaternary ammonium salts are disclosed in EP-B-0 026 527 and EP-B-0 026 528 and di-long-chain amides as disclosed in EP-B-0 242 919. Other useful organic ingredients of fabric softening systems include high molecular weight polyethylene oxide materials as disclosed in EP-A-0 299 575 and 0 313 146.

Levels of smectite clay are normally in the range from 2% to 20%, more preferably from 5% to 15% by weight, with the material being added as a dry mixed component to the remainder of the formulation. Organic fabric softening agents such as the water-insoluble tertiary amines or dilong chain amide materials are incorporated at levels of from 0.5% to 5% by weight, normally from 1 % to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

### Builder system

The compositions according to the present invention may further comprise a builder system. Any conventional builder system is suitable for use herein including aluminosilicate materials, silicates, polycarboxylates, alkyl- or alkenyl-succinic acid and fatty acids, materials such as ethylenediamine tetraacetate, diethylene triamine pentamethyleneacetate, metal ion sequestrants such as aminopolyphosphonates, particularly ethylenediamine tetramethylene phosphonic acid and diethylene triamine pentamethylenephosphonic acid. Phosphate builders can also be used herein.

Suitable builders can be an inorganic ion exchange material, commonly an inorganic hydrated aluminosilicate material, more particularly a hydrated synthetic zeolite such as hydrated zeolite A, X, B, HS or MAP.
Another suitable inorganic builder material is layered silicate, e.g. SKS-6 (Hoechst). SKS-6 is a crystalline layered silicate consisting of sodium silicate (Na₂Si₂O₅).
Suitable polycarboxylates containing one carboxy group include lactic acid, glycolic acid and ether derivatives thereof as disclosed in Belgian Patent Nos. 831,368, 821,369 and 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in German Offenlegenschrift 2,446,686, and 2,446,687 and U.S. Patent No. 3,935,257 and the sulfinyl carboxylates described in Belgian Patent No. 840,623. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in Netherlands Application 7205873, and the oxypolycarboxylate materials such as 2-oxa-1,1,3-propane tricarboxylates described in British Patent No. 1,387,447.

Polycarboxylates containing four carboxy groups include oxydisuccinates disclosed in British Patent No. 1,261,829, 1,1,2,2-ethane tetracarboxylates, 1,1,3,3-propane tetracarboxylates and 1,1,2,3-propane tetracarboxylates. Polycarboxylates containing sulfo substituents include the sulfosuccinate derivatives disclosed in British Patent Nos. 1,398,421 and 1,398,422 and in U.S. Patent No. 3,936,448, and the sulfonated pyrolysed citrates described in British Patent No. 1,082,179, while polycarboxylates containing phosphone substituents are disclosed in British Patent No. 1,439,000.

Alicyclic and heterocyclic polycarboxylates include cyclopentane-cis,cis,cis-tetracarboxylates, cyclopentadienide pentacarboxylates, 2,3,4,5-tetrahydro-furan - cis, cis, cis-tetracarboxylates, 2,5-tetrahydro-furan -cis - dicarboxylates, 2,2,5,5-tetrahydrofuran - tetracarboxylates, 1,2,3,4,5,6-hexane - hexacar-boxylates and and carboxymethyl derivatives of polyhydric alcohols such as sorbitol, mannitol and xylitol. Aromatic poly-carboxylates include mellitic acid, pyromellitic acid and the phthalic acid derivatives disclosed in British Patent No. 1,425,343.
Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid.

Preferred builder systems include a mixture of a water-insoluble aluminosilicate builder such as zeolite A, and a watersoluble carboxylate chelating agent such as citric acid. Preferred builder systems for use in liquid detergent compositions of the present invention are soaps and polycarboxylates.

Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated from each other by not more than two carbon atoms. Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in amounts of from 5% to 80% by weight of the composition preferably from 10% to 70% and most usually from 30% to 60% by weight.

### Chelating Agents

The cleaning compositions herein may also optionally contain one or more iron and/or manganese chelating agents. Such chelating agents can be selected from the group consisting of amino carboxylates, amino phosphonates, polyfunctionally-substituted aromatic chelating agents and mixtures therein, all as hereinafter defined. Without intending to be bound by theory, it is believed that the benefit of these materials is due in part to their exceptional ability to remove iron and manganese ions from washing solutions by formation of soluble chelates.

Amino carboxylates useful as optional chelating agents include ethylenediaminetetracetates, N-hydroxyethylethylenediaminetriacetates, nitrilotriacetates, ethylenediamine tetraproprionates, triethylenetetraaminehexacetates, diethylenetriaminepentaacetates, and ethanoldiglycines, alkali metal, ammonium, and substituted ammonium salts therein and mixtures therein.

Amino phosphonates are also suitable for use as chelating agents in the compositions of the invention when at lease low levels of total phosphorus are permitted in detergent compositions, and include ethylenediaminetetrakis (methylenephosphonates) as DEQUEST. Preferred, these amino phosphonates to not contain alkyl or alkenyl groups with more than about 6 carbon atoms.

Polyfunctionally-substituted aromatic chelating agents are also useful in the compositions herein. See U.S. Patent 3,812,044, issued May 21, 1974, to Connor et al. Preferred compounds of this type in acid form are dihydroxydisulfobenzenes such as 1,2-dihydroxy-3,5-disulfobenzene.

A preferred biodegradable chelator for use herein is ethylenediamine disuccinate ("EDDS"), especially the [S,S] isomer as described in U.S. Patent 4,704,233, November 3, 1987, to Hartman and Perkins.

The compositions herein may also contain water-soluble methyl glycine diacetic acid (MGDA) salts (or acid form) as a chelant or co-builder useful with, for example, insoluble builders such as zeolites, layered silicates and the like.

If utilized, these chelating agents will generally comprise from about 0.1 % to about 15% by weight of the detergent compositions herein. More preferably, if utilized, the chelating agents will comprise from about 0.1 % to about 3.0% by weight of such compositions.

### Suds suppressor

Another optional ingredient is a suds suppressor, exemplified by silicones, and silica-silicone mixtures. Silicones can be generally represented by alkylated polysiloxane materials while silica is normally used in finely divided forms exemplified by silica aerogels and xerogels and hydrophobic silicas of various types. These materials can be incorporated as particulates in which the suds suppressor is advantageously releasably incorporated in a water-soluble or water-dispersible, substantially non-surface-active detergent impermeable carrier. Alternatively the suds suppressor can be dissolved or dispersed in a liquid carrier and applied by spraying on to one or more of the other components.
A preferred silicone suds controlling agent is disclosed in Bartollota et al. U.S. Patent 3 933 672. Other particularly useful suds suppressors are the self-emulsifying silicone suds suppressors, described in German Patent Application DTOS 2 646 126 published April 28, 1977. An example of such a compound is DC-544, commercially available from Dow Coming, which is a siloxane-glycol copolymer. Especially preferred suds controlling agent are the suds suppressor system comprising a mixture of silicone oils and 2-alkyl-alcanols. Suitable 2-alkyl-alkanols are 2-butyl-octanol which are commercially available under the trade name Isofol 12 R.
Such suds suppressor system are described in EP 593 841 filed 10 November, 1992.
Especially preferred silicone suds controlling agents are described in EP 573 699. Said compositions can comprise a silicone/silica mixture in combination with fumed nonporous silica such as Aerosil^{R}:

The suds suppressors described above are normally employed at levels of from 0.001% to 2% by weight of the composition, preferably from 0.01 % to 1 % by weight.

### Others

Other components used in cleaning compositions may be employed, such as soil-suspending agents, soil-release agents, optical brighteners, abrasives, bactericides, tarnish inhibitors, coloring agents, and/or encapsulated or non-encapsulated perfumes.

Especially suitable encapsulating materials are water soluble capsules which consist of a matrix of polysaccharide and polyhydroxy compounds such as described in GB 1,464,616.

Other suitable water soluble encapsulating materials comprise dextrins derived from ungelatinized starch acid-esters of substituted dicarboxylic acids such as described in US 3,455,838. These acid-ester dextrins are,preferably, prepared from such starches as waxy maize, waxy sorghum, sago, tapioca and potato. Suitable examples of said encapsulating materials include N-Lok manufactured by National Starch. The N-Lok encapsulating material consists of a modified maize starch and glucose. The starch is modified by adding monofunctional substituted groups such as octenyl succinic acid anhydride.

Antiredeposition and soil suspension agents suitable herein include cellulose derivatives such as methylcellulose, carboxymethylcellulose and hydroxyethylcellulose, and homo- or co-polymeric polycarboxylic acids or their salts. Polymers of this type include the polyacrylates and maleic anhydride-acrylic acid copolymers previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10% by weight, more preferably from 0.75% to 8%, most preferably from 1% to 6% by weight of the composition.

Preferred optical brighteners are anionic in character, examples of which are disodium 4,4'-bis-(2-diethanolamino-4-anilino -s- triazin-6-ylamino)stilbene-2:2' disulphonate, disodium 4, - 4'-bis-(2-morpholino-4-anilino-s-triazin-6-ylamino-stilbene-2:2' - disulphonate, disodium 4,4' - bis-(2,4-dianilino-s-triazin-6-ylamino)stilbene-2:2' - disulphonate, monosodium 4',4" -bis-(2,4-dianilino-s-tri-azin-6 ylamino)stilbene-2-sulphonate, disodium 4,4' -bis-(2-anilino-4-(N-methyl-N-2-hydroxyethylamino)-s-triazin-6-ylamino)stilbene-2,2' - disulphonate, di-sodium 4,4' -bis-(4-phenyl-2,1,3-triazol-2-yl)-stilbene-2,2' disulphonate, di-so-dium 4,4'bis(2-anilino-4-(1-methyl-2-hydroxyethylamino)-s-triazin-6- ylami-no)stilbene-2,2'disulphonate, sodium 2(stilbyl-4"-(naphtho-1',2':4,5)-1,2,3 - triazole-2"-sulphonate and 4,4'-bis(2-sulphostyryl)biphenyl. Highly preferred brighteners are the specific brighteners of copending European Patent application No. 95201943.8.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

Soil release agents useful in compositions of the present invention are conventionally copolymers or terpolymers of terephthalic acid with ethylene glycol and/or propylene glycol units in various arrangements. Examples of such polymers are disclosed in the commonly assigned US Patent Nos. 4116885 and 4711730 and European Published Patent Application No. 0 272 033. A particular preferred polymer in accordance with EP-A-0 272 033 has the formula

(CH₃(PEG)₄₃)_{0.75}(POH)_{0.25}[T-PO)_{2.8}(T-PEG)_{0.4}]T(PO-

H)_{0.25}((PEG)₄₃CH₃)_{0.75} where PEG is -(OC₂H₄)O-,PO is (OC₃H₆O) and T is (pcOC₆H₄CO).

Also very useful are modified polyesters as random copolymers of dimethyl terephthalate, dimethyl sulfoisophthalate, ethylene glycol and 1-2 propane diol, the end groups consisting primarily of sulphobenzoate and secondarily of mono esters of ethylene glycol and/or propane-diol. The target is to obtain a polymer capped at both end by sulphobenzoate groups, "primarily", in the present context most of said copolymers herein will be end-capped by sulphobenzoate groups. However, some copolymers will be less than fully capped, and therefore their end groups may consist of monoester of ethylene glycol and/or propane 1-2 diol, thereof consist "secondarily" of such species.

The selected polyesters herein contain about 46% by weight of dimethyl terephthalic acid, about 16% by weight of propane -1.2 diol, about 10% by weight ethylene glycol about 13% by weight of dimethyl sulfobenzoic acid and about 15% by weight of sulfoisophthalic acid, and have a molecular weight of about 3.000. The polyesters and their method of preparation are described in detail in EPA 311 342.

Is is well known in the art that free chlorine in tap water rapidly deactivates the enzymes comprised in detergent compositions. Therefore, using chlorine scavenger such as perborate, ammonium sulfate, sodium sulphite or polyethyleneimine at a level above 0.1% by weight of total composition, in the formulas will provide improved through the wash stability of the detergent enzymes. Compositions comprising chlorine scavenger are described in the EP 553 607 filed January 31, 1992.

Alkoxylated polycarboxylates such as those prepared from polyacrylates are useful herein to provide additional grease removal performance. Such materials are described in WO 91/08281. Chemically, these materials comprise polyacrylates having one ethoxy side-chain per every 7-8 acrylate units. The side-chains are of the formula -(CH₂CH₂O)ₘ(CH₂)ₙCH₃ wherein m is 2-3 and n is 6-12. The side-chains are ester-linked to the polyacrylate "backbone" to provide a "comb" polymer type structure. The molecular weight can vary, but is typically in the range of about 2000 to about 50,000. Such alkoxylated polycarboxylates can comprise from about 0.05% to about 10%, by weight, of the compositions herein.

### Dispersants

The cleaning compositions of the present invention can also contain dispersants : Suitable water-soluble organic salts are the homo- or co-polymeric acids or their salts, in which the polycarboxylic acid comprises at least two carboxyl radicals separated from each other by not more than two carbon atoms. Polymers of this type are disclosed in GB-A-1,596,756. Examples of such salts are polyacrylates of MW 2000-5000 and their copolymers with maleic anhydride, such copolymers having a molecular weight of from 1,000 to 100,000.

Especially, copolymer of acrylate and methylacrylate such as the 480N having a molecular weight of 4000, at a level from 0.5-20% by weight of composition can be added in the cleaning compositions of the present invention.

The compositions of the invention may contain a lime soap peptiser compound, which has preferably a lime soap dispersing power (LSDP), as defined hereinafter of no more than 8, preferably no more than 7, most preferably no more than 6. The lime soap peptiser compound is preferably present at a level from 0% to 20% by weight.

A numerical measure of the effectiveness of a lime soap peptiser is given by the lime soap dispersant power (LSDP) which is determined using the lime soap dispersant test as described in an article by H.C. Borghetty and C.A. Bergman, J. Am. Oil. Chem. Soc., volume 27, pages 88-90, (1950). This lime soap dispersion test method is widely used by practitioners in this art field being referred to, for example, in the following review articles; W.N. Linfield, Surfactant science Series, Volume 7, page 3; W.N. Linfield, Tenside surf. det., volume 27, pages 159-163, (1990); and M.K. Nagarajan, W.F. Masler, Cosmetics and Toiletries, volume 104, pages 71-73, (1989). The LSDP is the % weight ratio of dispersing agent to sodium oleate required to disperse the lime soap deposits formed by 0.025g of sodium oleate in 30ml of water of 333ppm CaCo₃ (Ca:Mg=3:2) equivalent hardness.

Surfactants having good lime soap peptiser capability will include certain amine oxides, betaines, sulfobetaines, alkyl ethoxysulfates and ethoxylated alcohols.

Exemplary surfactants having a LSDP of no more than 8 for use in accord with the present invention include C₁₆-C₁₈ dimethyl amine oxide, C₁₂-C₁₈ alkyl ethoxysulfates with an average degree of ethoxylation of from 1-5, particularly C₁₂-C₁₅ alkyl ethoxysulfate surfactant with a degree of ethoxylation of amount 3 (LSDP=4), and the C₁₄-C₁₅ ethoxylated alcohols with an average degree of ethoxylation of either 12 (LSDP=6) or 30, sold under the tradenames Lutensol A012 and Lutensol A030 respectively, by BASF GmbH.

Polymeric lime soap peptisers suitable for use herein are described in the article by M.K. Nagarajan, W.F. Masler, to be found in Cosmetics and Toiletries, volume 104, pages 71-73, (1989).

Hydrophobic bleaches such as 4-[N-octanoyl-6-aminohexanoyl]benzene sulfonate, 4-[N-nonanoyl-fi-aminohexanoyl]benzene sulfonate, 4-[N-decanoyl-6-aminohexanoyl]benzene sulfonate and mixtures thereof; and nonanoyloxy benzene sulfonate together with hydrophilic / hydrophobic bleach formulations can also be used as lime soap peptisers compounds.

### Dye transfer inhibition

The cleaning compositions of the present invention can also include compounds for inhibiting dye transfer from one fabric to another of solubilized and suspended dyes encountered during fabric laundering operations involving colored fabrics.

### Polymeric dye transfer inhibiting agents

The cleaning compositions according to the present invention also comprise from 0.001% to 10 %, preferably from 0.01% to 2%, more preferably from 0.05% to 1% by weight of polymeric dye transfer inhibiting agents. Said polymeric dye transfer inhibiting agents are normally incorporated into cleaning compositions in order to inhibit the transfer of dyes from colored fabrics onto fabrics washed therewith. These polymers have the ability to complex or adsorb the fugitive dyes washed out of dyed fabrics before the dyes have the opportunity to become attached to other articles in the wash.
Especially suitable polymeric dye transfer inhibiting agents are polyamine N-oxide polymers, copolymers of N-vinylpyrrolidone and N-vinylimidazole, polyvinylpyrrolidone polymers, polyvinyloxazolidones and polyvinylimidazoles or mixtures thereof.
Addition of such polymers also enhances the performance of the enzymes according the invention.

### a) Polyamine N-oxide polymers

The polyamine N-oxide polymers suitable for use contain units having the following structure formula : 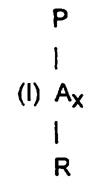 wherein P is a polymerisable unit, whereto the R-N-O group can be attached to or wherein the R-N-O group forms part of the polymerisable unit or a combination of both.
A is 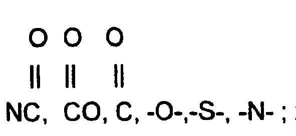 x is O or 1;
R are aliphatic, ethoxylated aliphatics, aromatic, heterocyclic or alicyclic groups or any combination thereof whereto the nitrogen of the N-O group can be attached or wherein the nitrogen of the N-O group is part of these groups.

The N-O group can be represented by the following general structures : 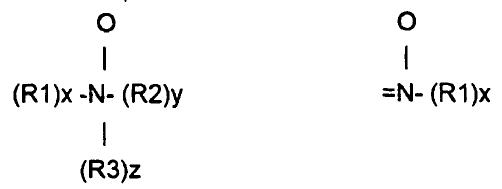 wherein R1, R2, and R3 are aliphatic groups, aromatic, heterocyclic or alicyclic groups or combinations thereof, x or/and y or/and z is 0 or 1 and wherein the nitrogen of the N-O group can be attached or wherein the nitrogen of the N-O group forms part of these groups.

The N-O group can be part of the polymerisable unit (P) or can be attached to the polymeric backbone or a combination of both.
Suitable polyamine N-oxides wherein the N-O group forms part of the polymerisable unit comprise polyamine N-oxides wherein R is selected from aliphatic, aromatic, alicyclic or heterocyclic groups.
One class of said polyamine N-oxides comprises the group of polyamine N-oxides wherein the nitrogen of the N-O group forms part of the R-group. Preferred polyamine N-oxides are those wherein R is a heterocyclic group such as pyrridine, pyrrole, imidazole, pyrrolidine, piperidine, quinoline, acridine and derivatives thereof.
Another class of said polyamine N-oxides comprises the group of polyamine N-oxides wherein the nitrogen of the N-O group is attached to the R-group.

Other suitable polyamine N-oxides are the polyamine oxides whereto the N-O group is attached to the polymerisable unit.
Preferred class of these polyamine N-oxides are the polyamine N-oxides having the general formula (I) wherein R is an aromatic, heterocyclic or alicyclic groups wherein the nitrogen of the N-0 functional group is part of said R group.
Examples of these classes are polyamine oxides wherein R is a heterocyclic compound such as pyrridine, pyrrole, imidazole and derivatives thereof.
Another preferred class of polyamine N-oxides are the polyamine oxides having the general formula (I) wherein R are aromatic, heterocyclic or alicyclic groups wherein the nitrogen of the N-0 functional group is attached to said R groups.
Examples of these classes are polyamine oxides wherein R groups can be aromatic such as phenyl.

Any polymer backbone can be used as long as the amine oxide polymer formed is water-soluble and has dye transfer inhibiting properties. Examples of suitable polymeric backbones are polyvinyls, polyalkylenes, polyesters, polyethers, polyamide, polyimides, polyacrylates and mixtures thereof.

The amine N-oxide polymers of the present invention typically have a ratio of amine to the amine N-oxide of 10:1 to 1:1000000. However the amount of amine oxide groups present in the polyamine oxide polymer can be varied by appropriate copolymerization or by appropriate degree of N-oxidation. Preferably, the ratio of amine to amine N-oxide is from 2:3 to 1:1000000. More preferably from 1:4 to 1:1000000, most preferably from 1:7 to 1:1000000. The polymers of the present invention actually encompass random or block copolymers where one monomer type is an amine N-oxide and the other monomer type is either an amine N-oxide or not. The amine oxide unit of the polyamine N-oxides has a PKa < 10, preferably PKa < 7, more preferred PKa < 6.
The polyamine oxides can be obtained in almost any degree of polymerisation. The degree of polymerisation is not critical provided the material has the desired water-solubility and dye-suspending power.
Typically, the average molecular weight is within the range of 500 to 1000,000; preferably from 1,000 to 50,000, more preferably from 2,000 to 30,000, most preferably from 3,000 to 20,000.

### b) Copolymers of N-vinylpyrrolidone and N-vinylimidazole

The N-vinylimidazole N-vinylpyrrolidone polymers used in the present invention have an average molecular weight range from 5,000-1,000,000, preferably from 5,000-200,000.
Highly preferred polymers for use in detergent compositions according to the present invention comprise a polymer selected from N-vinylimidazole N-vinylpyrrolidone copolymers wherein said polymer has an average molecular weight range from 5,000 to 50,000 more preferably from 8,000 to 30,000, most preferably from 10,000 to 20,000.
The average molecular weight range was determined by light scattering as described in Barth H.G. and Mays J.W. Chemical Analysis Vol 113,"Modern Methods of Polymer Characterization". Highly preferred N-vinylimidazole N-vinylpyrrolidone copolymers have an average molecular weight range from 5,000 to 50,000; more preferably from 8,000 to 30,000; most preferably from 10,000 to 20,000.

The N-vinylimidazole N-vinylpyrrolidone copolymers characterized by having said average molecular weight range provide excellent dye transfer inhibiting properties while not adversely affecting the cleaning performance of detergent compositions formulated therewith.
The N-vinylimidazole N-vinylpyrrolidone copolymer of the present invention has a molar ratio of N-vinylimidazole to N-vinylpyrrolidone from 1 to 0.2, more preferably from 0.8 to 0.3, most preferably from 0.6 to 0.4 .

### c) Polyvinylpyrrolidone

The detergent compositions of the present invention may also utilize polyvinylpyrrolidone ("PVP") having an average molecular weight of from about 2,500 to about 400,000, preferably from about 5,000 to about 200,000, more preferably from about 5,000 to about 50,000, and most preferably from about 5,000 to about 15,000. Suitable polyvinylpyrrolidones are commercially vailable from ISP Corporation, New York, NY and Montreal, Canada under the product names PVP K-15 (viscosity molecular weight of 10,000), PVP K-30 (average molecular weight of 40,000), PVP K-60 (average molecular weight of 160,000), and PVP K-90 (average molecular weight of 360,000). Other suitable polyvinylpyrrolidones which are commercially available from BASF Cooperation include Sokalan HP 165 and Sokalan HP 12; polyvinylpyrrolidones known to persons skilled in the detergent field (see for example EP-A-262,897 and EP-A-256,696).

### d) Polyvinyloxazolidone :

The detergent compositions of the present invention may also utilize polyvinyloxazolidone as a polymeric dye transfer inhibiting agent. Said polyvinyloxazolidones have an average molecular weight of from about 2,500 to about 400,000, preferably from about 5,000 to about 200,000, more preferably from about 5,000 to about 50,000, and most preferably from about 5,000 to about 15,000.

### e) Polyvinylimidazole :

The detergent compositions of the present invention may also utilize polyvinylimidazole as polymeric dye transfer inhibiting agent. Said polyvinylimidazoles have an average about 2,500 to about 400,000, preferably from about 5,000 to about 200,000, more preferably from about 5,000 to about 50,000, and most preferably from about 5,000 to about 15,000.

### f) Cross-linked polymers :

Cross-linked polymers are polymers whose backbone are interconnected to a certain degree; these links can be of chemical or physical nature, possibly with active groups n the backbone or on branches; cross-linked polymers have been described in the Journal of Polymer Science, volume 22, pages 1035-1039.
In one embodiment, the cross-linked polymers are made in such a way that they form a three-dimensional rigid structure, which can entrap dyes in the pores formed by the three-dimensional structure. In another embodiment, the cross-linked polymers entrap the dyes by swelling.
Such cross-linked polymers are described in the co-pending patent application 94870213.9

### Method of washing

The compositions of the invention may be used in essentially any washing or cleaning methods, including soaking methods, pretreatment methods and methods with rinsing steps for which a separate rinse aid composition may be added.

The process described herein comprises contacting fabrics with a laundering solution in the usual manner and exemplified hereunder.

The process of the invention is conveniently carried out in the course of the cleaning process. The method of cleaning is preferably carried out at 5°C to 95°C, especially between 10°C and 60°C. The pH of the treatment solution is preferably from 7 to 12.

A preferred machine dishwashing method comprises treating soiled articles with an aqueous liquid having dissolved or dispensed therein an effective amount of the machine diswashing or rinsing composition. A conventional effective amount of the machine dishwashing composition means from 8-60 g of product dissolved or dispersed in a wash volume from 3-10 litres.

According to a manual dishwashing method, soiled dishes are contacted with an effective amount of the diswashing composition, typically from 0.5-20g (per 25 dishes being treated). Preferred manual dishwashing methods include the application of a concentrated solution to the surfaces of the dishes or the soaking in large volume of dilute solution of the detergent composition.

The following examples are meant to exemplify compositions of the present invention, but are not necessarily meant to limit or otherwise define the scope of the invention.

In the cleaning compositions, the enzymes levels are expressed by pure enzyme by weight of the total composition and unless otherwise specified, the detergent ingredients are expressed by weight of the total compositions. The abbreviated component identifications therein have the following meanings:
- LAS: : Sodium linear C₁₁₋₁₃ alkyl benzene sulphonate.
- TAS: : Sodium tallow alkyl sulphate.
- CxyAS: : Sodium C₁ₓ-C_{1y} alkyl sulfate.
- CxySAS: : Sodium C₁ₓ-C_{1y} secondary (2,3) alkyl sulfate.
- CxyEz: : C₁ₓ-C_{1y} predominantly linear primary alcohol condensed with an average of z moles of ethylene oxide.
- CxyEzS: : C₁ₓ-C_{1y} sodium alkyl sulfate condensed with an average of z moles of ethylene oxide.
- QAS: : R₂.N+(CH₃)₂(C₂H₄OH) with R₂ = C₁₂-C₁₄.
- QAS 1: : R₂N+(CH₃)₂(C₂H₄OH) with R₂ = C₈-C₁₁.
- APA: : C₈₋₁₀ amido propyl dimethyl amine.
- Soap: : Sodium linear alkyl carboxylate derived from a 80/20 mixture of tallow and coconut fatty acids.
- Nonionic: : C₁₃-C₁₅ mixed ethoxylated/propoxylated fatty alcohol with an average degree of ethoxylation of 3.8 and an average degree of propoxylation of 4.5.
- Neodol 45-13: : C14-C15 linear primary alcohol ethoxylate, sold by Shell Chemical CO.
- STS: : Sodium toluene sulphonate.
- CFAA: : C₁₂-C₁₄ alkyl N-methyl glucamide.
- TFAA: : C₁₆-C₁₈ alkyl N-methyl glucamide.
- TPKFA: : C₁₂-C₁₄ topped whole cut fatty acids.
- DEQA: : Di-(tallow-oxy-ethyl) dimethyl ammonium chloride.
- DEQA (2): : Di-(soft-tallowyloxyethyl) hydroxyethyl methyl ammonium methylsulfate.
- DTDMAMS: : Ditalllow dimethyl ammonium methylsulfate.
- SDASA: : 1:2 ratio of stearyldimethyl amine:triple-pressed stearic acid.
- Silicate: : Amorphous Sodium Silicate (SiO₂:Na₂O ratio = 1.6-3.2).
- Metasilicate: : Sodium metasilicate (SiO₂:Na₂O ratio = 1.0).
- Zeolite A: : Hydrated Sodium Aluminosilicate of formula Na₁₂(A10₂Si0₂)₁₂. 27H₂O having a primary particle size in the range from 0.1 to 10 micrometers (Weight expressed on an anhydrous basis).
- Na-SKS-6: : Crystalline layered silicate of formula δ-Na₂Si₂O₅.
- Citrate: : Tri-sodium citrate dihydrate of activity 86.4% with a particle size distribution between 425 and 850 micrometres.
- Citric: : Anhydrous citric acid.
- Borate: : Sodium borate
- Carbonate: : Anhydrous sodium carbonate with a particle size between 200 and 900 micrometres.
- Bicarbonate: : Anhydrous sodium hydrogen carbonate with a particle size distribution between 400 and 1200 micrometres.
- Sulphate: : Anhydrous sodium sulphate.
- Mg Sulphate: : Anhydrous magnesium sulfate.
- STPP: : Sodium tripolyphosphate.
- TSPP: : Tetrasodium pyrophosphate.
- MA/AA: : Random copolymer of 4:1 acrylate/maleate, average molecular weight about 70,000-80,000.
- MA/AA 1: : Random copolymer of 6:4 acrylate/maleate, average molecular weight about 10,000.
- AA: : Sodium polyacrylate polymer of average molecular weight 4,500.
- PA30: : Polyacrylic acid of average molecular weight of between about 4,500 - 8,000.
- 480N: : Random copolymer of 7:3 acrylate/methacrylate, average molecular weight about 3,500.
- Polygel/carbopol: : High molecular weight crosslinked polyacrylates.
- PB1: : Anhydrous sodium perborate monohydrate of nominal formula NaBO₂.H₂O₂.
- PB4: : Sodium perborate tetrahydrate of nominal formula NaBO₂.3H₂O.H₂O₂.
- Percarbonate: : Anhydrous sodium percarbonate of nominal formula 2Na₂CO₃.3H₂O₂.
- NaDCC: : Sodium dichloroisocyanurate.
- TAED: : Tetraacetylethylenediamine.
- NOBS: : Nonanoyloxybenzene sulfonate in the form of the sodium salt.
- NACA-OBS: : (6-nonamidocaproyl) oxybenzene sulfonate.
- DTPA: : Diethylene triamine pentaacetic acid.
- HEDP: : 1,1-hydroxyethane diphosphonic acid.
- DETPMP: : Diethyltriamine penta (methylene) phosphonate, marketed by Monsanto under the Trade name Dequest 2060.
- EDDS: : Ethylenediamine-N,N'-disuccinic acid, (S,S) isomer in the form of its sodium salt
- MnTACN: : Manganese 1,4,7-trimethyl-1,4,7-triazacyclononane.
- Photoactivated Bleach: : Sulfonated zinc phtalocyanine encapsulated in dextrin soluble polymer.
- Photoactivated Bleach 1: : Sulfonated alumino phtalocyanine encapsulated in dextrin soluble polymer.
- PAAC: : Pentaamine acetate cobalt(III) salt.
- Paraffin: : Paraffin oil sold under the tradename Winog 70 by Wintershall.
- NaBz: : Sodium benzoate.
- BzP: : Benzoyl Peroxide.
- Oxygenase: : Phenyl alanine monooxygenase sold by Sigma under No. P6268 with 6-methyltetrahydroptarine sold by Sigma under the No. M4758 and DL-dithiothreitol sold by Sigma under the No. D0632, as cofactors - with a weight ratio of pure enzyme to cofactor between 1:2 and 1:5.
- Protease: : Proteolytic enzyme sold under the tradename Savinase, Alcalase, Durazym by Novo Nordisk A/S, Maxacal, Maxapem sold by Gist-Brocades and proteases described in patents WO91/06637 and/or WO95/10591 and/or EP 251 446.
- Amylase: : Amylolytic enzyme sold under the tradename Purafact Ox Am^{R} described in WO 94/18314, WO96/05295 sold by Genencor; Termamyl^{®}, Fungamyl^{®} and Duramyl^{®}, all available from Novo Nordisk A/S and those described in WO95/26397.
- Lipase: : Lipolytic enzyme sold under the tradename Lipolase, Lipolase Ultra by Novo Nordisk A/S and Lipomax by Gist-Brocades.
- Cellulase: : Cellulytic enzyme sold under the tradename Carezyme, Celluzyme and/or Endolase by Novo Nordisk A/S.
- CMC: : Sodium carboxymethyl cellulose.
- PVP: : Polyvinyl polymer, with an average molecular weight of 60,000.
- PVNO: : Polyvinylpyridine-N-Oxide, with an average molecular weight of 50,000.
- PVPVI: : Copolymer of vinylimidazole and vinylpyrrolidone, with an average molecular weight of 20,000.
- Brightener 1: : Disodium 4,4'-bis(2-sulphostyryl)biphenyl.
- Brightener 2: : Disodium 4,4'-bis(4-anilino-6-morpholino-1.3.5-triazin-2-yl) stilbene-2:2'-disulfonate.
- Silicone antifoam: : Polydimethylsiloxane foam controller with siloxane-oxyalkylene copolymer as dispersing agent with a ratio of said foam controller to said dispersing agent of 10:1 1 to 100:1.
- Suds Suppressor: : 12% Silicone/silica, 18% stearyl alcohol,70% starch in granular form.
- Opacifier: : Water based monostyrene latex mixture, sold by BASF Aktiengesellschaft under the tradename Lytron 621.
- SRP 1: : Anionically end capped poly esters.
- SRP 2: : Diethoxylated poly (1,2 propylene terephtalate) short block polymer.
- QEA: : bis((C₂H₅O)(C₂H₄O)ₙ)(CH₃) -N⁺-C₆H₁₂-N⁺-(CH₃) bis((C₂H₅O)-(C₂H₄O))ₙ, wherein n = from 20 to 30.
- PEI: : Polyethyleneimine with an average molecular weight of 1800 and an average ethoxylation degree of 7 ethyleneoxy residues per nitrogen.
- SCS: : Sodium cumene sulphonate.
- HMWPEO: : High molecular weight polyethylene oxide.
- PEGx: : Polyethylene glycol, of a molecular weight of x .
- PEO: : Polyethylene oxide, with an average molecular weight of 5,000.
- TEPAE: : Tetreaethylenepentaamine ethoxylate.
- BTA: : Benzotriazole.
- Silica dental abrasive: : Precipitated silica identified as Zeodent 119 offered by J.M. Huber.
- Carboxyvinyl polymer: : Carbopol offered by B.F. Goodrich Chemical Company.
- Carrageenan: : lota Carrageenan offered by Hercules Chemical Company.
- pH: : Measured as a 1% solution in distilled water at 20°C.

### Example 1

The following high density laundry detergent compositions were prepared according to the present invention :

| | **I** | **II** | **III** | **IV** | **V** | **VI** |
|---|---|---|---|---|---|---|
| LAS | 8.0 | 8.0 | 8.0 | 2.0 | 6.0 | 6.0 |
| TAS | - | 0.5 | - | 0.5 | 1.0 | 0.1 |
| C46(S)AS | 2.0 | 2.5 | - | - | - | - |
| | | | | | | |

| | **I** | **II** | **III** | **IV** | **V** | **VI** |
|---|---|---|---|---|---|---|
| C25AS | - | - | - | 7.0 | 4.5 | 5.5 |
| C68AS | 2.0 | 5.0 | 7.0 | - | - | - |
| C25E5 | - | - | 3.4 | 10.0 | 4.6 | 4.6 |
| C25E7 | 3.4 | 3.4 | 1.0 | - | - | - |
| C25E3S | - | - | - | 2.0 | 5.0 | 4.5 |
| QAS | - | 0.8 | - | - | - | - |
| QAS 1 | - | - | - | 0.8 | 0.5 | 1.0 |
| Zeolite A | 18.1 | 18.0 | 14.1 | 18.1 | 20.0 | 18.1 |
| Citric | - | - | - | 2.5 | - | 2.5 |
| Carbonate | 13.0 | 13.0 | 27.0 | 10.0 | 10.0 | 13.0 |
| Na-SKS-6 | - | - | - | 10.0 | - | 10.0 |
| Silicate | 1.4 | 1.4 | 3.0 | 0.3 | 0.5 | 0.3 |
| Citrate | - | 1.0 | - | 3.0 | - | - |
| Sulfate | 26.1 | 26.1 | 26.1 | 6.0 | - | - |
| Mg sulfate | 0.3 | - | - | 0.2 | - | 0.2 |
| MA/AA | 0.3 | 0.3 | 0.3 | 4.0 | 1.0 | 1.0 |
| CMC | 0.2 | 0.2 | 0.2 | 0.2 | 0.4 | 0.4 |
| PB4 | 9.0 | 9.0 | 5.0 | - | - | - |
| Percarbonate | - | - | - | - | 18.0 | 18.0 |
| TAED | 1.5 | 0.4 | 1.5 | - | 3.9 | 4.2 |
| NACA-OBS | - | 2.0 | 1.0 | - | - | - |
| DETPMP | 0.25 | 0.25 | 0.25 | 0.25 | - | - |
| SRP 1 | - | - | - | 0.2 | - | 0.2 |
| EDDS | - | 0.25 | 0.4 | - | 0.5 | 0.5 |
| CFAA | - | 1.0 | - | 2.0 | - | - |
| HEDP | 0.3 | 0.3 | 0.3 | 0.3 | 0.4 | 0.4 |
| QEA | - | - | - | 0.2 | - | 0.5 |
| Oxygenase | 0.05 | 0.05 | 0.02 | 0.002 | 0.005 | 0.005 |
| Protease | 0.009 | 0.009 | 0.01 | 0.04 | 0.05 | 0.03 |
| Amylase | 0.002 | 0.002 | 0.002 | 0.006 | 0.008 | 0.008 |
| Cellulase | 0.0007 | - | - | 0.0007 | 0.0007 | 0.0007 |
| Lipase | 0.006 | - | - | 0.01 | 0.01 | 0.01 |
| Photoactivated | 15 | 15 | 15 | - | 20 | 20 |
| bleach (ppm) | | | | | | |

| | **I** | **II** | **III** | **IV** | **V** | **VI** |
|---|---|---|---|---|---|---|
| PVNO/PVPVI | - | - | - | 0.1 | - | - |
| Brightener 1 | 0.09 | 0.09 | 0.09 | - | 0.09 | 0.09 |
| Perfume | 0.3 | 0.3 | 0.3 | 0.4 | 0.4 | 0.4 |
| Silicone antifoam | 0.5 | 0.5 | 0.5 | - | 0.3 | 0.3 |
| Density in g/litre | 850 | 850 | 850 | 850 | 850 | 850 |
| Miscellaneous and minors | | | Up to 100% | | | |

### Example 2

The following granular laundry detergent compositions of particular utility under European machine wash conditions were prepared according to the present invention :

| | **I** | **II** | **III** | **IV** | **V** | **VI** |
|---|---|---|---|---|---|---|
| LAS | 5.5 | 7.5 | 5.0 | 5.0 | 6.0 | 7.0 |
| TAS | 1.25 | 1.9 | - | 0.8 | 0.4 | 0.3 |
| C24AS/C25AS | - | 2.2 | 5.0 | 5.0 | 5.0 | 2.2 |
| C25E3S | - | 0.8 | 1.0 | 1.5 | 3.0 | 1.0 |
| C45E7 | 3.25 | - | - | - | - | 3.0 |
| TFAA | - | - | 2.0 | - | - | - |
| C25E5 | - | 5.5 | - | - | - | - |
| QAS | 0.8 | - | - | - | - | - |
| QAS 1 | - | 0.7 | 1.0 | 0.5 | 1.0 | 0.7 |
| STPP | 19.7 | - | - | - | - | - |
| Zeolite A | - | 19.5 | 25.0 | 19.5 | 20.0 | 17.0 |
| NaSKS-6/citric acid | - | 10.6 | - | 10.6 | - | - |
| (79:21) | | | | | | |
| Na-SKS-6 | - | - | 9.0 | - | 10.0 | 10.0 |
| Carbonate | 6.1 | 21.4 | 9.0 | 10.0 | 10.0 | 18.0 |
| Bicarbonate | - | 2.0 | 7.0 | 5.0 | - | 2.0 |
| Silicate | 6.8 | - | - | 0.3 | 0.5 | - |
| Citrate | - | - | 4.0 | 4.0 | - | - |
| Sulfate | 39.8 | - | - | 5.0 | - | 12.0 |

| | **I** | **II** | **III** | **IV** | **V** | **VI** |
|---|---|---|---|---|---|---|
| Mg sulfate | - | - | 0.1 | 0.2 | 0.2 | - |
| MA/AA | 0.5 | 1.6 | 3.0 | 4.0 | 1.0 | 1.0 |
| CMC | 0.2 | 0.4 | 1.0 | 1.0 | 0.4 | 0.4 |
| PB4 | 5.0 | 12.7 | - | - | - | - |
| Percarbonate | - | - | - | - | 18.0 | 15.0 |
| TAED | 0.5 | 3.1 | - | - | 5.0 | - |
| NACA-OBS | 1.0 | 3.5 | - | - | - | 2.5 |
| DETPMP | 0.25 | 0.2 | 0.3 | 0.4 | - | 0.2 |
| HEDP | - | 0.3 | - | 0.3 | 0.3 | 0.3 |
| QEA | - | - | 1.0 | 1.0 | 1.0 | - |
| Oxygenase | 0.002 | 0.004 | 0.006 | 0.008 | 0.01 | 0.1 |
| Protease | 0.009 | 0.03 | 0.03 | 0.05 | 0.05 | 0.02 |
| Lipase | 0.003 | 0.003 | 0.006 | 0.006 | 0.006 | 0.004 |
| Cellulase | 0.0006 | 0.0006 | 0.0005 | 0.0005 | 0.0007 | 0.0007 |
| Amylase | 0.002 | 0.002 | 0.006 | 0.006 | 0.01 | 0.003 |
| PVNO/PVPVI | - | - | 0.2 | 0.2 | - | - |
| PVP | 0.9 | 1.3 | - | - | - | 0.9 |
| SRP 1 | - | - | 0.2 | 0.2 | 0.2 | - |
| Photoactivated | 15 | 27 | - | - | 20 | 20 |
| bleach (ppm) | | | | | | |
| Photoactivated | 15 | - | - | - | - | - |
| bleach (1) (ppm) | | | | | | |
| Brightener 1 | 0.08 | 0.2 | - | - | 0.09 | 0.15 |
| Brightener 2 | - | 0.04 | - | - | - | - |
| Perfume | 0.3 | 0.5 | 0.4 | 0.3 | 0.4 | 0.3 |
| Silicone antifoam | 0.5 | 2.4 | 0.3 | 0.5 | 0.3 | 2.0 |
| Density in g/litre | 750 | 750 | 750 | 750 | 750 | 750 |
| Miscellaneous and minors | | | Up to 100% | | | |

### Example 3

The following detergent compositions of particular utility under European machine wash conditions were prepared according to the present invention :

| | **I** | **II** | **III** | **IV** |
|---|---|---|---|---|
| Blown Powder | | | | |
| LAS | 6.0 | 5.0 | 11.0 | 6.0 |
| TAS | 2.0 | - | - | 2.0 |
| Zeolite A | 24.0 | - | - | 20.0 |
| STPP | - | 27.0 | 24.0 | - |
| Sulfate | 4.0 | 6.0 | 13.0 | - |
| MA/AA | 1.0 | 4.0 | 6.0 | 2.0 |
| Silicate | 1.0 | 7.0 | 3.0 | 3.0 |
| CMC | 1.0 | 1.0 | 0.5 | 0.6 |
| Brightener 1 | 0.2 | 0.2 | 0.2 | 0.2 |
| Silicone antifoam | 1.0 | 1.0 | 1.0 | 0.3 |
| DETPMP | 0.4 | 0.4 | 0.2 | 0.4 |

| Spray On | | | | |
|---|---|---|---|---|
| Brightener | 0.02 | - | - | 0.02 |
| C45E7 | - | - | - | 5.0 |
| C45E2 | 2.5 | 2.5 | 2.0 | - |
| C45E3 | 2.6 | 2.5 | 2.0 | - |
| Perfume | 0.5 | 0.3 | 0.5 | 0.2 |
| Silicone antifoam | 0.3 | 0.3 | 0.3 | - |

| Dry additives | | | | |
|---|---|---|---|---|
| QEA | - | - | - | 1.0 |
| EDDS | 0.3 | - | - | - |
| Sulfate | 2.0 | 3.0 | 5.0 | 10.0 |
| Carbonate | 6.0 | 13.0 | 15.0 | 14.0 |
| Citric | 2.5 | - | - | 2.0 |
| QAS 1 | 0.5 | - | - | 0.5 |
| Na-SKS-6 | 10.0 | - | - | - |
| Percarbonate | 18.5 | - | - | - |
| PB4 | - | 18.0 | 10.0 | 21.5 |
| TAED | 2.0 | 2.0 | - | 2.0 |
| NACA-OBS | 3.0 | 2.0 | 4.0 | - |
| Oxygenase | 0.05 | 0.07 | 0.1 | 0.01 |
| Protease | 0.03 | 0.03 | 0.03 | 0.03 |
| Lipase | 0.008 | 0.008 | 0.008 | 0.004 |
| Amylase | 0.003 | 0.003 | 0.003 | 0.006 |

| | **I** | **II** | **III** | **IV** |
|---|---|---|---|---|
| Brightener 1 | 0.05 | - | - | 0.05 |
| Miscellaneous and minors | | Up to 100% | | |

### Example 4

The following granular detergent compositions were prepared according to the present invention :

| | **I** | **II** | **III** | **IV** | **V** | **VI** |
|---|---|---|---|---|---|---|
| Blown Powder | | | | | | |
| LAS | 23.0 | 8.0 | 7.0 | 9.0 | 7.0 | 7.0 |
| TAS | - | - | - | - | 1.0 | - |
| C45AS | 6.0 | 6.0 | 5.0 | 8.0 | - | - |
| C45AES | - | 1.0 | 1.0 | 1.0 | - | - |
| C45E35 | - | - | - | - | 2.0 | 4.0 |
| Zeolite A | 10.0 | 18.0 | 14.0 | 12.0 | 10.0 | 10.0 |
| MA/AA | - | 0.5 | - | - | - | 2.0 |
| MA/AA 1 | 7.0 | - | - | - | - | - |
| AA | - | 3.0 | 3.0 | 2.0 | 3.0 | 3.0 |
| Sulfate | 5.0 | 6.3 | 14.3 | 11.0 | 15.0 | 19.3 |
| Silicate | 10.0 | 1.0 | 1.0 | 1.0 | 1.0 | 1.0 |
| Carbonate | 15.0 | 20.0 | 10.0 | 20.7 | 8.0 | 6.0 |
| PEG 4000 | 0.4 | 1.5 | 1.5 | 1.0 | 1.0 | 1.0 |
| DTPA | - | 0.9 | 0.5 | - | - | 0.5 |
| Brightener 2 | 0.3 | 0.2 | 0.3 | - | 0.1 | 0.3 |

| Spray On | | | | | | |
|---|---|---|---|---|---|---|
| C45E7 | - | 2.0 | - | - | 2.0 | 2.0 |
| C25E9 | 3.0 | - | - | - | - | - |
| C23E9 | - | - | 1.5 | 2.0 | - | 2.0 |
| Perfume | 0.3 | 0.3 | 0.3 | 2.0 | 0.3 | 0.3 |

| Agglomerates | | | | | | |
|---|---|---|---|---|---|---|
| C45AS | - | 5.0 | 5.0 | 2.0 | - | 5.0 |
| LAS | - | 2.0 | 2.0 | - | - | 2.0 |
| | | | | | | |
| | **I** | **II** | **III** | **IV** | **V** | **VI** |
| Zeolite A | - | 7.5 | 7.5 | 8.0 | - | 7.5 |
| Carbonate | - | 4.0 | 4.0 | 5.0 | - | 4.0 |
| PEG 4000 | - | 0.5 | 0.5 | - | - | 0.5 |
| Misc (Water etc.) | - | 2.0 | 2.0 | 2.0 | - | 2.0 |

| Dry additives | | | | | | |
|---|---|---|---|---|---|---|
| QAS | - | - | - | - | 1.0 | - |
| Citric | - | - | - | - | 2.0 | - |
| PB4 | - | - | - | - | 12.0 | 1.0 |
| PB1 | 4.0 | 1.0 | 3.0 | 2.0 | - | - |
| Percarbonate | - | - | - | - | 2.0 | 10.0 |
| Carbonate | - | 5.3 | 1.8 | - | 4.0 | 4.0 |
| NOBS | 4.0 | - | 6.0 | - | - | 0.6 |
| Methyl cellulose | 0.2 | - | - | - | - | - |
| Na-SKS-6 | 8.0 | - | - | - | - | - |
| STS | - | - | 2.0 | - | 1.0 | - |
| Culmene sulfonic | - | 1.0 | - | - | - | 2.0 |
| acid | | | | | | |
| Oxygenase | 0.2 | 0.02 | 0.05 | 0.09 | 0.1 | 0.05 |
| Protease | 0.02 | 0.02 | 0.02 | 0.01 | 0.02 | 0.02 |
| Lipase | 0.004 | - | 0.004 | - | 0.004 | 0.008 |
| Amylase | 0.003 | - | 0.002 | - | 0.003 | - |
| Cellulase | 0.0005 | 0.0005 | 0.0005 | 0.0007 | 0.0005 | 0.0005 |
| PVPVI | - | - | - | - | 0.5 | 0.1 |
| PVP | - | - | - | - | 0.5 | - |
| PVNO | - | - | 0.5 | 0.3 | - | - |
| QEA | - | - | - | - | 1.0 | - |
| SRP 1 | 0.2 | 0.5 | 0.3 | - | 0.2 | - |
| Silicone antifoam | 0.2 | 0.4 | 0.2 | 0.4 | 0.1 | - |
| Mg sulfate | - | - | 0.2 | - | 0.2 | - |
| Miscellaneous and minors | | | Up to 100% | | | |

### Example 5

The following nil bleach-containing detergent compositions of particular use in the washing of coloured clothing were prepared according to the present invention :

| | | **I** | **II** | **III** |
|---|---|---|---|---|
| Blown Powder | | | | |
| | Zeolite A | 15.0 | 15.0 | - |
| | Sulfate | - | 5.0 | - |
| | LAS | 3.0 | 3.0 | - |
| | DETPMP | 0.4 | 0.5 | - |
| | CMC | 0.4 | 0.4 | - |
| | MA/AA | 4.0 | 4.0 | - |

| Agglomerates | | | | |
|---|---|---|---|---|
| | C45AS | - | - | 11.0 |
| | LAS | 6.0 | 5.0 | - |
| | TAS | 3.0 | 2.0 | - |
| | Silicate | 4.0 | 4.0 | - |
| | Zeolite A | 10.0 | 15.0 | 13.0 |
| | CMC | - | - | 0.5 |
| | MA/AA | - | - | 2.0 |
| | Carbonate | 9.0 | 7.0 | 7.0 |

| Spray-on | | | | |
|---|---|---|---|---|
| | Perfume | 0.3 | 0.3 | 0.5 |
| | C45E7 | 4.0 | 4.0 | 4.0 |
| | C25E3 | 2.0 | 2.0 | 2.0 |

| Dry additives | | | | |
|---|---|---|---|---|
| | MA/AA | - | - | 3.0 |
| | Na-SKS-6 | - | - | 12.0 |
| | Citrate | 10.0 | - | 8.0 |
| | Bicarbonate | 7.0 | 3.0 | 5.0 |
| | Carbonate | 8.0 | 5.0 | 7.0 |
| | PVPVI/PVNO | 0.5 | 0.5 | 0.5 |
| | Oxygenase | 0.0009 | 0.002 | 0.005 |
| | Protease | 0.03 | 0.02 | 0.05 |
| | Lipase | 0.008 | 0.008 | 0.008 |
| | Amylase | 0.01 | 0.01 | 0.01 |
| | | | | |

| | | **I** | **II** | **III** |
|---|---|---|---|---|
| | Cellulase | 0.001 | 0.001 | 0.001 |
| | Silicone antifoam | 5.0 | 5.0 | 5.0 |
| | Sulfate | - | 9.0 | - |
| Density (g/litre) | | 700 | 700 | 700 |
| Miscellaneous and minors | | | Up to 100% | |

### Example 6

The following detergent compositions were prepared according to the present invention :

| | | **I** | **II** | **III** | **IV** |
|---|---|---|---|---|---|
| Base granule | | | | | |
| | Zeolite A | 30.0 | 22.0 | 24.0 | 10.0 |
| | Sulfate | 10.0 | 5.0 | 10.0 | 7.0 |
| | MA/AA | 3.0 | - | - | - |
| | AA | - | 1.6 | 2.0 | - |
| | MA/AA 1 | - | 12.0 | - | 6.0 |
| | LAS | 14.0 | 10.0 | 9.0 | 20.0 |
| | C45AS | 8.0 | 7.0 | 9.0 | 7.0 |
| | C45AES | - | 1.0 | 1.0 | - |
| | Silicate | - | 1.0 | 0.5 | 10.0 |
| | Soap | - | 2.0 | - | - |
| | Brightener 1 | 0.2 | 0.2 | 0.2 | 0.2 |
| | Carbonate | 6.0 | 9.0 | 10.0 | 10.0 |
| | PEG 4000 | - | 1.0 | 1.5 | - |
| | DTPA | - | 0.4 | - | - |

| Spray On | | | | | |
|---|---|---|---|---|---|
| | C25E9 | - | - | - | 5.0 |
| | C45 E7 | 1.0 | 1.0 | - | - |
| | C23E9 | - | 1.0 | 2.5 | - |
| | Perfume | 0.2 | 0.3 | 0.3 | - |

| Dry additives | | | | | |
|---|---|---|---|---|---|
| | | **I** | **II** | **III** | **IV** |
| | Carbonate | 5.0 | 10.0 | 18.0 | 8.0 |
| | PVPVI/PVNO | 0.5 | - | 0.3 | - |
| | Oxygenase | 0.002 | 0.05 | 0.0015 | 0.1 |
| | Protease | 0.03 | 0.03 | 0.03 | 0.02 |
| | Lipase | 0.008 | - | - | 0.008 |
| | Amylase | 0.002 | - | - | 0.002 |
| | Cellulase | 0.0002 | 0.0005 | 0.0005 | 0.0002 |
| | NOBS | - | 4.0 | - | 4.5 |
| | PB1 | 1.0 | 5.0 | 1.5 | 6.0 |
| | Sulfate | 4.0 | 5.0 | - | 5.0 |
| | SRP 1 | - | 0.4 | - | - |
| | Suds suppressor | - | 0.5 | 0.5 | - |
| | Miscellaneous and minors | | Up | to 100% | |

### Example 7

The following granular detergent compositions were prepared according to the present invention :

| | | **I** | **II** | **III** |
|---|---|---|---|---|
| Blown Powder | | | | |
| | Zeolite A | 20.0 | - | 15.0 |
| | STPP | - | 20.0 | - |
| | Sulfate | - | - | 5.0 |
| | Carbonate | - | - | 5.0 |
| | TAS | - | - | 1.0 |
| | LAS | 6.0 | 6.0 | 6.0 |
| | C68AS | 2.0 | 2.0 | - |
| | Silicate | 3.0 | 8.0 | - |
| | MAIAA | 4.0 | 2.0 | 2.0 |
| | CMC | 0.6 | 0.6 | 0.2 |
| | Brightener 1 | 0.2 | 0.2 | 0.1 |
| | DETPMP | 0.4 | 0.4 | 0.1 |
| | STS | - | - | 1.0 |
| | | | | |

| | | **I** | **II** | **III** |
|---|---|---|---|---|
| Spray On | | | | |
| | C45E7 | 5.0 | 5.0 | 4.0 |
| | Silicone antifoam | 0.3 | 0.3 | 0.1 |
| | Perfume | 0.2 | 0.2 | 0.3 |

| Dry additives | | | | |
|---|---|---|---|---|
| | QEA | - | - | 1.0 |
| | Carbonate | 14.0 | 9.0 | 10.0 |
| | PB1 | 1.5 | 2.0 | - |
| | PB4 | 18.5 | 13.0 | 13.0 |
| | TAED | 2.0 | 2.0 | 2.0 |
| | QAS | - | - | 1.0 |
| | Photoactivated bleach | 15 ppm | 15 ppm | 15 ppm |
| | Na-SKS-6 | - | - | 3.0 |
| | Oxygenase | 0.01 | 0.02 | 0.007 |
| | Protease | 0.03 | 0.03 | 0.007 |
| | Lipase | 0.004 | 0.004 | 0.004 |
| | Amylase | 0.006 | 0.006 | 0.003 |
| | Cellulase | 0.0002 | 0.0002 | 0.0005 |
| | Sulfate | 10.0 | 20.0 | 5.0 |
| Density (g/litre) | | 700 | 700 | 700 |
| Miscellaneous and minors | | | Up to 100% | |

### Example 8

The following detergent compositions were prepared according to the present invention :

| | | **I** | **II** | **III** |
|---|---|---|---|---|
| Blown Powder | | | | |
| | Zeolite A | 15.0 | 15.0 | 15.0 |
| | Sulfate | - | 5.0 | - |
| | LAS | 3.0 | 3.0 | 3.0 |
| | QAS | - | 1.5 | 1.5 |
| | DETPMP | 0.4 | 0.2 | 0.4 |
| | | | | |
| | | **I** | **II** | **III** |
| | EDDS | - | 0.4 | 0.2 |
| | CMC | 0.4 | 0.4 | 0.4 |
| | MA/AA | 4.0 | 2.0 | 2.0 |

| Agglomerate | | | | |
|---|---|---|---|---|
| | LAS | 5.0 | 5.0 | 5.0 |
| | TAS | 2.0 | 2.0 | 1.0 |
| | Silicate | 3.0 | 3.0 | 4.0 |
| | Zeolite A | 8.0 | 8.0 | 8.0 |
| | Carbonate | 8.0 | 8.0 | 4.0 |

| Spray On | | | | |
|---|---|---|---|---|
| | Perfume | 0.3 | 0.3 | 0.3 |
| | C45E7 | 2.0 | 2.0 | 2.0 |
| | C25E3 | 2.0 | - | - |

| Dry Additives | | | | |
|---|---|---|---|---|
| | Citrate | 5.0 | - | 2.0 |
| | Bicarbonate | - | 3.0 | - |
| | Carbonate | 8.0 | 15.0 | 10.0 |
| | TAED | 6.0 | 2.0 | 5.0 |
| | PB1 | 14.0 | 7.0 | 10.0 |
| | PEO | - | - | 0.2 |
| | Bentonite clay | - | - | 10.0 |
| | Oxygenase | 0.008 | 0.01 | 0.009 |
| | Protease | 0.03 | 0.03 | 0.03 |
| | Lipase | 0.008 | 0.008 | 0.008 |
| | Cellulase | 0.001 | 0.001 | 0.001 |
| | Amylase | 0.01 | 0.01 | 0.01 |
| | Silicone antifoam | 5.0 | 5.0 | 5.0 |
| | Sulfate | - | 3.0 | - |
| Density (g/litre) | | 850 | 850 | 850 |
| Miscellaneous and minors | | | Up to 100% | |

### Example 9

The following detergent compositions were prepared according to the present invention :

| | **I** | **II** | **III** | **IV** |
|---|---|---|---|---|
| | | | | |
| LAS | 18.0 | 14.0 | 24.0 | 20.0 |
| QAS | 0.7 | 1.0 | - | 0.7 |
| TFAA | - | 1.0 | - | - |
| C23E56.5 | - | - | 1.0 | - |
| C45E7 | - | 1.0 | - | - |
| C45E3S | 1.0 | 2.5 | 1.0 | - |
| STPP | 32.0 | 18.0 | 30.0 | 22.0 |
| Silicate | 9.0 | 5.0 | 9.0 | 8.0 |
| Carbonate | 11.0 | 7.5 | 10.0 | 5.0 |
| Bicarbonate | - | 7.5 | - | - |
| PB1 | 3.0 | 1.0 | - | - |
| PB4 | - | 1.0 | - | - |
| NOBS | 2.0 | 1.0 | - | - |
| DETPMP | - | 1.0 | - | - |
| DTPA | 0.5 | - | 0.2 | 0.3 |
| SRP 1 | 0.3 | 0.2 | - | 0.1 |
| MA/AA | 1.0 | 1.5 | 2.0 | 0.5 |
| CMC | 0.8 | 0.4 | 0.4 | 0.2 |
| PEI | - | - | 0.4 | - |
| Sulfate | 20.0 | 10.0 | 20.0 | 30.0 |
| Mg sulfate | 0.2 | - | 0.4 | 0.9 |
| Oxygenase | 0.01 | 0.01 | 0.02 | 0.02 |
| Protease | 0.03 | 0.03 | 0.02 | 0.02 |
| Amylase | 0.008 | 0.007 | - | 0.004 |
| Lipase | 0.004 | - | 0.002 | - |
| Cellulase | 0.0003 | - | - | 0.0001 |
| Photoactivated bleach | 30 ppm | 20 ppm | - | 10 ppm |
| Perfume | 0.3 | 0.3 | 0.1 | 0.2 |
| Brightener 1/2 | 0.05 | 0.02 | 0.08 | 0.1 |
| Miscellaneous and minors | | up to 100% | | |

### Example 10

The following liquid detergent formulations were prepared according to the present invention (Levels are given in parts per weight, enzymes are expressed in pure enzyme) :

| | **I** | **II** | **III** | **IV** | **V** |
|---|---|---|---|---|---|
| | | | | | |
| LAS | 11.5 | 8.8 | - | 3.9 | - |
| C25E2.5S | - | 3.0 | 18.0 | - | 16.0 |
| C45E2.25S | 11.5 | 3.0 | - | 15.7 | - |
| C23E9 | - | 2.7 | 1.8 | 2.0 | 1.0 |
| C23E7 | 3.2 | - | - | - | - |
| CFAA | - | - | 5.2- | - | 3.1 |
| TPKFA | 1.6 | - | 2.0 | 0.5 | 2.0 |
| Citric (50%) | 6.5 | 1.2 | 2.5 | 4.4 | 2.5 |
| Ca formate | 0.1 | 0.06 | 0.1 | - | - |
| Na formate | 0.5 | 0.06 | 0.1 | 0.05 | 0.05 |
| SCS | 4.0 | 1.0 | 3.0 | 1.2 | - |
| Borate | 0.6 | - | 3.0 | 2.0 | 2.9 |
| Na hydroxide | 5.8 | 2.0 | 3.5 | 3.7 | 2.7 |
| Ethanol | 1.75 | 1.0 | 3.6 | 4.2 | 2.9 |
| 1,2 Propanediol | 3.3 | 2.0 | 8.0 | 7.9 | 5.3 |
| Monoethanolamine | 3.0 | 1.5 | 1.3 | 2.5 | 0.8 |
| TEPAE | 1.6 | - | 1.3 | 1.2 | 1.2 |
| Oxygenase | 0.007 | 0.005 | 0.002 | 0.01 | 0.01 |
| Protease | 0.03 | 0.01 | 0.03 | 0.02 | 0.02 |
| Lipase | - | - | 0.002 | - | - |
| Amylase | - | - | - | 0.002 | - |
| Cellulase | - | - | 0.0002 | 0.0005 | 0.0001 |
| SRP 1 | 0.2 | - | 0.1 | - | - |
| DTPA | - | - | 0.3 | - | - |
| PVNO | - | - | 0.3 | - | 0.2 |
| Brightener 1 | 0.2 | 0.07 | 0.1 | - | - |
| Silicone antifoam | 0.04 | 0.02 | 0.1 | 0.1 | 0.1 |
| Miscellaneous and water | | | | | |

### Example 11

The following liquid detergent formulations were prepared according to the present invention (Levels are given in parts per weight, enzymes are expressed in pure enzyme) :

| | **I** | **II** | **III** | **IV** |
|---|---|---|---|---|
| LAS | 10.0 | 13.0 | 9.0 | - |
| C25AS | 4.0 | 1.0 | 2.0 | 10.0 |
| C25E3S | 1.0 | - | - | 3.0 |
| C25E7 | 6.0 | 8.0 | 13.0 | 2.5 |
| TFAA | - | - | - | 4.5 |
| APA | - | 1.4 | - | - |
| TPKFA | 2.0 | - | 13.0 | 7.0 |
| Citric | 2.0 | 3.0 | 1.0 | 1.5 |
| Dodecenyl / tetradecenyl succinic acid | 12.0 | 10.0 | - | - |
| Rapeseed fatty acid | 4.0 | 2.0 | 1.0 | - |
| Ethanol | 4.0 | 4.0 | 7.0 | 2.0 |
| 1,2 Propanediol | 4.0 | 4.0 | 2.0 | 7.0 |
| Monoethanolamine | - | - | - | 5.0 |
| Triethanolamine | - | - | 8.0 | - |
| TEPAE | 0.5 | - | 0.5 | 0.2 |
| DETPMP | 1.0 | 1.0 | 0.5 | 1.0 |
| Oxygenase | 0.02 | 0.02 | 0.009 | 0.009 |
| Protease | 0.02 | 0.02 | 0.01 | 0.008 |
| Lipase | - | 0.002 | - | 0.002 |
| Amylase | 0.004 | 0.004 | 0.01 | 0.008 |
| Cellulase | - | - | - | 0.002 |
| SRP 2 | 0.3 | - | 0.3 | 0.1 |
| Boric acid | 0.1 | 0.2 | 1.0 | 2.0 |
| Ca chloride | - | 0.02 | - | 0.01 |
| Brightener 1 | - | 0.4 | - | - |
| Suds suppressor | 0.1 | 0.3 | - | 0.1 |
| Opacifier | 0.5 | 0.4 | - | 0.3 |
| NaOH up to pH | 8.0 | 8.0 | 7.6 | 7.7 |
| Miscellaneous and water | | | | |

### Example 12

The following liquid detergent compositions were prepared according to the present invention (Levels are given in parts per weight, enzymes are expressed in pure enzyme) :

| | **I** | **II** | **III** | **IV** |
|---|---|---|---|---|
| LAS | 25.0 | - | - | - |
| C25AS | - | 13.0 | 18.0 | 15.0 |
| C25E3S | - | 2.0 | 2.0 | 4.0 |
| C25E7 | - | - | 4.0 | 4.0 |
| TFAA | - | 6.0 | 8.0 | 8.0 |
| APA | 3.0 | 1.0 | 2.0 | - |
| TPKFA | - | 15.0 | 11.0 | 11.0 |
| Citric | 1.0 | 1.0 | 1.0 | 1.0 |
| Dodecenyl / tetradecenyl succinic | 15.0 | - | - | - |
| acid | | | | |
| Rapeseed fatty acid | 1.0 | - | 3.5 | - |
| Ethanol | 7.0 | 2.0 | 3.0 | 2.0 |
| 1,2 Propanediol | 6.0 | 8.0 | 10.0 | 13.0 |
| Monoethanolamine | - | - | 9.0 | 9.0 |
| TEPAE | - | - | 0.4 | 0.3 |
| DETPMP | 2.0 | 1.2 | 1.0 | - |
| Oxygenase | 0.02 | 0.04 | 0.08 | 0.1 |
| Protease | 0.08 | 0.02 | 0.01 | 0.02 |
| Lipase | - | - | 0.003 | 0.003 |
| Amylase | 0.004 | 0.01 | 0.01 | 0.01 |
| Cellulase | - | - | 0.004 | 0.003 |
| SRP 2 | - | - | 0.2 | 0.1 |
| Boric acid | 1.0 | 1.5 | 2.5 | 2.5 |
| Bentonite clay | 4.0 | 4.0 | - | - |
| Brightener 1 | 0.1 | 0.2 | 0.3 | - |
| Suds suppressor | 0.4 | - | - | - |
| Opacifier | 0.8 | 0.7 | - | - |
| NaOH up to pH | 8.0 | 7.5 | 8.0 | 8.2 |
| Miscellaneous and water | | | | |

### Example 13

The following liquid detergent compositions were prepared according to the present invention (Levels are given in parts by weight, enzymes are expressed in pure enzyme) :

| | **I** | **II** |
|---|---|---|
| LAS | 27.6 | 18.9 |
| C45AS | 13.8 | 5.9 |
| C13E8 | 3.0 | 3.1 |
| Oleic acid | 3.4 | 2.5 |
| Citric | 5.4 | 5.4 |
| Na hydroxide | 0.4 | 3.6 |
| Ca Formate | 0.2 | 0.1 |
| Na Formate | - | 0.5 |
| Ethanol | 7.0 | - |
| Monoethanolamine | 16.5 | 8.0 |
| 1,2 propanediol | 5.9 | 5.5 |
| Xylene sulfonic acid | - | 2.4 |
| TEPAE | 1.5 | 0.8 |
| Protease | 0.05 | 0.02 |
| Oxygenase | 0.05 | 0.08 |
| PEG | - | 0.7 |
| Brightener 2 | 0.4 | 0.1 |
| Perfume | 0.5 | 0.3 |
| Miscellaneous and water | | |

### Example 14

The following granular fabric detergent compositions which provide "softening through the wash" capability were prepared according to the present invention :

| | **I** | **II** |
|---|---|---|
| C45AS | - | 10.0 |
| LAS | 7.6 | - |
| C68AS | 1.3 | - |
| C45E7 | 4.0 | - |
| | | |

| | **I** | **II** |
|---|---|---|
| C25E3 | - | 5.0 |
| Coco-alkyl-dimethyl hydroxy- | 1.4 | 1.0 |
| ethyl ammonium chloride | | |
| Citrate | 5.0 | 3.0 |
| Na-SKS-6 | - | 11.0 |
| Zeolite A | 15.0 | 15.0 |
| MA/AA | 4.0 | 4.0 |
| DETPMP | 0.4 | 0.4 |
| PB1 | 15.0 | - |
| Percarbonate | - | 15.0 |
| TAED | 5.0 | 5.0 |
| Smectite clay | 10.0 | 10.0 |
| HMWPEO | - | 0.1 |
| Oxygenase | 0.05 | 0.05 |
| Protease | 0.02 | 0.01 |
| Lipase | 0.02 | 0.01 |
| Amylase | 0.03 | 0.005 |
| Cellulase | 0.001 | - |
| Silicate | 3.0 | 5.0 |
| Carbonate | 10.0 | 10.0 |
| Suds suppressor | 1.0 | 4.0 |
| CMC | 0.2 | 0.1 |
| Water and minors | Up to 100% | |

### Example 15

The following rinse added fabric softener composition was prepared according to the present invention :

| | |
|---|---|
| DEQA (2) | 20.0 |
| Oxygenase | 0.01 |
| Cellulase | 0.001 |
| HCL | 0.03 |
| Antifoam agent | 0.01 |
| Blue dye | 25ppm |
| CaCl₂ | 0.20 |
| Perfume | 0.90 |
| Miscellaneous and water | Up to 100% |

### Example 16

The following fabric softener and dryer added fabric conditioner compositions were prepared according to the present invention :

| | **I** | **II** | **III** | **IV** | **V** |
|---|---|---|---|---|---|
| DEQA | 2.6 | 19.0 | - | - | - |
| DEQA(2) | - | - | - | - | 51.8 |
| DTMAMS | - | - | - | 26.0 | - |
| SDASA | - | - | 70.0 | 42.0 | 40.2 |
| Stearic acid of IV=0 | 0.3 | - | - | - | - |
| Neodol 45-13 | - | - | 13.0 | - | - |
| Hydrochloride acid | 0.02 | 0.02 | - | - | - |
| Ethanol | - | - | 1.0 | - | - |
| Oxygenase | 0.02 | 0.05 | 0.07 | 0.07 | 0.1 |
| Perfume | 1.0 | 1.0 | 0.75 | 1.0 | 1.5 |
| Glycoperse S-20 | - | - | - | - | 15.4 |
| Glycerol monostearate | - | - | - | 26.0 | - |
| Digeranyl Succinate | - | - | 0.38 | - | - |
| Silicone antifoam | 0.01 | 0.01 | - | - | - |
| Electrolyte | - | 0.1 | - | - | - |
| Clay | - | - | - | 3.0 | - |
| Dye | 10ppm | 25ppm | 0.01 | - | - |
| Water and minors | 100% | 100% | - | - | - |

### Example 17

The following laundry bar detergent compositions were prepared according to the present invention (Levels are given in parts per weight, enzymes are expressed in pure enzyme) :

| | **I** | **II** | **III** | **VI** | **V** | **III** | **VI** | **V** |
|---|---|---|---|---|---|---|---|---|
| LAS | - | - | 19.0 | 15.0 | 21.0 | 6.75 | 8.8 | - |
| C28AS | 30.0 | 13.5 | - | - | - | 15.75 | 11.2 | 22.5 |
| Na Laurate | 2.5 | 9.0 | - | - | - | - | - | - |
| Zeolite A | 2.0 | 1.25 | - | - | - | 1.25 | 1.25 | 1.25 |
| Carbonate | 20.0 | 3.0 | 13.0 | 8.0 | 10.0 | 15.0 | 15.0 | 10.0 |
| Ca Carbonate | 27.5 | 39.0 | 35.0 | - | - | 40.0 | - | 40.0 |
| Sulfate | 5.0 | 5.0 | 3.0 | 5.0 | 3.0 | - | - | 5.0 |
| TSPP | 5.0 | - | - | - | - | 5.0 | 2.5 | - |
| STPP | 5.0 | 15.0 | 10.0 | - | - | 7.0 | 8.0 | 10.0 |
| Bentonite clay | - | 10.0 | - | - | 5.0 | - | - | - |
| DETPMP | - | 0.7 | 0.6 | - | 0.6 | 0.7 | 0.7 | 0.7 |
| CMC | - | 1.0 | 1.0 | 1.0 | 1.0 | - | - | 1.0 |
| Talc | - | - | 10.0 | 15.0 | 10.0 | - | - | - |
| Silicate | - | - | 4.0 | 5.0 | 3.0 | - | - | - |
| PVNO | 0.02 | 0.03 | - | 0.01 | - | 0.02 | - | - |
| MA/AA | 0.4 | 1.0 | - | - | 0.2 | 0.4 | 0.5 | 0.4 |
| SRP 1 | 0.3 | 0.3 | 0.3 | 0.3 | 0.3 | 0.3 | 0.3 | 0.3 |
| Oxygenase | 0.001 | 0.002 | 0.005 | 0.005 | 0.008 | 0.01 | 0.01 | 0.5 |
| Amylase | - | - | 0.01 | - | - | - | 0.002 | - |
| Protease | - | 0.004 | - | 0.003 | 0.003 | - | - | 0.003 |
| Lipase | - | 0.002 | - | 0.002 | - | - | - | - |
| Cellulase | - | .0003 | - | - | .0003 | .0002 | - | - |
| PEO | - | 0.2 | - | 0.2 | 0.3 | - | - | 0.3 |
| Perfume | 1.0 | 0.5 | 0.3 | 0.2 | 0.4 | - | - | 0.4 |
| Mg sulfate | - | - | 3.0 | 3.0 | 3.0 | - | - | - |
| Brightener | 0.15 | 0.1 | 0.15 | - | - | - | - | 0.1 |
| Photoactivated | - | 15.0 | 15.0 | 15.0 | 15.0 | - | - | 15.0 |
| bleach (ppm) | | | | | | | | |

### Example 18

The following detergent additive compositions were prepared according to the present invention :

| | **I** | **II** | **III** |
|---|---|---|---|
| LAS | - | 5.0 | 5.0 |
| STPP | 30.0 | - | 20.0 |
| Zeolite A | - | 35.0 | 20.0 |
| PB1 | 20.0 | 15.0 | - |
| TAED | 10.0 | 8.0 | - |
| Protease | - | 0.3 | 0.3 |
| Amylase | - | 0.06 | 0.06 |
| Oxygenase | 0.1 | 0.1 | 0.1 |
| Minors, water and miscellaneous | | Up to 100% | |

### Example 19

The following compact high density (0.96Kg/l) dishwashing detergent compositions were prepared according to the present invention :

| | **I** | **II** | **III** | **IV** | **V** | **VI** | **VII** | **VIII** |
|---|---|---|---|---|---|---|---|---|
| STPP | - | - | 54.3 | 51.4 | 51.4 | - | - | 50.9 |
| Citrate | 35.0 | 17.0 | - | - | - | 46.1 | 40.2 | - |
| Carbonate | - | 17.5 | 14.0 | 14.0 | 14.0 | - | 8.0 | 32.1 |
| Bicarbonate | - | - | - | - | - | 25.4 | - | - |
| Silicate | 32.0 | 14.8 | 14.8 | 10.0 | 10.0 | 1.0 | 25.0 | 3.1 |
| Metasilicate | - | 2.5 | - | 9.0 | 9.0 | - | - | - |
| PB1 | 1.9 | 9.7 | 7.8 | 7.8 | 7.8 | - | - | - |
| PB4 | 8.6 | - | - | - | - | - | - | - |
| Percarbonate | - | - | - | - | - | 6.7 | 11.8 | 4.8 |
| Nonionic | 1.5 | 2.0 | 1.5 | 1.7 | 1.5 | 2.6 | 1.9 | 5.3 |
| TAED | 5.2 | 2.4 | - | - | - | 2.2 | - | 1.4 |
| HEDP | - | 1.0 | - | - | - | - | - | - |
| DETPMP | - | 0.6 | - | - | - | - | - | - |
| MnTACN | - | - | - | - | - | - | 0.008 | - |
| PAAC | - | - | 0.008 | 0.01 | 0.007 | - | - | - |
| BzP | - | - | - | - | 1.4 | - | - | - |
| Paraffin | 0.5 | 0.5 | 0.5 | 0.5 | 0.5 | 0.6 | - | - |
| Oxygenase | 0.002 | 0.004 | 0.008 | 0.01 | 0.02 | 0.05 | 0.1 | 0.2 |
| Protease | 0.072 | 0.072 | 0.029 | 0.053 | 0.046 | 0.026 | 0.059 | 0.06 |
| | | | | | | | | |

| | **I** | **II** | **III** | **IV** | **V** | **VI** | **VII** | **VIII** |
|---|---|---|---|---|---|---|---|---|
| | | | | | | | | |
| Amylase | 0.012 | 0.012 | 0.006 | 0.012 | 0.013 0.009 | 0.009 | 0.017 | 0.03 |
| Lipase | - | 0.001 | - | 0.005 | - | - | - | - |
| BTA | 0.3 | 0.3 | 0.3 | 0.3 | 0.3 - | - | 0.3 | 0.3 |
| MA/AA | - | - | - | - | - | - | 4.2 | - |
| 480N | 3.3 | 6.0 | - | - | - | - | - | 0.9 |
| Perfume | 0.2 | 0.2 | 0.2 | 0.2 | 0.2 | 0.2 | 0.1 | 0.1 |
| Sulphate | 7.0 | 20.0 | 5.0 | 2.2 | 0.8 | 12.0 | 4.6 | - |
| pH | 10.8 | 11.0 | 10.8 | 11.3 | 11.3 | 9.6 | 10.8 | 10.9 |
| Miscellaneous and water | | | | Up to 100% | | | | |

### Example 20

The following granular dishwashing detergent compositions of bulk density 1.02Kg/L were prepared according to the present invention :

| | **I** | **II** | **III** | **IV** | **V** | **VI** | **VII** | **VII** |
|---|---|---|---|---|---|---|---|---|
| | | | | | | | | |
| STPP | 30.0 | 30.0 | 33.0 | 34.2 | 29.6 | 31.1 | 26.6 | 17.6 |
| Carbonate | 30.5 | 30.5 | 31.0 | 30.0 | 23.0 | 39.4 | 4.2 | 45.0 |
| Silicate | 7.4 | 7.4 | 7.5 | 7.2 | 13.3 | 3.4 | 43.7 | 12.4 |
| Metasilicate | - | - | 4.5 | 5.1 | - | - | - | - |
| Percarbonate | - | - | - | - | - | 4.0 | - | - |
| PB1 | 4.4 | 4.2 | 4.5 | 4.5 | - | - | - | - |
| NADCC | - | - | - | - | 2.0 | - | 1.6 | 1.0 |
| Nonionic | 1.2 | 1.0 | 0.7 | 0.8 | 1.9 | 0.7 | 0.6 | 0.3 |
| TAED | 1.0 | - | - | - | - | 0.8 | - | - |
| PAAC | - | 0.004 | 0.004 | 0.004 | - | - | - | - |
| BzP | - | - | - | 1.4 | - | - | - | - |
| Paraffin | 0.25 | 0.25 | 0.25 | 0.25 | - | - | - | - |
| Oxygenase | 0.007 | 0.005 | 0.01 | 0.02 | 0.04 | 0.06 | 0.08 | 0.1 |
| Protease | 0.036 | 0.015 | 0.026 | 0.028 | - | 0.01 | - | - |
| Amylase | 0.003 | 0.003 | 0.006 | 0.006 | - | 0.006 | - | - |
| Lipase | 0.005 | - | 0.001 | - | - | - | - | - |
| BTA | 0.15 | 0.15 | 0.15 | 0.15 | - | - | - | - |
| | | | | | | | | |

| | **I** | **II** | **III** | **IV** | V | **VI** | **VII** | **VII** |
|---|---|---|---|---|---|---|---|---|
| | | | | | | | | |
| Perfume | 0.2 | 0.2 | 0.2 | 0.2 | 0.1 | 0.2 | 0.2 | - |
| Sulphate | 23.4 | 25.0 | 22.0 | 18.5 | 30.1 | 19.3 | 23.1 | 23.6 |
| pH | 10.8 | 10.8 | 11.3 | 11.3 | 10.7 | 11.5 | 12.7 | 10.9 |
| Miscellaneous and water | | | | Up to 100% | | | | |

### Example 21

The following tablet detergent compositions were prepared according to the present invention by compression of a granular dishwashing detergent composition at a pressure of 13KN/cm² using a standard 12 head rotary press:

| | **I** | **II** | **III** | **IV** | **V** | **VI** |
|---|---|---|---|---|---|---|
| | | | | | | |
| STPP | - | 48.8 | 49.2 | 38.0 | - | 46.8 |
| Citrate | 26.4 | - | - | - | 31.1 | - |
| Carbonate | - | 5.0 | 14.0 | 15.4 | 14.4 | 23.0 |
| Silicate | 26.4 | 14.8 | 15.0 | 12.6 | 17.7 | 2.4 |
| Oxygenase | 0.05 | 0.07 | 0.02 | 0.01 | 0.04 | 0.1 |
| Protease | 0.058 | 0.072 | 0.041 | 0.033 | 0.052 | 0.013 |
| Amylase | 0.01 | 0.012 | 0.012 | 0.007 | 0.016 | 0.002 |
| Lipase | 0.005 | - | - | - | - | - |
| PB1 | 1.6 | 7.7 | 12.2 | 10.6 | 15.7 | - |
| PB4 | 6.9 | - | - | - | - | 14.4 |
| Nonionic | 1.5 | 2.0 | 1.5 | 1.65 | 0.8 | 6.3 |
| PAAC | - | - | 0.02 | 0.009 | - | - |
| MnTACN | - | - | - | - | 0.007 | - |
| TAED | 4.3 | 2.5 | - | - | 1.3 | 1.8 |
| HEDP | 0.7 | - | - | 0.7 | - | 0.4 |
| DETPMP | 0.65 | - | - | - | - | - |
| Paraffin | 0.4 | 0.5 | 0.5 | 0.55 | - | - |
| BTA | 0.2 | 0.3 | 0.3 | 0.3 | - | - |
| PA30 | 3.2 | - | - | - | - | - |
| MA/AA | - | - | - | - | 4.5 | 0.55 |
| Perfume | - | - | 0.05 | 0.05 | 0.2 | 0.2 |
| | | | | | | |

| | **I** | **II** | **III** | **IV** | **V** | **VI** |
|---|---|---|---|---|---|---|
| | | | | | | |
| Sulphate | 24.0 | 13.0 | 2.3 | - | 10.7 | 3.4 |
| Weight of tablet | 25g | 25g | 20g | 30g | 18g | 20g |
| pH | 10.6 | 10.6 | 10.7 | 10.7 | 10.9 | 11.2 |
| Miscellaneous and water | | | Up to 100% | | | |

### Example 22

The following liquid dishwashing detergent compositions of density 1.40Kg/L were prepared according to the present invention :

| | **I** | **II** | **III** | **IV** |
|---|---|---|---|---|
| | | | | |
| STPP | 17.5 | 17.5 | 17.2 | 16.0 |
| Carbonate | 2.0 | - | 2.4 | - |
| Silicate | 5.3 | 6.1 | 14.6 | 15.7 |
| NaOCl | 1.15 | 1.15 | 1.15 | 1.25 |
| Polygen/carbopol | 1.1 | 1.0 | 1.1 | 1.25 |
| Nonionic | - | - | 0.1 | - |
| NaBz | 0.75 | 0.75 | - | - |
| Oxygenase | 0.008 | 0.005 | 0.01 | 0.02 |
| NaOH | - | 1.9 | - | 3.5 |
| KOH | 2.8 | 3.5 | 3.0 | - |
| pH | 11.0 | 11.7 | 10.9 | 11.0 |
| Sulphate, miscellaneous and water | | up to 100% | | |

### Example 23

The following liquid rinse aid compositions were prepared according to the present invention :

| | **I** | **II** | **III** |
|---|---|---|---|
| Nonionic | 12.0 | - | 14.5 |
| Nonionic blend | - | 64.0 | - |
| Citric | 3.2 | - | 6.5 |
| | | | |

| | **I** | **II** | **III** |
|---|---|---|---|
| | | | |
| HEDP | 0.5 | - | - |
| PEG | - | 5.0 | - |
| Oxygenase | 0.01 | 0.03 | 0.02 |
| SCS | 4.8 | - | 7.0 |
| Ethanol | 6.0 | 8.0 | - |
| pH of the liquid | 2.0 | 7.5 | / |

### Example 24

The following liquid dishwashing compositions were prepared according to the present invention :

| | **I** | **II** | **III** | **IV** | **V** |
|---|---|---|---|---|---|
| | | | | | |
| C17ES | 28.5 | 27.4 | 19.2 | 34.1 | 34.1 |
| Amine oxide | 2.6 | 5.0 | 2.0 | 3.0 | 3.0 |
| C12 glucose amide | - | - | 6.0 | - | - |
| Betaine | 0.9 | - | - | 2.0 | 2.0 |
| Xylene sulfonate | 2.0 | 4.0 | - | 2.0 | - |
| Neodol C11E9 | - | - | 5.0 | - | - |
| Polyhydroxy fatty acid amide | - | - | - | 6.5 | 6.5 |
| Sodium diethylene penta acetate | - | - | 0.03 | - | - |
| (40%) | | | | | |
| TAED | - | - | - | 0.06 | 0.06 |
| Sucrose | - | - | - | 1.5 | 1.5 |
| Ethanol | 4.0 | 5.5 | 5.5 | 9.1 | 9.1 |
| Alkyl diphenyl oxide disulfonate | - | - | - | - | 2.3 |
| Ca formate | - | - | - | 0.5 | 1.1 |
| Ammonium citrate | 0.06 | 0.1 | - | - | - |
| Na chloride | - | 1.0 | - | - | - |
| Mg chloride | 3.3 | - | 0.7 | - | - |
| Ca chloride | - | - | 0.4 | - | - |
| Na sulfate | - | - | 0.06 | - | - |
| Mg sulfate | 0.08 | - | - | - | - |
| | | | | | |

| | **I** | **II** | **III** | **IV** | **V** |
|---|---|---|---|---|---|
| | | | | | |
| Mg hydroxide | - | - | - | 2.2 | 2.2 |
| Na hydroxide | - | - | - | 1.1 | 1.1 |
| Hydrogen peroxide | 200ppm | 0.16 | 0.06 | - | - |
| Oxygenase | 0.01 | 0.01 | 0.02 | 0.02 | 0.2 |
| Protease | 0.017 | 0.005 | .0035 | 0.003 | 0.002 |
| Perfume | 0.18 | 0.09 | 0.09 | 0.2 | 0.2 |
| Water and minors | Up to 100% | | | | |

### Example 25

The following liquid hard surface cleaning compositions were prepared according to the present invention :

| | **I** | **II** | **II**I |
|---|---|---|---|
| | | | |
| Oxygenase | 0.1 | 0.002 | 0.01 |
| Amylase | 0.01 | 0.002 | 0.005 |
| Protease | 0.05 | 0.01 | 0.02 |
| EDTA* | 0.05 | 0.05 | 0.05 |
| Citrate | 2.9 | 2.9 | 2.9 |
| LAS | 0.5 | 0.5 | 0.5 |
| C12 AS | 0.5 | 0.5 | 0.5 |
| C12(E)S | 0.5 | 0.5 | 0.5 |
| C12,13 E6.5 nonionic | 7.0 | 7.0 | 7.0 |
| Perfume | 1.0 | 1.0 | 1.0 |
| Hexyl carbitol** | 1.0 | 1.0 | 1.0 |
| SCS | 1.3 | 1.3 | 1.3 |
| Water | Balance to 100% | | |

| | | | |
|---|---|---|---|
| *Na4 ethylenediamine diacetic acid **Diethylene glycol monohexyl ether ***All formulas adjusted to pH 7-12 | | | |

### Example 26

The following spray composition for cleaning of hard surfaces and removing household mildew was prepared according to the present invention :

| | |
|---|---|
| Oxygenase | 0.05 |
| Amylase | 0.01 |
| Protease | 0.01 |
| Na octyl sulfate | 2.0 |
| Na dodecyl sulfate | 4.0 |
| Na hydroxide | 0.8 |
| Silicate | 0.04 |
| Butyl carbitol* | 4.0 |
| Perfume | 0.35 |
| Water/minors | up to 100% |
| *Diethylene glycol monobutyl ether | |

### Example 27

The following lavatory cleansing block compositions were prepared according to the present invention.

| | **I** | **II** | **III** |
|---|---|---|---|
| C16-18 fatty alcohol/50EO | 80.0 | - | - |
| LAS | - | - | 80.0 |
| Nonionic | - | 1.0 | - |
| Oleoamide surfactant | - | 26.0 | - |
| Partially esterified copolymer of vinylmethyl | 5.0 | - | - |
| ether and maleic anhydride, viscosity 0.1-0.5 | | | |
| Polyethylene glycol MW 8000 | - | 39.0 | - |
| Water-soluble K-polyacrylate MW 4000-8000 | - | 12.0 | - |
| Water-soluble Na-copolymer of acrylamide | - | 19.0 | - |
| (70%) and acryclic acid (30%) low MW | | | |
| Na triphosphate | 10.0 | - | - |
| Oxygenase | 0.05 | 0.09 | 0.15 |
| Dye | 2.5 | 1.0 | 1.0 |

| | **I** | **II** | **III** |
|---|---|---|---|
| | | | |
| Perfume | 3.0 | - | 7.0 |
| KOH / HCL solution | | pH 6-11 | |

### Example 28

The following toilet bowl cleaning composition was prepared according to the present invention.

| | **I** | **II** |
|---|---|---|
| | | |
| C14-15 linear alcohol 7EO | 2.0 | 10.0 |
| Citric acid | 10.0 | 5.0 |
| Oxygenase | 0.05 | 0.1 |
| DETPMP | - | 1.0 |
| Dye | 2.0 | 1.0 |
| Perfume | 3.0 | 3.0 |
| NaOH | pH 6-11 | |
| Water and minors | Up to 100% | |

### Example 29

The following single layer effervescent denture cleansing tablets were prepared according to the present invention :

| | **I** | **II** |
|---|---|---|
| | | |
| Oxygenase | 0.1 | 0.08 |
| Protease | 0.05 | 2.0 |
| Sodium bicarbonate | 39.0 | 39.0 |
| Malic acid | 14.0 | 14.0 |
| Sulphamic acid | 3.0 | 3.0 |
| TAED | 2.0 | 2.0 |
| Dye / Flavor | 2.0 | 2.0 |
| PB1 | 16.0 | 16.0 |
| EDTA | 3.0 | 3.0 |

| | **I** | **II** |
|---|---|---|
| | | |
| PEG 10,000 | 6.0 | 6.0 |
| K monopersulfate | 13.0 | 13.0 |
| Na carbonate | 1.0 | 1.0 |
| LAS | 1.0 | 1.0 |
| Pyrogenic silica | 2.0 | 2.0 |

### Example 30

The following dentifrice compositions were prepared according to the present invention :

| | **I** | **II** | **III** | **IV** |
|---|---|---|---|---|
| Sorbitol (70% aqueous solution) | 35.0 | 35.0 | 35.0 | 35.0 |
| PEG-6 | 1.0 | 1.0 | 1.0 | 1.0 |
| Silica dental abrasive | 20.0 | 20.0 | 20.0 | 20.0 |
| Sodium fluoride | 0.2 | 0.2 | 0.2 | 0.2 |
| Titanium dioxide | 0.5 | 0.5 | 0.5 | 0.5 |
| Sodium saccharin | 0.3 | 0.3 | 0.3 | 0.3 |
| Oxygenase | 0.05 | 0.08 | 0.1 | 0.2 |
| Protease | 0.05 | 0.1 | 0.9 | 2.0 |
| Sodium alkyl sulfate (27.9% | 4.0 | 4.0 | 4.0 | 4.0 |
| aqueous solution) | | | | |
| Flavor | 1.0 | 1.0 | 1.0 | 1.0 |
| Carboxyvinyl polymer | 0.3 | 0.3 | 0.3 | 0.3 |
| Carrageenan | 0.8 | 0.8 | 0.8 | 0.8 |
| Miscellaneous and water | | Up to 100% | | |

### Example 31

The following mouthwash compositions were prepared according to the present invention :

| | **I** | **II** | **III** | **IV** |
|---|---|---|---|---|
| SDA 40 Alcohol | 8.0 | 8.0 | 8.0 | 8.0 |
| Flavor | 0.08 | 0.08 | 0.08 | 0.08 |
| | | | | |

| | **I** | **II** | **III** | **IV** |
|---|---|---|---|---|
| Emulsifier | 0.08 | 0.08 | 0.08 | 0.08 |
| Sodium fluoride | 0.05 | 0.05 | 0.05 | 0.05 |
| Glycerin | 10.0 | 10.0 | 10.0 | 10.0 |
| Sweetener | 0.02 | 0.02 | 0.02 | 0.02 |
| Oxygenase | 0.05 | 0.08 | 0.1 | 0.2 |
| Protease | 0.01 | 0.09 | 0.2 | 2.0 |
| Benzoic acid | 0.05 | 0.05 | 0.05 | 0.05 |
| Sodium hydroxide | 0.2 | 0.2 | 0.2 | 0.2 |
| Dye | 0.04 | 0.04 | 0.04 | 0.04 |
| Miscellaneous and water | | Up to 100% | | |

## Claims

1. A cleaning composition comprising a surfactant and a proteinic substrate based oxygenase, selected from the group consisting of:
| | |
|---|---|
| 1.13.11.11 | TRYPTOPHAN 2,3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11.30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12. 9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROUN,2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN.2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYLGLYCINE MONOOXYGENASE. |

2. A cleaning composition according to claim 1 wherein said proteinic substrate based oxygenase is further **characterised by** being an iron sulphur or iron heme oxygenase and/or a heavy metal dependant oxygenase.

3. A cleaning composition according to claims 1-2 wherein said proteinic substrate based oxygenase is present at a level of from 0.0001% to 2%, preferably from 0.001% to 0.5%, more preferably from 0.002% to 0.1 % pure enzyme by weight of total composition.

4. A cleaning composition according to any of the preceding claims wherein said proteinic substrate based oxygenase is alkaline.

5. A cleaning composition according to any of the preceding claims further comprising a cofactor.

6. A cleaning composition according to claim 5 wherein the cofactor is comprised at a weight ratio of pure proteinic substrate based oxygenase to cofactor between 10:1 to 1:10, preferably 5:1 to 1:8, more preferably between 1:2 to 1:5.

7. A cleaning composition according to any of the preceding claims further comprising a detergent enzyme, preferably selected from cellulase, lipase, protease, amylase and/or mixtures thereof.

8. A cleaning composition according to claim 6 wherein said enzyme is a serine protease, preferably a bacterial serine protease obtained from *Bacillus, preferably Bacillus subtilis and*/*or Bacillus licheniformis.*

9. A cleaning composition according to any of the preceding claims further comprising another bleach system.

10. A cleaning composition according to claim 9 wherein said bleach system is a conventional activated bleach system.

11. A cleaning composition according to claim 10 wherein the bleaching agent is selected from perborate and/or percarbonate and the activator selected from tetraacetylethylenediamine, nonanoyloxybenzenesulfonate and/or 3,5,-trimethylhexanoloxybenzenesulfonate.

12. A cleaning composition according to claim 9 wherein said bleach system is another enzymatic bleach system.

13. A cleaning composition according to claim 9 wherein said bleach system is a metallo catalyst based bleach system.

14. A cleaning composition according to claim 13 wherein said metallo catalyst is a transition metal complex of a macropolycyclic rigid ligand.

15. A cleaning composition according to claims 13-14 wherein said metallo catalyst is manganese.

16. A cleaning composition according to any of the preceding claims which is in the form of an additive.

17. A fabric softening composition comprising proteinic based oxygenase selected from the group consisting of:
| | |
|---|---|
| 1.13.11.11 | TRYPTOPHAN 2.3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11-30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12.9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROLIN.2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN.2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYIGLYCINE MONOOXYGENASE, |
and a cationic surfactant comprising two long chain lengths.

18. Use of a proteinic substrate based oxygenase selected from the group consisting of:
| | |
|---|---|
| 1.13.11.11 | TRYPTOPHAN 2.3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11-30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12.9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROLIN.2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN.2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYIGLYCINE MONOOXYGENASE, |
in a cleaning and/or softening composition for fabric cleaning and/or fabric stain removal and/or fabric whiteness maintenance and/or fabric softening and/or fabric colour appearance and/or fabric dye transfer inhibition.

19. Use of a proteinic substrate based oxygenase selected from the group consisting of:
| | |
|---|---|
| 1.13.11.11 | TRYPTOPHAN 2.3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11-30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12.9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROLIN.2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN.2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYIGLYCINE MONOOXYGENASE, |
in a cleaning composition for cleaning hard surfaces such as floors, walls, bathroom tiles and the like.

20. Use of a proteinic substrate based oxygenase selected from the group consisting of:
| | |
|---|---|
| 1.13.11.11 | TRYPTOPHAN 2.3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11-30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12.9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROLIN.2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN.2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYIGLYCINE MONOOXYGENASE, |
in a cleaning composition for hand and machine dishwashing.

21. Use of a proteinic substrate based oxygenase selected from the group consisting of:
| | |
|---|---|
| 1.13.11.11 | TRYPTOPHAN 2.3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11-30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12.9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROLIN.2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN.2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYIGLYCINE MONOOXYGENASE, |
in a cleaning composition for oral and/or dental applications.

22. Use of a proteinic substrate based oxygenase selected from the group consisting of:
| | |
|---|---|
| 1.13.11.11 | TRYPTOPHAN 2.3-DIOXYGENASE |
| 1.13.11.20 | CYSTEINE DIOXYGENASE |
| 1.13.11.26 | PEPTIDE-TRYPTOPHAN 2,3-DIOXYGENASE |
| 1.13.11.29 | STIZOLOBATE SYNTHASE |
| 1.13.11-30 | STIZOLOBINATE SYNTHASE |
| 1.13.12.1 | ARGININE 2-MONOOXYGENASE |
| 1.13.12.2 | LYSINE 2-MONOOXYGENASE |
| 1.13.12.3 | TRYPTOPHAN 2-MONOOXYGENASE |
| 1.13.12.9 | PHENYLALANINE 2-MONOOXYGENASE |
| 1.13.12.10 | LYSINE 6-MONOOXYGENASE |
| 1.13.99.3 | TRYPTOPHAN 2'-DIOXYGENASE |
| 1.14.11.1 | γ-BUTYROBETAINE DIOXYGENASE |
| 1.14.11.2 | PROCOLLAGEN-PROLIN.2-OXOGLUTARATE 4-DIOXYGENASE |
| 1.14.11.4 | PROCOLLAGEN-LYSINE,2-OXOGLUTARATE 5-DIOXYGENASE |
| 1.14.11.7 | PROCOLLAGEN-PROLIN.2-OXOGLUTARATE 3-DIOXYGENASE |
| 1.14.11.8 | TRIMETHYLLYSINE,2-OXOGLUTARATE DIOXYGENASE |
| 1.14.11.16 | PEPTIDE-ASPARTATE β-DIOXYGENASE |
| 1.14.16.1 | PHENYLALANINE 4-MONOOXYGENASE |
| 1.14.16.2 | TYROSINE 3-MONOOXYGENASE |
| 1.14.16.4 | TRYPTOPHAN 5-MONOOXYGENASE |
| 1.14.17.3 | PEPTIDYIGLYCINE MONOOXYGENASE, |
in a cleaning and/or softening composition for the sanitisation of the treated surfaces.

## Patentansprüche

1. Reinigungszusammensetzung, umfassend ein Tensid und eine proteinsubstratbasierte Oxygenase, ausgewählt aus der Gruppe, bestehend aus:
| | |
|---|---|
| 1.13.11.11 | TRYPTOPHAN-2,3-DIOXYGENASE |
| 1.13.11.20 | CYSTEINDIOXYGENASE |
| 1.13.11.26 | PEPTIDTRYPTOPHAN-2,3-DIOXYGENASE |
| 1.13.11.29 | STIZOLOBATSYNTHASE |
| 1.13.11.30 | STIZOLOBINATSYNTHASE |
| 1.13.12.1 | ARGININ-2-MONOOXYGENASE |
| 1.13.12.2 | LYSIN-2-MONOOXYGENASE |
| 1.13.12.3 | TRYPTOPHAN-2-MONOOXYGENASE |
| 1.13.12.9 | PHENYLALANIN-2-MONOOXYGENASE |
| 1.13.12.10 | LYSIN-6-MONOOXYGENASE |
| 1.13.99.3 | TRYPTOPHAN-2'-DIOXYGENASE |
| 1.14.11.1 | γ-BUTYROBETAINDIOXYGENASE |
| 1.14.11.2 | PROCOLLAGEN-PROLIN-,2-OXOGLUTARAT-4-DIOXY-GENASE |
| 1.14.11.4 | PROCQLLAGEN-LYSIN-,2-OXOGLUTARAT-5-DIOXY-GENASE |
| 1.14.11.7 | PROCOLLAGEN-PROLIN-,2-OXOGLUTARAT-3-DIOXY-GENASE |
| 1.14.11.8 | TRIMETHYLLYSIN-,2-OXOGLUTARATDIOXYGENASE |
| 1.14.11.16 | PEPTIDASPARTAT-β-DIOXYGENASE |
| 1.14.16.1 | PHENYLALANIN,4-MONOOXYGENASE |
| 1.14.16.2 | TYROSIN-3-MONOOXYGENASE |
| 1.14.16.4 | TRYPTOPHAN-5-MONOOXYGENASE |
| 1.14.17.3 | PEPTIDYLGLYCINMONOOXYGENASE |

2. Reinigungszusammensetzung nach Anspruch 1, wobei die proteinsubstratbasierte Oxygenase ferner **dadurch gekennzeichnet ist, dass** sie eine Eisen- ' Schwefel- oder Eisen-Hämoxygenase und/oder eine schwermetallabhängige Oxygenase ist.

3. Reinigungszusammensetzung nach Ansprüchen 1-2, wobei die proteinsubstratbasierte Oxygenase in einer Konzentration an reinem Enzym von 0,0001 Gew.-% bis 2 Gew.-%, vorzugsweise von 0,001 Gew.-% bis 0,5 Gew.-%, mehr bevorzugt von 0,002 Gew.-% bis 0,1 Gew.-% der Gesamtzusammensetzung vorhanden ist.

4. Reinigungszusammensetzung nach einem der vorstehenden Ansprüche, wobei die proteinsubstratbasierte Oxygenase alkalisch ist.

5. Reinigungszusammensetzung nach einem der vorstehenden Ansprüche, die ferner einen Cofaktor umfasst.

6. Reinigungszusammensetzung nach Anspruch 5, wobei der Cofaktor in einem Gewichtsverhältnis von reiner proteinsubstratbasierter Oxygenase zu Cofaktor von 10:1 bis 1:10, vorzugsweise 5:1 bis 1:8, mehr bevorzugt von 1:2 bis 1:5 enthalten ist.

7. Reinigungszusammensetzung nach einem der vorstehenden Ansprüche, die ferner ein Reinigungsenzym umfasst, das vorzugsweise aus Cellulase, Lipase, Protease, Amylase und/oder Mischungen davon ausgewählt ist.

8. Reinigungszusammensetzung nach Anspruch 6, wobei das Enzym eine Serinprotease ist, vorzugsweise eine bakterielle Serinprotease, die aus *Bacillus,* vorzugsweise *Bacillus subtilis* und/oder *Bacillus licheniformis,* gewonnen wird.

9. Reinigungszusammensetzung nach einem der vorstehenden Ansprüche, die ferner ein weiteres Bleichsystem umfasst.

10. Reinigungszusammensetzung nach Anspruch 9, wobei das Bleichsystem ein herkömmliches aktiviertes Bleichsystem ist.

11. Reinigungszusammensetzung nach Anspruch 10, wobei das Bleichmittel aus Perborat und/oder Percarbonat ausgewählt ist und der Aktivator aus Tetraacetylethylendiamin, Nonanoyloxybenzolsulfonat und/oder 3,5,-Trimethylhexanoloxybenzolsulfonat ausgewählt ist.

12. Reinigungszusammensetzung nach Anspruch 9, wobei das Bleichsystem ein weiteres enzymatisches Bleichsystem ist.

13. Reinigungszusammensetzung nach Anspruch 9, wobei das Bleichsystem ein Bleichsystem auf Metallkatalysatorbasis ist.

14. Reinigungszusammensetzung nach Anspruch 13, wobei der Metallkatalysator ein Übergangsmetallkomplex eines makropolycyclischen unnachgiebigen Liganden ist.

15. Reinigungszusammensetzung nach Ansprüchen 13-14, wobei der Metallkatalysator Mangan ist.

16. Reinigungszusammensetzung nach einem der vorstehenden Ansprüche, die in der Form eines Zusatzes ist.

17. Stoffweichmacherzusammensetzung, umfassend proteinbasierte Oxygenase, ausgewählt aus der Gruppe, bestehend aus:
| | |
|---|---|
| 1.13.11.11 | TRYPTOPHAN-2,3-DIOXYGENASE |
| 1.13.11.20 | CYSTEINDIOXYGENASE |
| 1.13.11.26 | PEPTIDTRYPTOPHAN-2,3-DIOXYGENASE |
| 1.13.11.29 | STIZOLOBATSYNTHASE |
| 1.13.11.30 | STIZOLOBINATSYNTHASE |
| 1.13.12.1 | ARGININ-2-MONOOXYGENASE |
| 1.13.12.2 | LYSIN-2-MONOOXYGENASE |
| 1.13.12.3 | TRYPTOPHAN-2-MONOOXYGENASE |
| 1.13.12.9 | PHENYLALANIN-2-MONOOXYGENASE |
| 1.13.12.10 | LYSIN-6-MONOOXYGENASE |
| 1.13.99.3 | TRYPTOPHAN-2'-DIOXYGENASE |
| 1.14.11.1 | γ-BUTYROBETAINDIOXYGENASE . |
| 1.14.11.2 | PROCOLLAGEN-PROLIN-,2-OXOGLUTARAT-4-DIOXYGENASE |
| 1.14.11.4 | PROCOLLAGEN-LYSIN-,2-OXOGLUTARAT-5-DIOXYGENASE |
| 1.14.11.7 | PROCOLLAGEN-PROLIN-,2-OXOGLUTARAT-3-DIOXY-GENASE |
| 1.14.11.8 | TRIMETHYLLYSIN-,2-OXOGLUTARATDIOXYGENASE |
| 1.14.11.16 | PEPTIDASPARTAT-β-DIOXYGENASE |
| 1.14.16.1 | PHENYLALANIN,4-MONOOXYGENASE |
| 1.14.16.2 | TYROSIN-3-MONOOXYGENASE |
| 1.14.16.4 | TRYPTOPHAN-5-MONOOXYGENASE |
| 1.14.17.3 | PEPTIDYLGLYCINMONOOXYGENASE, |
und ein kationisches Tensid, das zwei lange Ketten umtasst.

18. Verwendung einer proteinsubstratbasierten Oxygenase, ausgewählt aus der Gruppe, bestehend aus:
| | |
|---|---|
| 1.13.11.11 | TRYPTOPHAN-2,3-DIOXYGENASE |
| 1.13.11.20 | CYSTEINDIOXYGENASE |
| 1.13.11.26 | PEPTIDTRYPTOPHAN-2,3-DIOXYGENASE |
| 1.13.11.29 | STIZOLOBATSYNTHASE |
| 1.13.11.30 | STIZOLOBINATSYNTHASE |
| 1.13.12.1 | ARGININ-2-MONOOXYGENASE |
| 1.13.12.2 | LYSIN-2-MONOOXYGENASE |
| 1.13.12.3 | TRYPTOPHAN-2-MONOOXYGENASE |
| 1.13.12.9 | PHENYLALANIN-2-MONOOXYGENASE |
| 1.13.12.10 | LYSIN-6-MONOOXYGENASE |
| 1.13.99.3 | TRYPTOPHAN-2'-DIOXYGENASE |
| 1.14.11.1 | γ-BUTYROBETAINDIOXYGENASE |
| 1.14.11.2 | PROCOLLAGEN-PROLIN-,2-OXOGLUTARAT-4-DIOXYGENASE |
| 1.14.11.4 | PROCOLLAGEN-LYSIN-,2-OXOGLUTARAT-5-DIOXYGENASE |
| 1.14.11.7 | PROCOLLAGEN-PROLIN-,2-OXOGLUTARAT-3-DIOXYGENASE |
| 1.14.11.8 | TRIMETHYLLYSIN-,2-OXOGLUTARATDIOXYGENASE |
| 1.14.11.16 | PEPTIDASPARTAT-β-DIOXYGENASE |
| 1.14.16.1 | PHENYLALANIN,4-MONOOXYGENASE |
| 1.14.16.2 | TYROSIN-3-MONOOXYGENASE |
| 1.14.16.4 | TRYPTOPHAN-5-MONOOXYGENASE |
| 1.14.17.3 | PEPTIDYLGLYCINMONOOXYGENASE, |
in einer Reinigungs- und/oder Weichmacherzusammensetzung zur Stoffreinigung und/oder Fleckenentfernung aus Stoff und/oder Weißbeständigkeit von Stoff und/oder Stoffweichmachung und/oder für das Farbaussehen des Stoffes und/oder zur Hemmung der Farbstoffübertragung auf Stoff. .

19. Verwendung einer proteinsubstratbasierten Oxygenase, ausgewählt aus der Gruppe, bestehend aus:
| | |
|---|---|
| 1.13.11.11 | TRYPTOPHAN-2,3-DIOXYGENASE |
| 1.13.11.20 | CYSTEINDIOXYGENASE |
| 1.13.11.26 | PEPTIDTRYPTOPHAN-2,3-DIOXYGENASE |
| 1.13.11.29 | STIZOLOBATSYNTHASE |
| 1.13.11.30 | STIZOLOBINATSYNTHASE |
| 1.13.12.1 | ARGININ-2-MONOOXYGENASE |
| 1.13.12.2 | LYSIN-2-MONOOXYGENASE |
| 1.13.12.3 | TRYPTOPHAN-2-MONOOXYGENASE |
| 1.13.12.9 | PHENYLALANIN-2-MONOOXYGENASE |
| 1.13.12.10 | LYSIN-6-MONOOXYGENASE |
| 1.13.99.3 | TRYPTOPHAN-2'-DIOXYGENASE |
| 1.14.11.1 | γ-BUTYROBETAINDIOXYGENASE |
| 1.14.11.2 | PROCOLLAGEN-PROLIN-,2-OXOGLUTARAT-4-DIOXYGENASE |
| 1.14.11.4 | PROCOLLAGEN-LYSIN-,2-OXOGLUTARAT-5-DIOXYGENASE |
| 1.14.11.7 | PROCOLLAGEN-PROLIN-,2-OXOGLUTARAT-3-DIOXYGENASE |
| 1.14.11.8 | TRIMETHYLLYSIN-,2-OXOGLUTARATDIOXYGENASE |
| 1.14.11.16 | PEPTIDASPARTAT-β-DIOXYGENASE |
| 1.14.16.1 | PHENYLALANIN,4-MONOOXYGENASE |
| 1.14.16.2 | TYROSIN-3-MONOOXYGENASE |
| 1.14.16.4 | TRYPTOPHAN-5-MONOOXYGENASE |
| 1.14.17.3 | PEPTIDYLGLYCINMONOOXYGENASE, |
in einer Reinigungszusammensetzung zur Reinigung harter Oberflächen, wie Fußböden, Wänden, Badfliesen und dergleichen.

20. Verwendung einer proteinsubstratbasierten Oxygenase, ausgewählt aus der Gruppe, bestehend aus:
| | |
|---|---|
| 1.13.11.11 | TRYPTOPHAN-2,3-DIOXYGENASE |
| 1.13.11.20 | CYSTEINDIOXYGENASE |
| 1.13.11.26 | PEPTIDTRYPTOPHAN-2,3-DIOXYGENASE |
| 1.13.11.29 | STIZOLOBATSYNTHASE |
| 1.13.11.30 | STIZOLOBINATSYNTHASE |
| 1.13.12.1 | ARGININ-2-MONOOXYGENASE |
| 1.13.12.2 | LYSIN-2-MONOOXYGENASE |
| 1.13.12.3 | TRYPTOPHAN-2-MONOOXYGENASE |
| 1.13.12.9 | PHENYLALANIN-2-MONOOXYGENASE |
| 1.13.12.10 | LYSIN-6-MONOOXYGENASE |
| 1.13.99.3 | TRYPTOPHAN-2'-DIOXYGENASE |
| 1.14.11.1 | γ-BUTYROBETAINDIOXYGENASE |
| 1.14.11.2 1.14.11.4 | PROCOLLAGEN-PROLIN-,2-OXOGLUTARAT-4-DIOXYGENASE PROCOLLAGEN-LYSIN-,2-OXOGLUTARAT-5-DIOXYGENASE |
| 1.14.11.7 | PROCOLLAGEN-PROLIN-,2-OXOGLUTARAT-3-DIOXYGENASE |
| 1.14.11.8 | TRIMETHYLLYSIN-,2-OXOGLUTARATDIOXYGENASE |
| 1.14.11.16 | PEPTIDASPARTAT-β-DIOXYGENASE |
| 1.14.16.1 | PHENYLALANIN,4-MONOOXYGENASE |
| 1.14.16.2 | TYROSIN-3-MONOOXYGENASE |
| 1.14.16.4 | TRYPTOPHAN-5-MONOOXYGENASE |
| 1.14.17.3 | PEPTIDYLGLYCINMONOOXYGENASE, |
in einer Reinigungszusammensetzung zum manuellen und maschinellen Geschirrspülen.

21. Verwendung einer proteinsubstratbasierten Oxygenase, ausgewählt aus der Gruppe, bestehend aus:
| | |
|---|---|
| 1.13.11.11 | TRYPTOPHAN-2,3-DIOXYGENASE |
| 1.13.11.20 | CYSTEINDIOXYGENASE |
| 1.13.11.26 | PEPTIDTRYPTOPHAN-2,3-DIOXYGENASE |
| 1.13.11.29 | STIZOLOBATSYNTHASE |
| 1.13.11.30 | STIZOLOBINATSYNTHASE |
| 1.13.12.1 | ARGININ-2-MONOOXYGENASE |
| 1.13.12.2 | LYSIN-2-MONOOXYGENASE |
| 1.13.12.3 | TRYPTOPHAN-2-MONOOXYGENASE |
| 1.13.12.9 | PHENYLALANIN-2-MONOOXYGENASE |
| 1.13.12.10 | LYSIN-6-MONOOXYGENASE |
| 1.13.99.3 | TRYPTOPHAN-2'-DIOXYGENASE |
| 1.14.11.1 | γ-BUTYROBETAINDIOXYGENASE |
| 1.14.11.2 | PROCOLLAGEN-PROLIN-,2-OXOGLUTARAT-4-DIOXYGENASE |
| 1.14.11.4 | PROCOLLAGEN-LYSIN-,2-OXOGLUTARAT-5-DIOXYGENASE |
| 1.14.11.7 | PROCOLLAGEN-PROLIN-,2-OXOGLUTARAT-3-DIOXYGENASE |
| 1.14.11.8 | TRIMETHYLLYSIN-,2-OXOGLUTARATDIOXYGENASE |
| 1.14.11.16 | PEPTIDASPARTAT-β-DIOXYGENASE |
| 1.14.16.1 | PHENYLALANIN,4-MONOOXYGENASE |
| 1.14.16.2 | TYROSIN-3-MONOOXYGENASE |
| 1.14.16.4 | TRYPTOPHAN-5-MONOOXYGENASE |
| 1.14.17.3 | PEPTIDYLGLYCINMONOOXYGENASE, |
in einer Reinigungszusammensetzung für orale und/oder dentale Anwendungen.

22. Verwendung einer proteinsubstratbasierten Oxygenase, ausgewählt aus der Gruppe, bestehend aus:
| | |
|---|---|
| 1.13.11.11 | TRYPTOPHAN-2,3-DIOXYGENASE |
| 1.13.11.20 | CYSTEINDIOXYGENASE |
| 1.13.11.26 | PEPTIDTRYPTOPHAN-2,3-DIOXYGENASE |
| 1.13.11.29 | STIZOLOBATSYNTHASE |
| 1.13.11.30 | STIZOLOBINATSYNTHASE |
| 1.13.12.1 | ARGININ-2-MONOOXYGENASE |
| 1.13.12.2 | LYSIN-2-MONOOXYGENASE |
| 1.13.12.3 | TRYPTOPHAN-2-MONOOXYGENASE |
| 1.13.12.9 | PHENYLALANIN-2-MONOOXYGENASE |
| 1.13.12.10 | LYSIN-6-MONOOXYGENASE |
| 1.13.99.3 | TRYPTOPHAN-2'-DIOXYGENASE |
| 1.14.11.1 | γ-BUTYROBETAINDIOXYGENASE |
| 1.14.11.2 | PROCOLLAGEN-PROLIN-,2-OXOGLUTARAT-4-DIOXYGENASE |
| 1.14.11.4 | PROCOLLAGEN-LYSIN-,2-OXOGLUTARAT-5-DIOXYGENASE |
| 1.14.11.7 | PROCOLLAGEN-PROLIN-,2-OXOGLUTARAT-3-DIOXYGENASE |
| 1.14.11.8 | TRIMETHYLLYSIN-,2-OXOGLUTARATDIOXYGENASE |
| 1.14.11.16 | PEPTIDASPARTAT-β-DIOXYGENASE |
| 1.14.16.1 | PHENYLALANIN,4-MONOOXYGENASE |
| 1.14.16.2 | TYROSIN-3-MONOOXYGENASE |
| 1.14.16.4 | TRYPTOPHAN-5-MONOOXYGENASE |
| 1.14.17.3 | PEPTIDYLGLYCINMONOOXYGENASE, |
in einer Reinigungs- und/oder Weichmacherzusammensetzung zur Desinfektion der behandelten Oberflächen.

## Revendications

1. Composition de nettoyage comprenant un agent tensioactif et une oxygénase à base de substrat protéinique, choisie dans le groupe constitué de:
| | |
|---|---|
| 1.13.11.11 | TRYPTOPHANE 2,3-DIOXYGÉNASE |
| 1.13.11.20 | CYSTÉINE DIOXYGÉNASE |
| 1.13.11.26 | PEPTIDE-TRYPTOPHANE 2,3-DIOXYGÉNASE |
| 1.13.11.29 | STIZOLOBATE SYNTHASE |
| 1.13.11.30 | STIZOLOBINATE SYNTHASE |
| 1.13.12.1 | ARGININE 2-MONOOXYGÉNASE |
| 1.13.12.2 | LYSINE 2-MONOOXYGÉNASE |
| 1.13.12.3 | TRYPTOPHANE 2-MONOOXYGÉNASE |
| 1.13.12.9 | PHÉNYLALANINE 2-MONOOXYGÉNASE |
| 1.13.12.10 | LYSINE 6-MONOOXYGÉNASE |
| 1.13.99.3 | TRYPTOPHANE 2'-DIOXYGÉNASE |
| 1.14.11.1 | γ-BUTYROBÉTAÏNE DIOXYGÉNASE |
| 1.14.11.2 | PROCOLLAGÈNE-PROLINE,2-OXOGLUTARATE 4-DIOXYGÉNASE |
| 1.14.11.4 | PROCOLLAGÈNE-LYSINE,2-OXOGLUTARATE 5-DIOXYGÉNASE |
| 1.14.11.7 | PROCOLLAGÈNE-PROLINE,2-OXOGLUTARATE 3-DIOXYGÉNASE |
| 1.14.11.8 | TRIMÉTHYL-LYSINE,2-OXOGLUTARATE DIOXYGÉNASE |
| 1.14.11.16 | PEPTIDE-ASPARTATE β-DIOXYGÉNASE |
| 1.14.16.1 | PHÉNYLALANINE 4-MONOOXYGÉNASE |
| 1.14.16.2 | TYROSINE 3-MONOOXYGÉNASE |
| 1.14.16.4 | TRYPTOPHANE 5-MONOOXYGÉNASE |
| 1.14.17.3 | PEPTIDYLGLYCINE MONOOXYGÉNASE |

2. Composition de nettoyage selon la revendication 1, dans laquelle ladite oxygénase à base de substrat protéinique est en outre **caractérisée en ce qu'**elle est une oxygénase de soufre ferrique ou d'hème ferrique et/ou une oxygénase dépendante des métaux lourds.

3. Composition de nettoyage selon les revendications 1 à 2, dans laquelle ladite oxygénase à base de substrat protéinique est présente à un niveau allant de 0,0001 % à 2 %, de préférence de 0,001 % à 0,5 %, plus préférablement de 0,002 % à 0,1 % d'enzyme pure en poids de composition totale.

4. Composition de nettoyage selon l'une quelconque des revendications précédentes, dans laquelle ladite oxygénase à base de substrat protéinique est alcaline.

5. Composition de nettoyage selon l'une quelconque des revendications précédentes, comprenant en outre un cofacteur.

6. Composition de nettoyage selon la revendication 5, dans laquelle le cofacteur est compris à un rapport pondéral de l'oxygénase pure à base de substrat protéinique au cofacteur compris entre 10:1 et 1:10, de préférence 5:1 à 1:8, plus préférablement 1:2 et 1:5.

7. Composition de nettoyage selon l'une quelconque des revendications précédentes, comprenant en outre une enzyme détergente, de préférence choisie parmi la cellulase, la lipase, la protéase, l'amylase et/ou leurs mélanges.

8. Composition de nettoyage selon la revendication 6, dans laquelle ladite enzyme est une sérine protéase, de préférence une sérine protéase bactérienne obtenue à partir de *Bacillus,* de préférence *Bacillus subtilis* et/ou *Bacillus licheniformis.*

9. Composition de nettoyage selon l'une quelconque des revendications précédentes, comprenant en outre un autre système de blanchiment.

10. Composition de nettoyage selon la revendication 9, dans laquelle ledit système de blanchiment est un système de blanchiment activé classique.

11. Composition de nettoyage selon la revendication 10, dans laquelle l'agent de blanchiment est choisi parmi le perborate et/ou le percarbonate et l'activateur choisi parmi la tétraacétyléthylènediamine, le nonanoyloxybenzènesulfonate et/ou le 3,5,-triméthylhexanoloxybenzènesulfonate.

12. Composition de nettoyage selon la revendication 9, dans laquelle ledit système de blanchiment est un autre système de blanchiment enzymatique.

13. Composition de nettoyage selon la revendication 9, dans laquelle ledit système de blanchiment est un système de blanchiment à base de catalyseur métallocène.

14. Composition de nettoyage selon la revendication 13, dans laquelle ledit catalyseur métallocène est un complexe de métaux de transition d'un ligand rigide macropolycyclique.

15. Composition de nettoyage selon les revendications 13 à 14, dans laquelle ledit catalyseur métallocène est du manganèse.

16. Composition de nettoyage selon l'une quelconque des revendications précédentes, laquelle est sous la forme d'un additif.

17. Composition d'adoucissement des tissus comprenant une oxygénase à base protéinique choisie dans le groupe constitué de:
| | |
|---|---|
| 1.13.11.11 | TRYPTOPHANE 2,3-DIOXYGÉNASE |
| 1.13.11.20 | CYSTÉINE DIOXYGÉNASE |
| 1.13.11.26 | PEPTIDE-TRYPTOPHANE 2,3-DIOXYGÉNASE |
| 1.13.11.29 | STIZOLOBATE SYNTHASE |
| 1.13.11.30 | STIZOLOBINATE SYNTHASE |
| 1.13.12.1 | ARGININE 2-MONOOXYGÉNASE |
| 1.13.12.2 | LYSINE 2-MONOOXYGÉNASE |
| 1.13.12.3 | TRYPTOPHANE 2-MONOOXYGÉNASE |
| 1.13.12.9 | PHÉNYLALANINE 2-MONOOXYGÉNASE |
| 1.13.12.10 | LYSINE 6-MONOOXYGÉNASE |
| 1.13.99.3 | TRYPTOPHANE 2'-DIOXYGÉNASE |
| 1.14.11.1 | γ-BUTYROBÉTAÏNE DIOXYGÉNASE |
| 1.14.11.2 | PROCOLLAGÈNE-PROLINE,2-OXOGLUTARATE 4-DIOXYGÉNASE |
| 1.14.11.4 | PROCOLLAGÈNE-LYSINE,2-OXOGLUTARATE 5-DIOXYGÉNASE |
| 1.14.11.7 | PROCOLLAGÈNE-PROLINE,2-OXOGLUTARATE 3-DIOXYGÉNASE |
| 1.14.11.8 | TRIMÉTHYL-LYSINE,2-OXOGLUTARATE DIOXYGÉNASE |
| 1.14.11.16 | PEPTIDE-ASPARTATE β-DIOXYGÉNASE |
| 1.14.16.1 | PHÉNYLALANINE 4-MONOOXYGÉNASE |
| 1.14.16.2 | TYROSINE 3-MONOOXYGÉNASE |
| 1.14.16.4 | TRYPTOPHANE 5-MONOOXYGÉNASE |
| 1.14.17.3 | PEPTIDYLGLYCINE MONOOXYGÉNASE, |
et un agent tensioactif cationique comprenant deux longueurs de chaînes longues.

18. Utilisation d'une oxygénase à base de substrat protéinique choisie dans le groupe constitué de:
| | |
|---|---|
| 1.13.11.11 | TRYPTOPHANE 2,3-DIOXYGÉNASE |
| 1.13.11.20 | CYSTÉINE DIOXYGÉNASE |
| 1.13.11.26 | PEPTIDE-TRYPTOPHANE 2,3-DIOXYGÉNASE |
| 1.13.11.29 | STIZOLOBATE SYNTHASE |
| 1.13.11.30 | STIZOLOBINATE SYNTHASE |
| 1.13.12.1 | ARGININE 2-MONOOXYGÉNASE |
| 1.13.12.2 | LYSINE 2-MONOOXYGÉNASE |
| 1.13.12.3 | TRYPTOPHANE 2-MONOOXYGÉNASE |
| 1.13.12. 9 | PHÉNYLALANINE 2-MONOOXYGÉNASE |
| 1.13.12.10 | LYSINE 6-MONOOXYGÉNASE |
| 1.13.99.3 | TRYPTOPHANE 2'-DIOXYGÉNASE |
| 1.14.11.1 | γ-BUTYROBÉTAÏNE DIOXYGÉNASE |
| 1.14.11.2 | PROCOLLAGÈNE-PROLINE,2-OXOGLUTARATE 4-DIOXYGÉNASE |
| 1.14.11.4 | PROCOLLAGÈNE-LYSINE,2-OXOGLUTARATE 5-DIOXYGÉNASE |
| 1.14.11.7 | PROCOLLAGÈNE-PROLINE,2-OXOGLUTARATE 3-DIOXYGÉNASE |
| 1.14.11.8 | TRIMÉTHYL-LYSINE,2-OXOGLUTARATE DIOXYGÉNASE |
| 1.14.11.16 | PEPTIDE-ASPARTATE β-DIOXYGÉNASE |
| 1.14.16.1 | PHÉNYLALANINE 4-MONOOXYGÉNASE |
| 1.14.16.2 | TYROSINE 3-MONOOXYGÉNASE |
| 1.14.16.4 | TRYPTOPHANE 5-MONOOXYGÉNASE |
| 1.14.17.3 | PEPTIDYLGLYCINE MONOOXYGÉNASE, |
dans une composition de nettoyage et/ou d'adoucissement pour le nettoyage des tissus et/ou l'élimination des taches des tissus et/ou le maintien de la blancheur des tissus et/ou l'adoucissement des tissus et/ou l'apparence de couleur des tissus et/ou l'inhibition du transfert de couleur des tissus.

19. Utilisation d'une oxygénase à base de substrat protéinique choisie dans le groupe constitué de:
| | |
|---|---|
| 1.13.11.11 | TRYPTOPHANE 2,3-DIOXYGÉNASE |
| 1.13.11.20 | CYSTÉINE DIOXYGÉNASE |
| 1.13.11.26 | PEPTIDE-TRYPTOPHANE 2,3-DIOXYGÉNASE |
| 1.13.11.29 | STIZOLOBATE SYNTHASE |
| 1.13.11.30 | STIZOLOBINATE SYNTHASE |
| 1.13.12.1 | ARGININE 2-MONOOXYGÉNASE |
| 1.13.12.2 | LYSINE 2-MONOOXYGÉNASE |
| 1.13.12.3 | TRYPTOPHANE 2-MONOOXYGÉNASE |
| 1.13.12. 9 | PHÉNYLALANINE 2-MONOOXYGÉNASE |
| 1.13.12.10 | LYSINE 6-MONOOXYGÉNASE |
| 1.13.99.3 | TRYPTOPHANE 2'-DIOXYGÉNASE |
| 1.14.11.1 | γ-BUTYROBÉTAÏNE DIOXYGÉNASE |
| 1.14.11.2 | PROCOLLAGÈNE-PROLINE,2-OXOGLUTARATE 4-DIOXYGÉNASE |
| 1.14.11.4 | PROCOLLAGÈNE-LYSINE,2-OXOGLUTARATE 5-DIOXYGÉNASE |
| 1.14.11.7 | PROCOLLAGÈNE-PROLINE,2-OXOGLUTARATE 3-DIOXYGÉNASE |
| 1.14.11.8 | TRIMÉTHYL-LYSINE,2-OXOGLUTARATE DIOXYGÉNASE |
| 1.14.11.16 | PEPTIDE-ASPARTATE β-DIOXYGÉNASE |
| 1.14.16.1 | PHÉNYLALANINE 4-MONOOXYGÉNASE |
| 1.14.16.2 | TYROSINE 3-MONOOXYGÉNASE |
| 1.14.16.4 | TRYPTOPHANE 5-MONOOXYGÉNASE |
| 1.14.17.3 | PEPTIDYLGLYCINE MONOOXYGÉNASE, |
dans une composition de nettoyage des surfaces dures telles que les sols, les murs, les carreaux de salle de bain et similaires.

20. Utilisation d'une oxygénase à base de substrat protéinique choisie dans le groupe constitué de:
| | |
|---|---|
| 1.13.11.11 | TRYPTOPHANE 2,3-DIOXYGÉNASE |
| 1.13.11.20 | CYSTÉINE DIOXYGÉNASE |
| 1.13.11.26 | PEPTIDE-TRYPTOPHANE 2,3-DIOXYGÉNASE |
| 1.13.11.29 | STIZOLOBATE SYNTHASE |
| 1.13.11.30 | STIZOLOBINATE SYNTHASE |
| 1.13.12.1 | ARGININE 2-MONOOXYGÉNASE |
| 1.13.12.2 | LYSINE 2-MONOOXYGÉNASE |
| 1.13.12.3 | TRYPTOPHANE 2-MONOOXYGÉNASE |
| 1.13.12. 9 | PHÉNYLALANINE 2-MONOOXYGÉNASE |
| 1.13.12.10 | LYSINE 6-MONOOXYGÉNASE |
| 1.13.99.3 | TRYPTOPHANE 2'-DIOXYGÉNASE |
| 1.14.11.1 | γ-BUTYROBÉTAÏNE DIOXYGÉNASE |
| 1.14.11.2 | PROCOLLAGÈNE-PROLINE,2-OXOGLUTARATE 4- |
| | DIOXYGÉNASE |
| 1.14.11.4 | PROCOLLAGÈNE-LYSINE,2-OXOGLUTARATE 5-DIOXYGÉNASE |
| 1.14.11.7 | PROCOLLAGÈNE-PROLINE,2-OXOGLUTARATE 3-DIOXYGÉNASE |
| 1.14.11.8 | TRIMÉTHYL-LYSINE,2-OXOGLUTARATE DIOXYGÉNASE |
| 1.14.11.16 | PEPTIDE-ASPARTATE β-DIOXYGÉNASE |
| 1.14.16.1 | PHÉNYLALANINE 4-MONOOXYGÉNASE |
| 1.14.16.2 | TYROSINE 3-MONOOXYGÉNASE |
| 1.14.16.4 | TRYPTOPHANE 5-MONOOXYGÉNASE |
| 1.14.17.3 | PEPTIDYLGLYCINE MONOOXYGÉNASE, |
dans une composition de nettoyage pour le lavage de la vaisselle à la main et en machine.

21. Utilisation d'une oxygénase à base de substrat protéinique choisie dans le groupe constitué de:
| | |
|---|---|
| 1.13.11.11 | TRYPTOPHANE 2,3-DIOXYGÉNASE |
| 1.13.11.20 | CYSTÉINE DIOXYGÉNASE |
| 1.13.11.26 | PEPTIDE-TRYPTOPHANE 2,3-DIOXYGÉNASE |
| 1.13.11.29 | STIZOLOBATE SYNTHASE |
| 1.13.11.30 | STIZOLOBINATE SYNTHASE |
| 1.13.12.1 | ARGININE 2-MONOOXYGÉNASE |
| 1.13.12.2 | LYSINE 2-MONOOXYGÉNASE |
| 1.13.12.3 | TRYPTOPHANE 2-MONOOXYGÉNASE |
| 1.13.12. 9 | PHÉNYLALANINE 2-MONOOXYGÉNASE |
| 1.13.12.10 | LYSINE 6-MONOOXYGÉNASE |
| 1.13.99.3 | TRYPTOPHANE 2'-DIOXYGÉNASE |
| 1.14.11.1 | γ-BUTYROBÉTAÏNE DIOXYGÉNASE |
| 1.14.11.2 | PROCOLLAGÈNE-PROLINE,2-OXOGLUTARATE 4-DIOXYGÉNASE |
| 1.14.11.4 | PROCOLLAGÈNE-LYSINE,2-OXOGLUTARATE 5-DIOXYGÉNASE |
| 1.14.11.7 | PROCOLLAGÈNE-PROLINE,2-OXOGLUTARATE 3-DIOXYGÉNASE |
| 1.14.11.8 | TRIMÉTHYL-LYSINE,2-OXOGLUTARATE DIOXYGÉNASE |
| 1.14.11.16 | PEPTIDE-ASPARTATE β-DIOXYGÉNASE |
| 1.14.16.1 | PHÉNYLALANINE 4-MONOOXYÉNASE |
| 1.14.16.2 | TYROSINE 3-MONOOXYGÉNASE |
| 1.14.16.4 | TRYPTOPHANE 5-MONOOXYGÉNASE |
| 1.14.17.3 | PEPTIDYLGLYCINE MONOOXYGÉNASE, |
dans une composition de nettoyage pour des applications orales et/ou dentaires.

22. Utilisation d'une oxygénase à base de substrat protéinique choisie dans le groupe constitué par:
| | |
|---|---|
| 1.13.11.11 | TRYPTOPHANE 2,3-DIOXYGÉNASE |
| 1.13.11.20 | CYSTÉINE DIOXYGÉNASE |
| 1.13.11.26 | PEPTIDE-TRYPTOPHANE 2,3-DIOXYGÉNASE |
| 1.13.11.29 | STIZOLOBATE SYNTHASE |
| 1.13.11.30 | STIZOLOBINATE SYNTHASE |
| 1.13.12.1 | ARGININE 2-MONOOXYGÉNASE |
| 1.13.12.2 | LYSINE 2-MONOOXYGÉNASE |
| 1.13.12.3 | TRYPTOPHANE 2-MONOOXYGÉNASE |
| 1.13.12. 9 | PHÉNYLALANINE 2-MONOOXYGÉNASE |
| 1.13.12.10 | LYSINE 6-MONOOXYGÉNASE |
| 1.13.99.3 | TRYPTOPHANE 2'-DIOXYGÉNASE |
| 1.14.11.1 | γ-BUTYROBÉTAÏNE DIOXYGÉNASE |
| 1.14.11.2 | PROCOLLAGÈNE-PROLINE,2-OXOGLUTARATE 4-DIOXYGÉNASE |
| 1.14.11.4 | PROCOLLAGÈNE-LYSINE,2-OXOGLUTARATE 5-DIOXYGÉNASE |
| 1.14.11.7 | PROCOLLAGÈNE-PROLINE,2-OXOGLUTARATE 3-DIOXYGÉNASE |
| 1.14.11.8 | TRIMÉTHYL-LYSINE,2-OXOGLUTARATE DIOXYGÉNASE |
| 1.14.11.16 | PEPTIDE-ASPARTATE β-DIOXYGÉNASE |
| 1.14.16.1 | PHÉNYLALANINE 4-MONOOXYGÉNASE |
| 1.14.16.2 | TYROSINE 3-MONOOXYGÉNASE |
| 1.14.16.4 | TRYPTOPHANE 5-MONOOXYGÉNASE |
| 1.14.17.3 | PEPTIDYLGLYCINE MONOOXYGÉNASE, |
dans une composition de nettoyage et/ou d'adoucissement pour l'assainissement des surfaces traitées.
